# EUROPEAN PATENT SPECIFICATION

(11) **EP 3 303 283 B1**
(45) Date of publication and mention of the grant of the patent: **21.04.2021**
(21) Application number: 16813689.3
(22) Date of filing: 20.06.2016
(51) Int. Cl.: C07C 255/51, C07C 255/53, A61K 31/277, A61P 3/00, A61P 3/04, A61P 3/06, A61P 3/10, A61P 9/00, A61P 9/12, A61P 9/10, A61P 25/16

(54) **FTO INHIBITORS**
FTO-INHIBITOREN
INHIBITEURS DE FTO

(30) Priority: 23.06.2015 WO PCT/CN2015/082052
(43) Date of publication of application: 11.04.2018
(73) Proprietor: National Institute Of Biological Sciences, Beijing, Beijing 102206 (CN)
(72) Inventor: HUANG, Niu, Beijing 102206 (CN); PENG, Shiming, Beijing 102206 (CN)
(74) Representative: Zwicker, Jörk
(86) International application number: PCT/CN2016/086340
(87) International publication number: WO 2016/206573

(56) References cited:
- WO-A1-2008/119793
- WO-A1-2014/082544

## Description

### Introduction

Obesity is a severe health problem worldwide and many factors contribute to this chronic disease, including environmental factors and genetic factors. Genome-wide association studies to investigate patients with obesity revealed a gene for FTO (fat mass and obesity) to strongly associate with obesity. FTO's functional role in obesity was confirmed in transgenic animal models, such as FTO knockout mouse, FTO-overexpression mouse and FTO-I367F mutation mouse. FTO protein is an α-ketoglutarate and iron (II) dependent nucleic acid demethylase. Its preferred substrate is N6-meA in message RNA, which locates near the stop codon and influences gene translation.

We disclosed in US2014/0148383A1 identification of a known FDA approved drug - entacapone as an FTO inhibitor using structure-based virtual screening method in combination with biological activity measurements, including enzymatic activity, cellular activity and in high-fat diet induced obesity (DIO) animal model. Entacapone is a COMT (Catechol-O-methyltransferase) inhibitor used for treating Parkinson disease.

We synthesized numerous derivative and analogs, however activity assays revealed many substitutions reduced or obliterated FTO inhibitory activity, discouraging conventional SAR investigation. Undeterred we pursued a radical derivitization program introducing disruptive functional groups. Here we disclose a novel structural class of FTO inhibitors, composition and methods of use.

EP1978014 discloses processes for preparing entacapone (I) by demethylation of dimethoxy-entacapone (II), wherein II may be prepared by reacting a hydroxyl intermediary (III) with MHB(OCOR)₃. This hydroxyl intermediary is coincidentally structurally related to some of the subject compounds.

WO 2008/119793 discloses 2-cyano-3-(3,4-dimethoxy-5-nitrophenyl)-*N,N*-diethyl-3-hydroxyacrylamide and *E2*-cyano-3-3,4-dimethoxy-5-nitrophenyl)-*N,N*-diethylacrylamide for use in the treatment of Parkinson's disease.

WO 2014/082544 discloses a method for for inhibiting FTO comprising administering to a patient an effective amount of (2E)-2-cyano-3-(3,4-dihydroxy-5-nitrophenyl)-*N,N*-diethylprop-2-enamide.

The pharmaceutical compositions of the invention comprising the specified compounds are not known from the prior art.

### Summary of the Invention

The invention providespharmaceutical compositions and these compositions for use in inhibiting FTO and in treating a disease associated with excess FTO activity, including obesity, obesity-related diseases and Alzheimer's disease. In one aspect the invention provides a pharmaceutical composition an FTO inhibitor selected from a compound formula I, a stereoisomer thereof, a hydrate thereof, and a pharmaceutically-acceptable salt thereof, formulated in a unit dosage form: 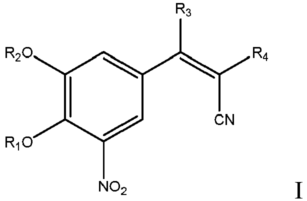 wherein :
R1 and R2 are independently H or Me;
R3 is OH or NHR, wherein R is H or an optionally substituted, optionally hetero-, optionally cyclic C1-C18 hydrocarbyl; and
R4 is optionally substituted, optionally hetero-, optionally cyclic C1-C18 hydrocarbyl;

In embodiments of the composition the optionally substituted, optionally hetero-, optionally cyclic C1-C18 hydrocarbyl in each instance is an optionally substituted C1-C9 alkyl, C2-C9 alkenyl, C2-C9 alkynyl, or C5-C14 aryl hydrocarbon, comprising 1-5 heteroatoms that are N, S, O or P, including 1-5 nitrogen atoms, or a heteroatom substituted with the hydrocarbon.

In embodiments of the inhibitor or composition:
one or both R1 and R2 is H;
R3 is OH; and/or
R is H or C1-C4 alkyl.

In embodiments the inhibitor is of the following Tables. We measured compound inhibition activity in a demethylation reaction catalyzed by FTO (US2014/0148383A1). The reaction system was incubated at 37 °C for 2 h and stopped by heating at 95 °C for 5 min. ssDNA was digested by nuclease P1 and alkaline phosphatase. The concentrations of N⁶-mA and A were analyzed by HPLC-MS/MS. When concentration of substrate and enzyme are 0.5 µM and 0.1 µM, respectively, the measured IC₅₀ value of entacapone against FTO is ∼ 3 µM.

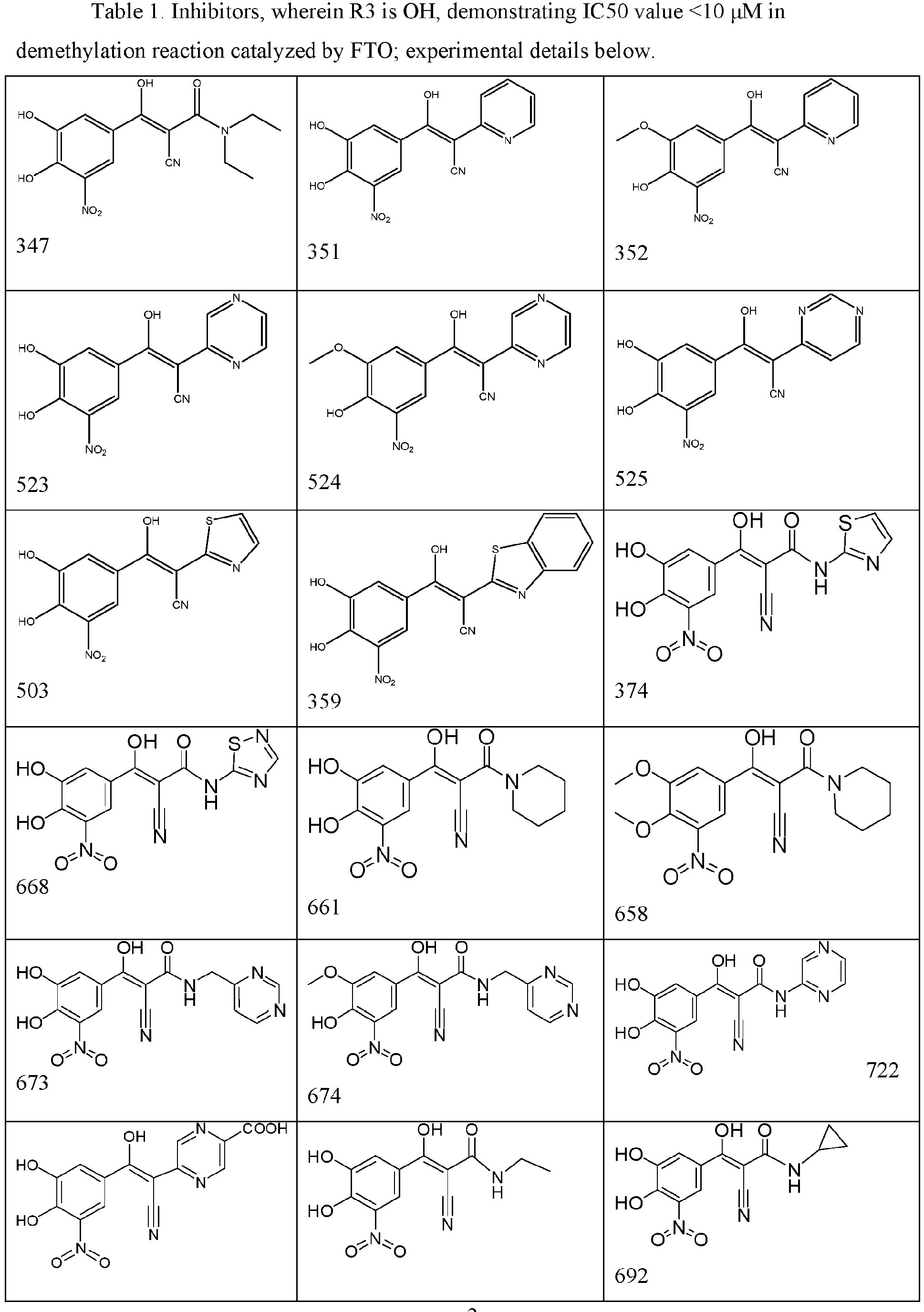

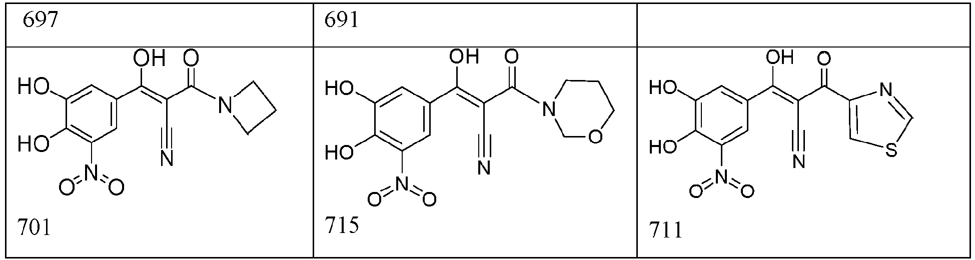

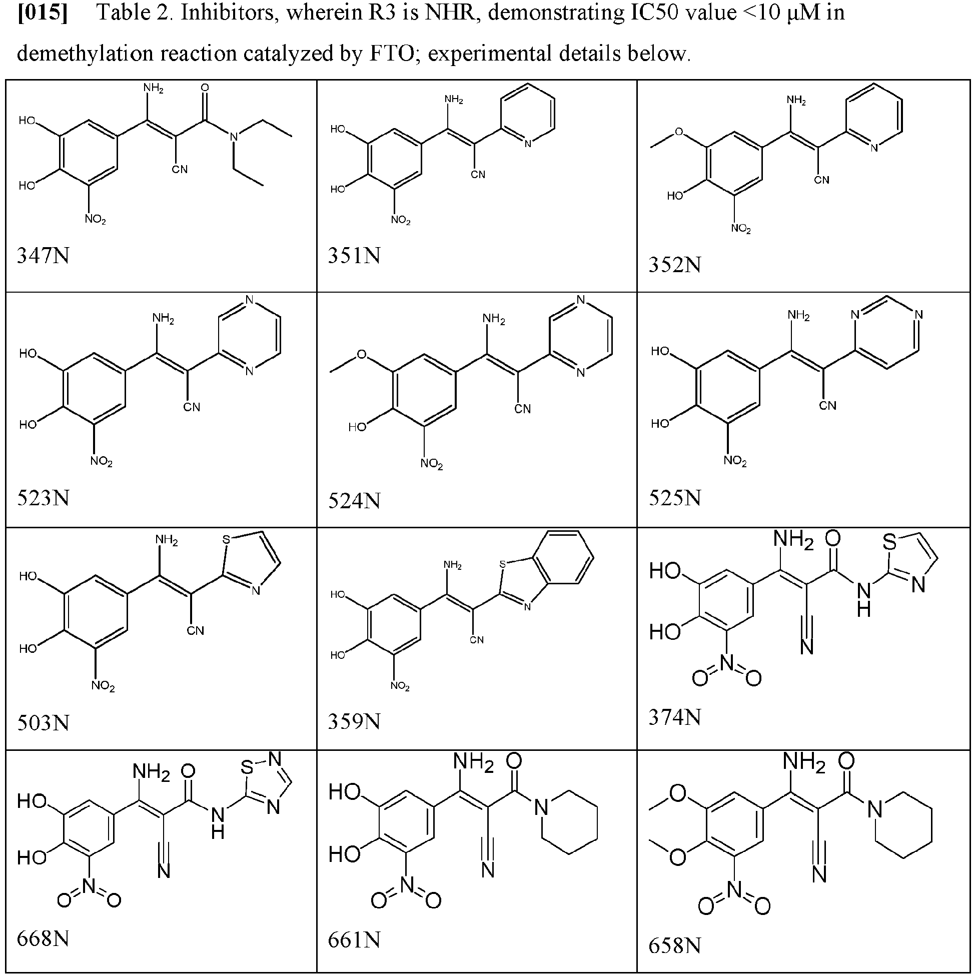

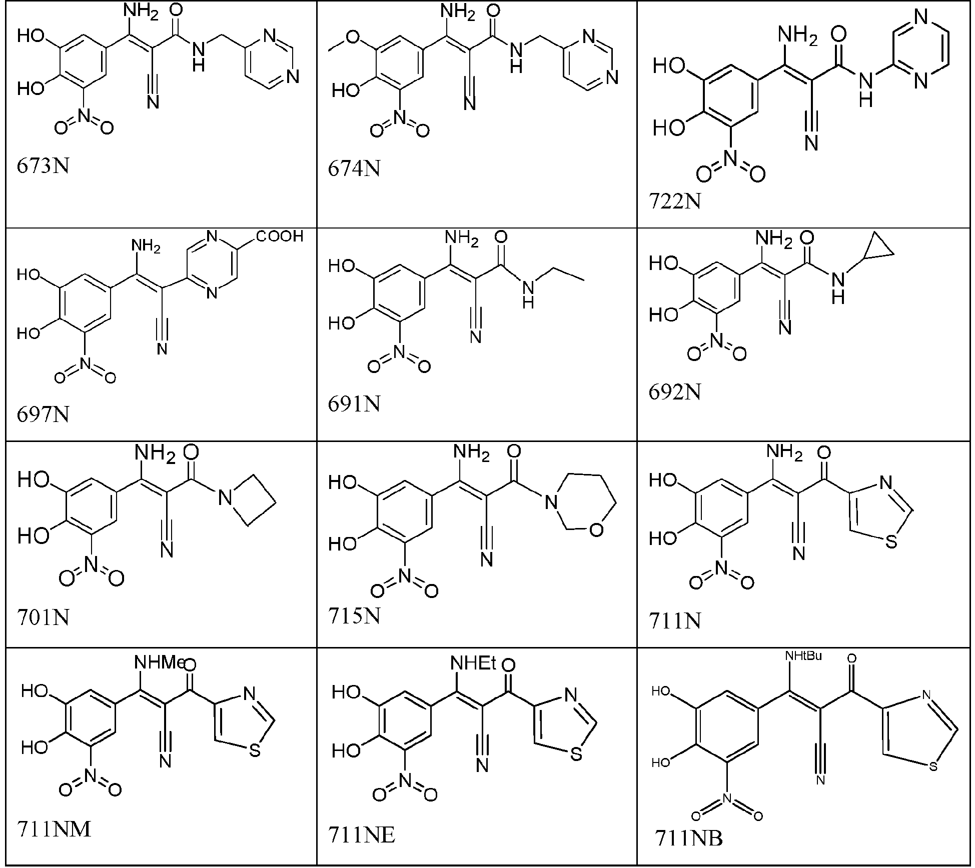

**Table 3. Other inhibitors not forming part of the present invention, wherein R4 is CONHR5, demonstrating IC50 value <10 µM in demethylation reaction catalyzed by FTO; experimental details below.**

| | | |
|---|---|---|
| 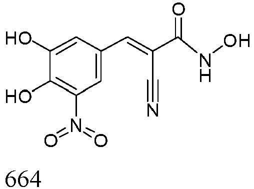 | 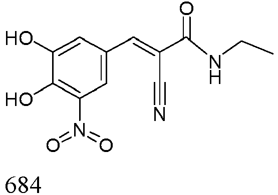 | 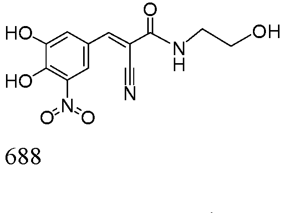 |
| 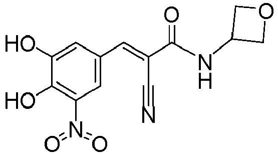 | 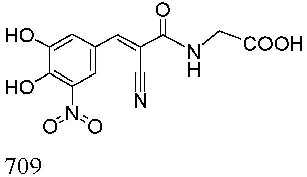 | 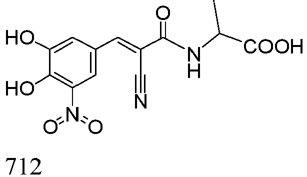 |
| 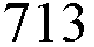 | | |
| 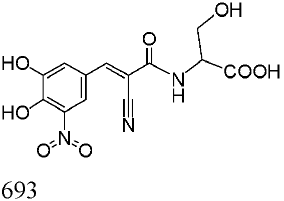 | 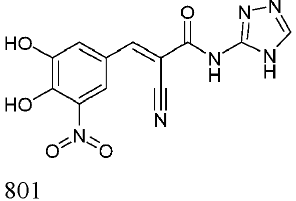 | 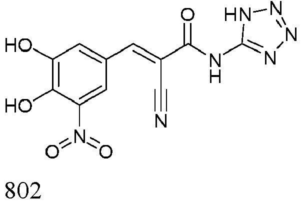 |
| 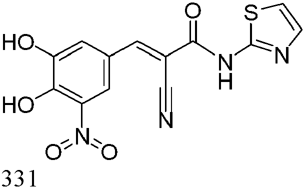 | 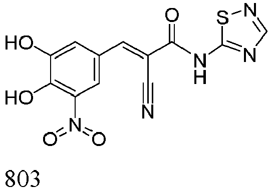 | 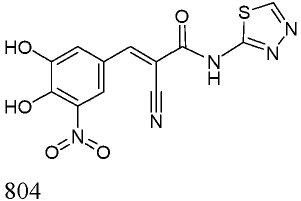 |
| 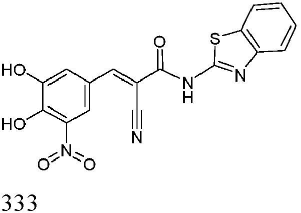 | 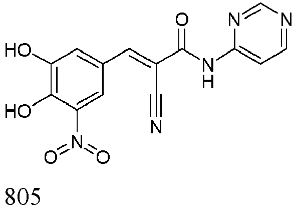 | 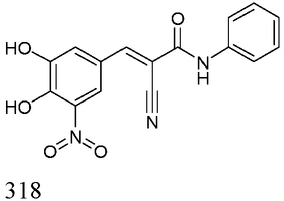 |
| 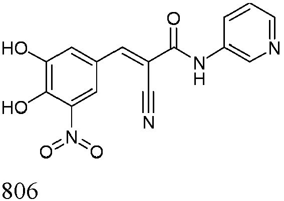 | 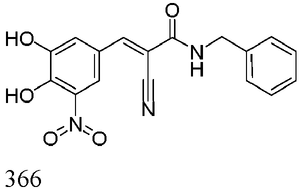 | 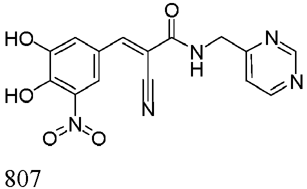 |
| 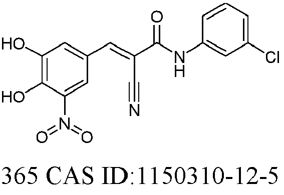 | 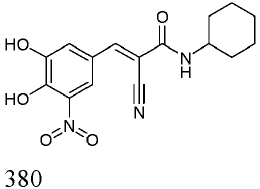 | 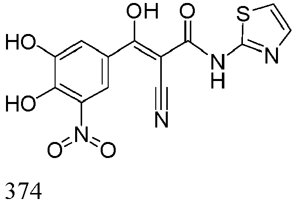 |
| 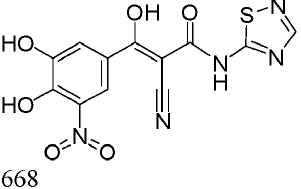 | 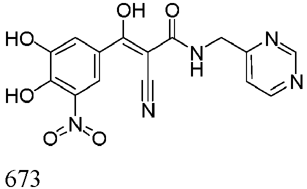 | 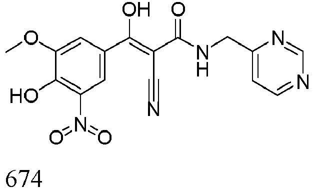 |
| 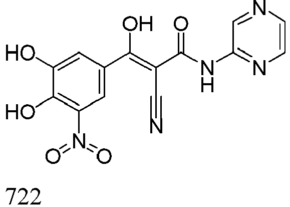 | 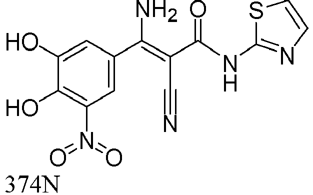 | 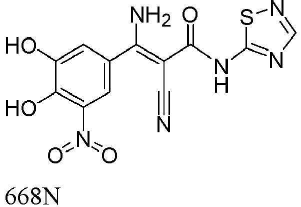 |
| 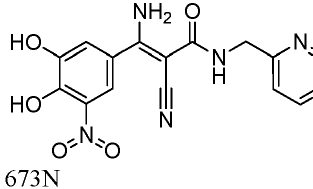 | 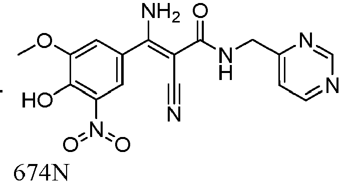 | 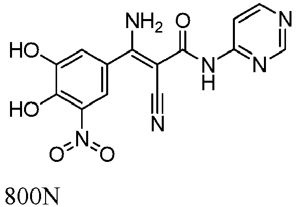 |
| 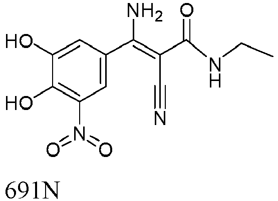 | 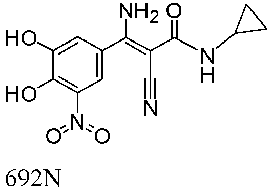 | 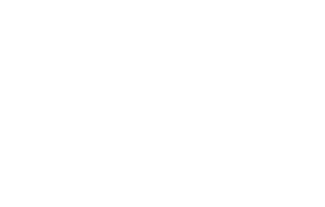 |

**Table 4. not forming part of the present invention inhibitors, wherein R4 is COR5, demonstrating IC50 value <10 µM in demethylation reaction catalyzed by FTO; experimental details below.**

| | | |
|---|---|---|
| 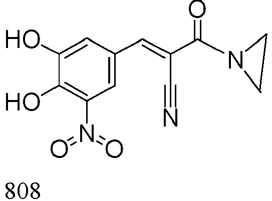 | 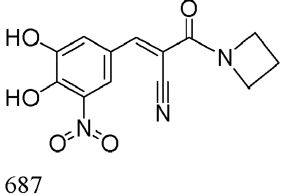 | 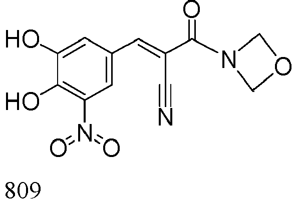 |
| 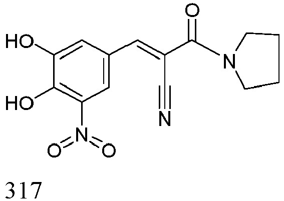 | 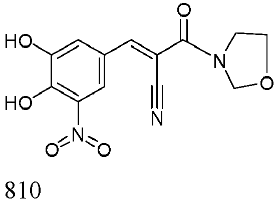 | 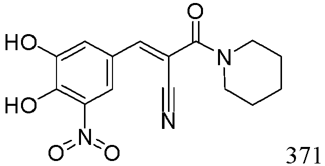 |
| | | CAS ID: 1150310-15-8 |
| 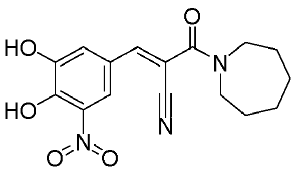 | 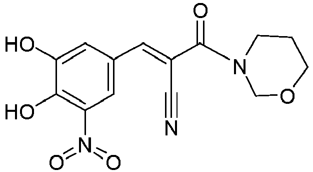 | 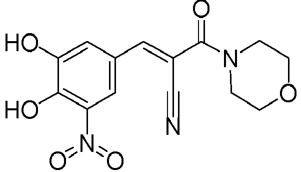 |
| 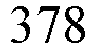 | 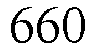 | 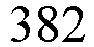 |
| 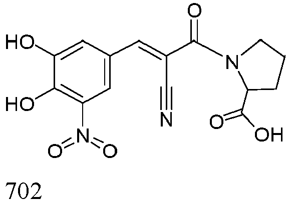 | 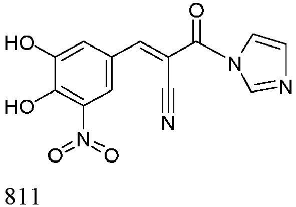 | 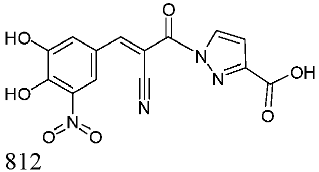 |
| 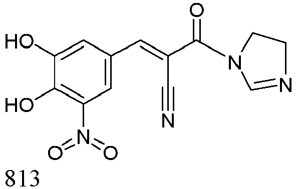 | 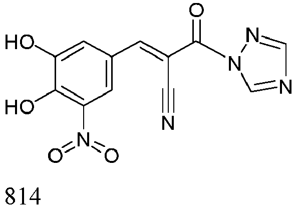 | 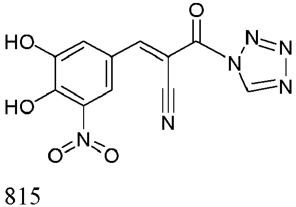 |
| 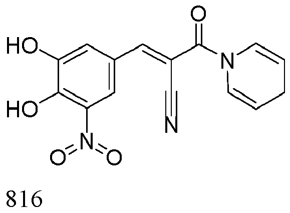 | 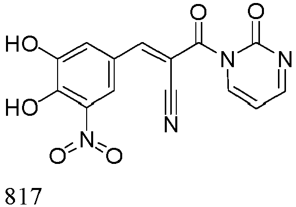 | 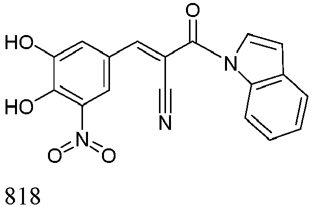 |
| 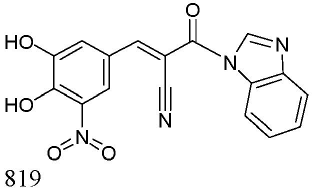 | 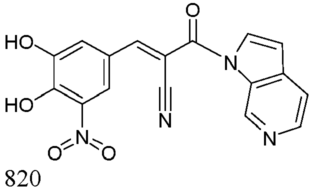 | 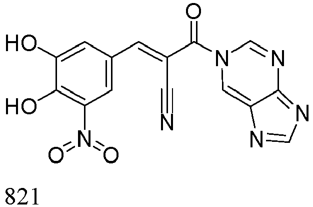 |
| 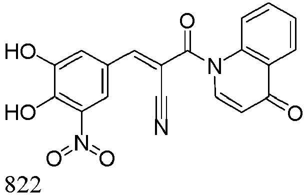 | 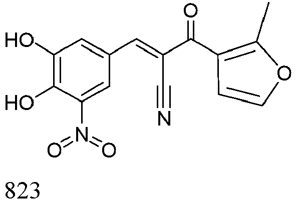 | 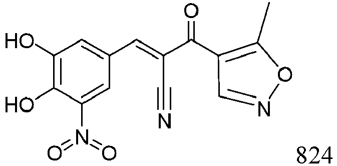 |
| 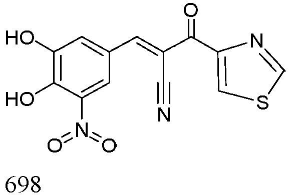 | 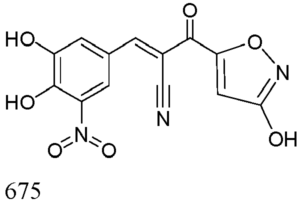 | 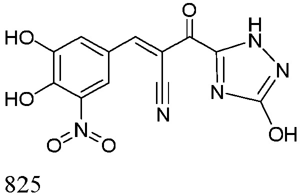 |
| 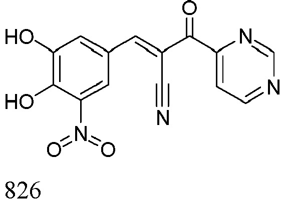 | 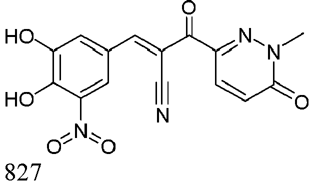 | 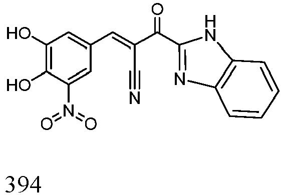 |
| 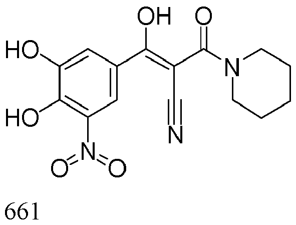 | 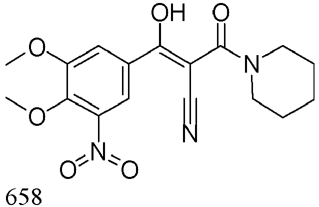 | 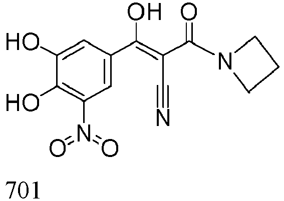 |
| 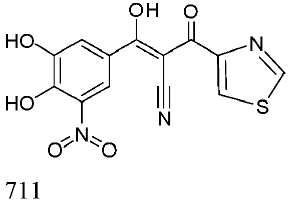 | 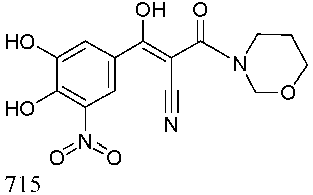 | 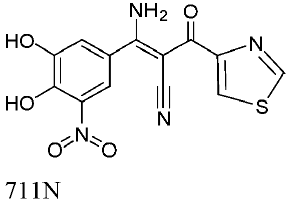 |
| 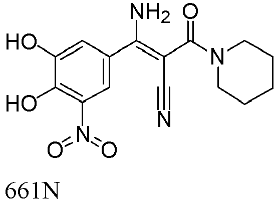 | 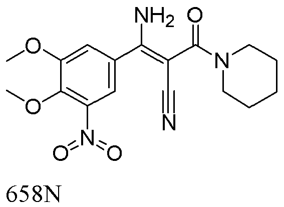 | 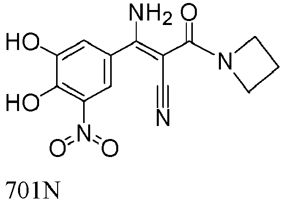 |
| 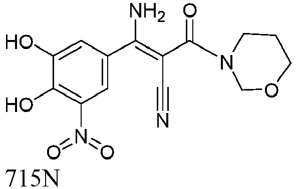 | | |

**Table 5. not forming part of the present invention inhibitors, wherein R4 is heterocyclic, demonstrating IC50 value <10 µM in demethylation reaction catalyzed by FTO; experimental details below.**

| | | |
|---|---|---|
| 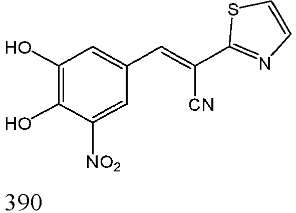 | 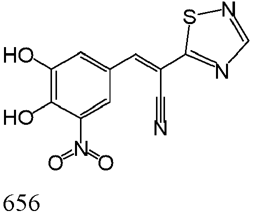 | 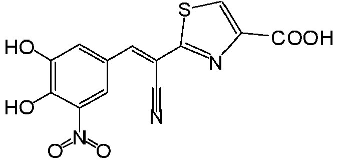 |
| | | 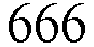 |
| 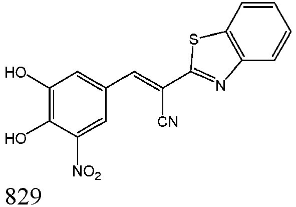 | 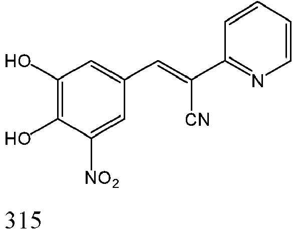 | 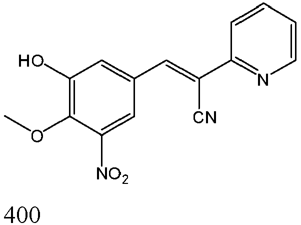 |
| 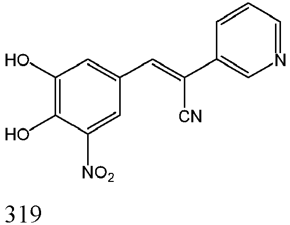 | 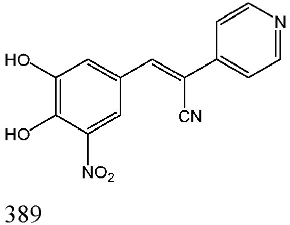 | 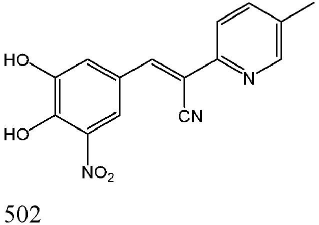 |
| 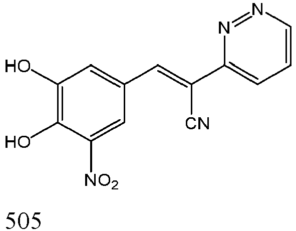 | 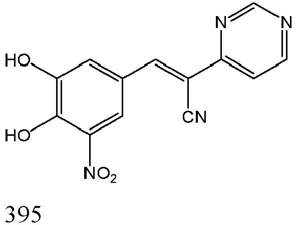 | 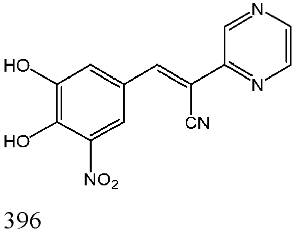 |
| 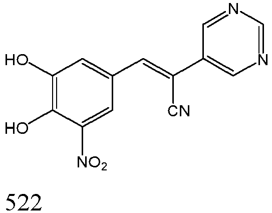 | 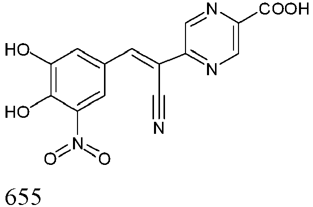 | 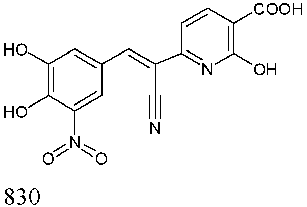 |
| 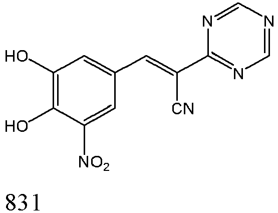 | 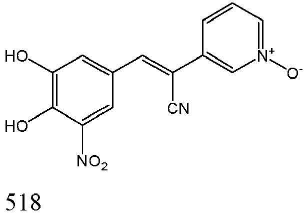 | 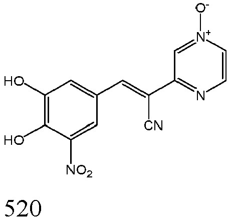 |
| 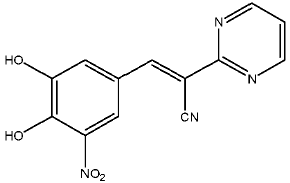 | 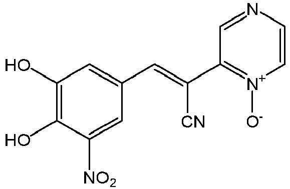 | 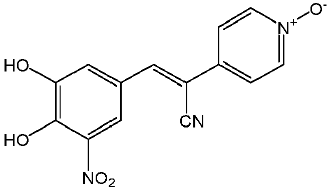 |
| 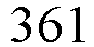 | 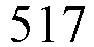 | 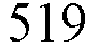 |
| CAS ID: 143542-72-7 | | |
| 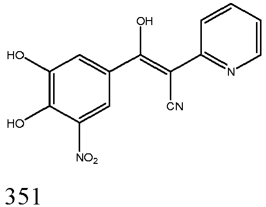 | 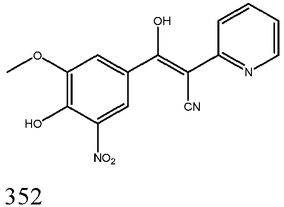 | 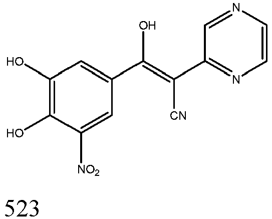 |
| 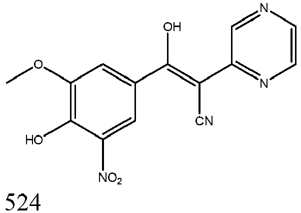 | 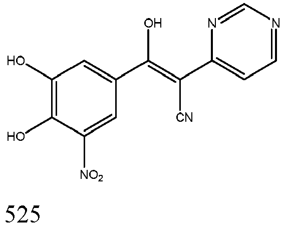 | 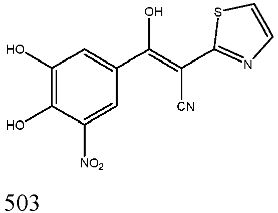 |
| 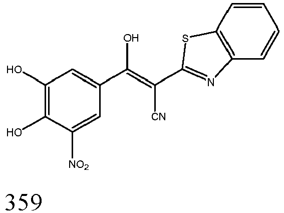 | 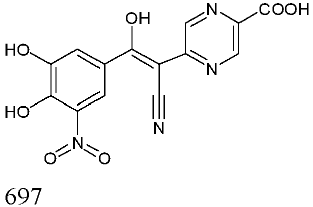 | 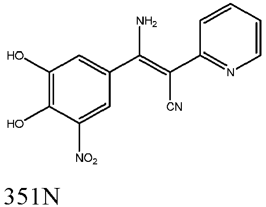 |
| 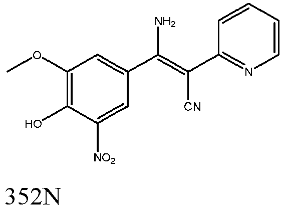 | 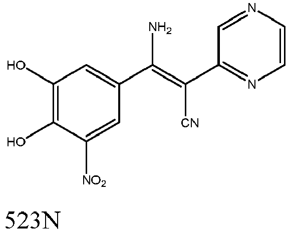 | 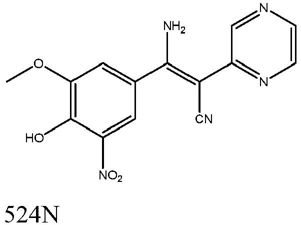 |
| 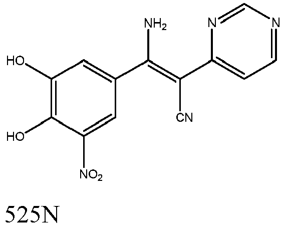 | 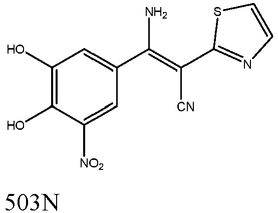 | 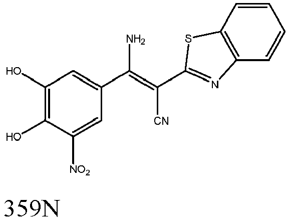 |
| 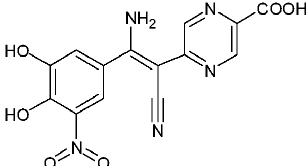 | | |
| 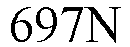 | | |

The pharmaceutical composition is preferably suitable for administration to a human.

The compositions may comprise a pharmaceutically-acceptable excipient, be in effective, unit dosage form, and/or comprise another, different therapeutic agents for the targeted disease or condition. In embodiments, the compositions may further comprise or be copackaged or coformulated with a second, different medicament for inhibiting weight gain, promoting weight loss, reducing serum LDL, cholesterol, LDL-c, or triglycerides, or treating obesity or an obesity related disease (esp. obesity-related diabetes, hyperglycemia, diabetic nephropathy, hyperlipemia, coronary heart disease, atherosclerosis, hypertension, cardiovascular or cerebrovascular disease) or Alzheimer's disease.

The medicament can be an AD drug that is an acetylcholinesterase inhibitor (esp. tacrine, rivastigmine, galantamine and donepezil) or an NMDA receptor antagonist (esp. memantine);

According to one embodiment, the pharmaceutical compositon is for use in inhibiting weight gain, promoting weight loss, reducing serum LDL, cholesterol, LDL-c, or triglycerides, or treating obesity, an obesity related disease or Alzheimer's disease.;

Further aspects and embodiments are disclosed in the accompanying claims.

Also disclosed but not forming part of the present invention are methods of treating a person in need thereof with an effective amount of the subject inhibitor or pharmaceutical composition, and optionally, detecting a resultant improvement in the person's health or condition. The methods may also optionally include the antecedent step of determining that the person, particularly diagnosing and applicable disease or condition (herein). The invention describes methods and uses of a subject inhibitor or composition in a person in need thereof, to inhibit FTO, inhibit weight gain, promote weight loss, reduce serum LDL, cholesterol, LDL-c, or triglycerides, or treat obesity or an obesity related disease or Alzheimer's Disease.

The invention encompasses all combination of the particular embodiments recited herein, as if each had been separately, laboriously recited. For example, the inhibitor may encompass combinations wherein: R1 and R2 are H; R3 is NH₂; and R4 is a 6 membered ring that is pyridine.

### Description of Particular Embodiments of the Invention

The following descriptions of particular embodiments and examples are provided by way of illustration and not by way of limitation. Those skilled in the art will readily recognize a variety of noncritical parameters that could be changed or modified to yield essentially similar results.

Unless contraindicated or noted otherwise, in these descriptions and throughout this specification, the terms "a" and "an" mean one or more, the term "or" means and/or and polynucleotide sequences are understood to encompass opposite strands as well as alternative backbones described herein. Furthermore, genuses are recited as shorthand for a recitation of all members of the genus; for example, the recitation of (C1-C3) alkyl is shorthand for a recitation of all C1-C3 alkyls: methyl, ethyl and propyl, including isomers thereof.

A hydrocarbyl group is a substituted or unsubstituted, straight-chain, branched or cyclic alkyl, alkenyl, alkynyl, acyl, aryl, arylalkyl, arylalkenyl, arylalkynyl, alkylaryl, alkenylaryl or alkynylaryl group which comprises 1-15 carbon atoms and optionally includes one or more heteroatoms in its carbon skeleton.

The term "heteroatom" as used herein generally means any atom other than carbon or hydrogen. Preferred heteroatoms include oxygen (O), phosphorus (P), sulfur (S), nitrogen (N), and halogens, and preferred heteroatom functional groups are haloformyl, hydroxyl, aldehyde, amine, azo, carboxyl, cyanyl, thocyanyl, carbonyl, halo, hydroperoxyl, imine, aldimine, isocyanide, iscyante, nitrate, nitrile, nitrite, nitro, nitroso, phosphate, phosphono, sulfide, sulfonyl, sulfo, and sulfhydryl.

The term "alkyl," by itself or as part of another substituent, means, unless otherwise stated, a straight or branched chain, or cyclic hydrocarbon radical, or combination thereof, which is fully saturated, having the number of carbon atoms designated (i.e. C1-C8 means one to eight carbons). Examples of alkyl groups include methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl and the like.

The term "alkenyl", by itself or as part of another substituent, means a straight or branched chain, or cyclic hydrocarbon radical, or combination thereof, which may be mono- or polyunsaturated, having the number of carbon atoms designated (i.e. C2-C8 means two to eight carbons) and one or more double bonds. Examples of alkenyl groups include vinyl, 2-propenyl, crotyl, 2-isopentenyl, 2-(butadienyl), 2,4-pentadienyl, 3-(1,4-pentadienyl) and higher homologs and isomers thereof.

The term "alkynyl", by itself or as part of another substituent, means a straight or branched chain hydrocarbon radical, or combination thereof, which may be mono- or polyunsaturated, having the number of carbon atoms designated (i.e. C2-C8 means two to eight carbons) and one or more triple bonds. Examples of alkynyl groups include ethynyl, 1- and 3-propynyl, 3-butynyl and higher homologs and isomers thereof.

The term "alkylene" by itself or as part of another substituent means a divalent radical derived from alkyl, as exemplified by -CH₂-CH₂-CH₂-CH₂-. Typically, an alkyl (or alkylene) group will have from 1 to 24 carbon atoms, with those groups having 10 or fewer carbon atoms being preferred in the invention. A "lower alkyl" or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and from one to three heteroatoms selected from the group consisting of O, N, P, Si and S, wherein the nitrogen, sulfur, and phosphorous atoms may optionally be oxidized and the nitrogen heteroatom may optionally be quaternized. The heteroatom(s) O, N, P and S may be placed at any interior position of the heteroalkyl group. The heteroatom Si may be placed at any position of the heteroalkyl group, including the position at which the alkyl group is attached to the remainder of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, and -CH=CH-N(CH3)-CH₃. Up to two heteroatoms may be consecutive, such as, for example, -CH₂-NH-OCH₃ and -CH₂-O-Si(CH₃)₃.

Similarly, the term "heteroalkylene," by itself or as part of another substituent means a divalent radical derived from heteroalkyl, as exemplified by -CH₂-CH₂-S-CH₂-CH₂- and-CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied.

The terms "cycloalkyl" and "heterocycloalkyl", by themselves or in combination with other terms, represent, unless otherwise stated, cyclic versions of "alkyl" and "heteroalkyl", respectively. Accordingly, a cycloalkyl group has the number of carbon atoms designated (i.e., C3-C8 means three to eight carbons) and may also have one or two double bonds. A heterocycloalkyl group consists of the number of carbon atoms designated and from one to three heteroatoms selected from the group consisting of O, N, Si and S, and wherein the nitrogen and sulfur atoms may optionally be oxidized and the nitrogen heteroatom may optionally be quaternized. Additionally, for heterocycloalkyl, a heteroatom can occupy the position at which the heterocycle is attached to the remainder of the molecule. Examples of cycloalkyl include cyclopentyl, cyclohexyl, 1-cyclohexenyl, 3-cyclohexenyl, cycloheptyl, and the like. Examples of heterocycloalkyl include 1-(1,2,5,6-tetrahydropyrid-yl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" and "halogen," by themselves or as part of another substituent, mean, unless otherwise stated, a fluorine, chlorine, bromine, or iodine atom. Additionally, terms such as "haloalkyl," are meant to include alkyl substituted with halogen atoms, which can be the same or different, in a number ranging from one to (2m'+1), where m' is the total number of carbon atoms in the alkyl group. For example, the term "halo(C1-C4)alkyl" is mean to include trifluoromethyl, 2,2,2-trifluoroethyl, 4-chlorobutyl, 3-bromopropyl, and the like. Thus, the term "haloalkyl" includes monohaloalkyl (alkyl substituted with one halogen atom) and polyhaloalkyl (alkyl substituted with halogen atoms in a number ranging from two to (2m'+1) halogen atoms, where m' is the total number of carbon atoms in the alkyl group). The term "perhaloalkyl" means, unless otherwise stated, alkyl substituted with (2m'+1) halogen atoms, where m' is the total number of carbon atoms in the alkyl group. For example the term "perhalo(C1-C4)alkyl" is meant to include trifluoromethyl, pentachloroethyl, 1,1,1-trifluoro-2-bromo-2-chloroethyl and the like.

The term "acyl" refers to those groups derived from an organic acid by removal of the hydroxy portion of the acid. Accordingly, acyl is meant to include, for example, acetyl, propionyl, butyryl, decanoyl, pivaloyl, benzoyl and the like.

The term "aryl" means, unless otherwise stated, a polyunsaturated, typically aromatic, hydrocarbon substituent which can be a single ring or multiple rings (up to three rings) which are fused together or linked covalently. Non-limiting examples of aryl groups include phenyl, 1-naphthyl, 2-naphthyl, 4-biphenyl and 1,2,3,4-tetrahydronaphthalene.

The term heteroaryl," refers to aryl groups (or rings) that contain from zero to four heteroatoms selected from N, O, and S, wherein the nitrogen and sulfur atoms are optionally oxidized and the nitrogen heteroatom are optionally quaternized. A heteroaryl group can be attached to the remainder of the molecule through a heteroatom. Non-limiting examples of heteroaryl groups include 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl and 6-quinolyl.

For brevity, the term "aryl" when used in combination with other terms (e.g., aryloxy, arylthioxy, arylalkyl) includes both aryl and heteroaryl rings as defined above. Thus, the term "arylalkyl" is meant to include those radicals in which an aryl group is attached to an alkyl group (e.g., benzyl, phenethyl, pyridylmethyl and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like).

Each of the above terms (e.g., "alkyl," "heteroalkyl," "aryl" and "heteroaryl") is meant to include both substituted and unsubstituted forms of the indicated radical. Preferred substituents for each type of radical are provided below.

Substituents for the alkyl and heteroalkyl radicals (as well as those groups referred to as alkylene, alkenyl, heteroalkylene, heteroalkenyl, alkynyl, cycloalkyl, heterocycloalkyl, cycloalkenyl and heterocycloalkenyl) can be a variety of groups selected from: -OR', =O, =NR', =N-OR', -NR'R", -SR', halogen, -SiR'R"R''', -OC(O)R', -C(O)R', -CO₂R', -CONR'R",-OC(O)NR'R", -NR"C(O)R', -NR'-C(O)NR"R''', -NR'-SO₂NR''', -NR"CO₂R', -NH-C(NH₂)=NH, -NR'C(NH₂)=NH, -NH-C(NH₂)=NR', -S(O)R', -SO₂R', -SO₂NR'R", -NR"SO₂R, -CN and -NO₂, in a number ranging from zero to three, with those groups having zero, one or two substituents being particularly preferred. R', R" and R''' each independently refer to hydrogen, unsubstituted (C1-C8)alkyl and heteroalkyl, unsubstituted aryl, aryl substituted with one to three halogens, unsubstituted alkyl, alkoxy or thioalkoxy groups, or aryl-(C1-C4)alkyl groups. When R' and R" are attached to the same nitrogen atom, they can be combined with the nitrogen atom to form a 5-, 6- or 7-membered ring. For example, -NR'R" is meant to include 1-pyrrolidinyl and 4-morpholinyl. Typically, an alkyl or heteroalkyl group will have from zero to three substituents, with those groups having two or fewer substituents being preferred in the invention. More preferably, an alkyl or heteroalkyl radical will be unsubstituted or monosubstituted. Most preferably, an alkyl or heteroalkyl radical will be unsubstituted. From the above discussion of substituents, one of skill in the art will understand that the term "alkyl" is meant to include groups such as trihaloalkyl (e.g., -CF₃ and -CH₂CF₃).

Preferred substituents for the alkyl and heteroalkyl radicals are selected from: -OR', =O, -NR'R", -SR', halogen, -SiR'R"R''', -OC(O)R', -C(O)R', -CO₂R', -CONR'R",-OC(O)NR'R", -NR"C(O)R', -NR"CO₂R', -NR'-SO₂NR"R''', -S(O)R', -S02R', -SO₂NR'R",-NR"SO₂R, -CN and -NO₂, where R' and R" are as defined above. Further preferred substituents are selected from: -OR', =O, -NR'R", halogen, -OC(O)R', -CO₂R', -CONR'R",-OC(O)NR'R", -NR"C(O)R', -NR"CO₂R', -NR'-SO₂NR"R''', -SO₂R', -SO₂NR'R", -NR"SO₂R,-CN and -NO₂.

Similarly, substituents for the aryl and heteroaryl groups are varied and selected from: halogen, -OR', -OC(O)R', -NR'R", -SR', -R', -CN, -NO₂, -CO₂R', -CONR'R", -C(O)R',-OC(O)NR'R", -NR"C(O)R', -NR"CO2R', -NR'-C(O)NR"R''', -NR'-SO₂NR"R''', -NH-C(NH2)=NH, -NR'C(NH₂)=NH, -NH-C(NH₂)=NR', -S(O)R', -SO₂R', -SO₂NR'R", -NR"SO₂R, -N₃, -CH(Ph)₂, perfluoro(C1-C4)alko- xy and perfluoro(C1-C4)alkyl, in a number ranging from zero to the total number of open valences on the aromatic ring system; and where R', R" and R''' are independently selected from hydrogen, (C1-C8)alkyl and heteroalkyl, unsubstituted aryl and heteroaryl, (unsubstituted aryl)-(C1-C4)alkyl and (unsubstituted aryl)oxy-(C1-C4)alkyl. When the aryl group is 1,2,3,4-tetrahydronaphthalene, it may be substituted with a substituted or unsubstituted (C3-C7)spirocycloalkyl group. The (C3-C7)spirocycloalkyl group may be substituted in the same manner as defined herein for "cycloalkyl". Typically, an aryl or heteroaryl group will have from zero to three substituents, with those groups having two or fewer substituents being preferred in the invention. An aryl or heteroaryl group can be unsubstituted or monosubstituted. Alternatively, an aryl or heteroaryl group can be unsubstituted.

Preferred substituents for aryl and heteroaryl groups are selected from: halogen, -OR', -OC(O)R', -NR'R", -SR', -R', -CN, -NO₂, -CO₂R', -CONR'R", -C(O)R',-OC(O)NR'R",-NR"C(O)R', -S(O)R', -SO₂R', -SO₂NR'R", -NR"SO₂R, -N₃, -CH(Ph)₂, perfluoro(C1-C4)alkoxy and perfluoro(C1-C4)alkyl, where R' and R" are as defined above. Further preferred substituents are selected from: halogen, -OR', -OC(O)R', -NR'R", -R', -CN, -NO₂,-CO₂R', -CONR'R", -NR"C(O)R', -SO₂R', -SO₂NR'R", -NR"SO₂R, perfluoro(C1-C4)alkoxy and perfluoro(C1-C4)alkyl.

The substituent -CO₂H, as used herein, includes bioisosteric replacements therefor; see, e.g., The Practice of Medicinal Chemistry; Wermuth, C. G., Ed.; Academic Press: New York, 1996; p. 203.

Two of the substituents on adjacent atoms of the aryl or heteroaryl ring may optionally be replaced with a substituent of the formula -T-C(O)-(CH₂)q-U-, wherein T and U are independently -NH-, -O-, -CH₂- or a single bond, and q is an integer of from 0 to 2. Alternatively, two of the substituents on adjacent atoms of the aryl or heteroaryl ring may optionally be replaced with a substituent of the formula -A-(CH2)r-B-, wherein A and B are independently -CH₂-, -O-, -NH-, -S-, -S(O)-, -S(O)₂-, -S(O)₂NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)s-X-(CH₂)t- -, where s and t are independently integers of from 0 to 3, and X is -O-, -NR'-, -S-,-S(O)-, -S(O)₂-, or -S(O)₂NR'-. The substituent R' in -NR'- and -S(O)₂NR'- is selected from hydrogen or unsubstituted (C1-C6)alkyl.

Preferred substituents are disclosed herein and exemplified in the tables, structures, examples, and claims, and may be applied across different compounds as disclosed herein, i.e. substituents of any given compound may be combinatorially used with other compounds.

Applicable substituents can be independently substituted or unsubstituted heteroatom, substituted or unsubstituted, optionally heteroatom C1-C6 alkyl, substituted or unsubstituted, optionally heteroatom C2-C6 alkenyl, substituted or unsubstituted, optionally heteroatom C2-C6 alkynyl, or substituted or unsubstituted, optionally heteroatom C6-C14 aryl, wherein each heteroatom is independently oxygen, phosphorus, sulfur or nitrogen.

In particular, applicable substituents can be independently aldehyde, aldimine, alkanoyloxy, alkoxy, alkoxycarbonyl, alkyloxy, alkyl, amine, azo, halogens, carbamoyl, carbonyl, carboxamido, carboxyl, cyanyl, ester, halo, haloformyl, hydroperoxyl, hydroxyl, imine, isocyanide, iscyante, N-tert-butoxycarbonyl, nitrate, nitrile, nitrite, nitro, nitroso, phosphate, phosphono, sulfide, sulfonyl, sulfo, sulfhydryl, thiol, thiocyanyl, trifluoromethyl or trifluromethyl ether (OCF3).

The term "pharmaceutically acceptable salts" is meant to include salts of the active compounds which are prepared with relatively nontoxic acids or bases, depending on the particular substituents found on the compounds described herein, and suitable for pharmaceutical use. When the disclosed compounds contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When the disclosed compounds contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, oxalic, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds as disclosed herein contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the invention.

In addition to salt forms, the invention provides compounds which are in a prodrug form. Prodrugs of the compounds described herein are those compounds that undergo chemical changes under physiological conditions to provide the compounds disclosed herein. Additionally, prodrugs can be converted to the compounds disclosed herein by chemical or biochemical methods in an ex vivo environment. For example, prodrugs can be slowly converted to the disclosed compounds when placed in a transdermal patch reservoir with a suitable enzyme or chemical reagent. Prodrugs are often useful because, in some situations, they may be easier to administer than the parent drug. They may, for instance, be more bioavailable by oral administration than the parent drug. The prodrug may also have improved solubility in pharmacological compositions over the parent drug. A wide variety of prodrug derivatives are known in the art, such as those that rely on hydrolytic cleavage or oxidative activation of the prodrug. An example, without limitation, of a prodrug would be a compound as disclosed herein which is administered as an ester (the "prodrug"), but then is metabolically hydrolyzed to the carboxylic acid, the active entity. Additional examples include peptidyl derivatives of a disclosed compound.

Certain compounds as described herein can exist in unsolvated forms as well as solvated forms, including hydrated forms. In general, the solvated forms are equivalent to unsolvated forms. Certain compounds as disclosed herein may exist in multiple crystalline or amorphous forms. In general, all physical forms are equivalent for the uses contemplated by the invention.

Some of the subject compounds possess asymmetric carbon atoms (optical centers) or double bonds; the racemates, diastereomers, geometric isomers and specifically designated or depicted chirality is preferred and in many cases critical for optimal activity.

The compounds disclosed herein may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds disclosed herein, whether radioactive or not, are intended to be also disclosed.

The term "therapeutically effective amount" refers to the amount of the subject compound that will elicit, to some significant extent, the biological or medical response of a tissue, system, animal or human that is being sought by the researcher, veterinarian, medical doctor or other clinician, such as when administered, is sufficient to prevent development of, or alleviate to some extent, one or more of the symptoms of the condition or disorder being treated. The therapeutically effective amount will vary depending on the compound, the disease and its severity and the age, weight, etc., of the mammal to be treated.

The invention particularly provides pharmaceutical compositions comprising the subject compounds and a pharmaceutically acceptable excipient, particularly such compositions comprising a unit dosage of the subject compounds, particularly such compositions copackaged with instructions describing use of the composition to treat an applicable disease or condition (herein).

The compositions for administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules, lozenges or the like in the case of solid compositions. In such compositions, the compound is usually a minor component (from about 0.1 to about 50% by weight or preferably from about 1 to about 40% by weight) with the remainder being various vehicles or carriers and processing aids helpful for forming the desired dosing form.

Suitable excipients or carriers and methods for preparing administrable compositions are known or apparent to those skilled in the art and are described in more detail in such publications as Remington's Pharmaceutical Science, Mack Publishing Co, NJ (1991). In addition, the compounds may be advantageously used in conjunction with other therapeutic agents as described herein or otherwise known in the art, particularly other anti-necrosis agents. Hence the compositions may be administered separately, jointly, or combined in a single dosage unit.

The amount administered depends on the compound formulation, route of administration, etc. and is generally empirically determined in routine trials, and variations will necessarily occur depending on the target, the host, and the route of administration, etc. Generally, the quantity of active compound in a unit dose of preparation may be varied or adjusted from about 1, 5, 25 or 100 to about 5, 25, 100, 500, 1000 or 2000 mg, according to the particular application. Unit dosage forms can be packaged in a multipack adapted for sequential use, such as blisterpack, comprising sheets of at least 6, 9 or 12 unit dosage forms. The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day if desired.

The compounds can be administered by a variety of methods including, but not limited to, parenteral, topical, oral, or local administration, such as by aerosol or transdermally, for prophylactic and/or therapeutic treatment. Also, in accordance with the knowledge of the skilled clinician, the therapeutic protocols (e.g., dosage amounts and times of administration) can be varied in view of the observed effects of the administered therapeutic agents on the patient, and in view of the observed responses of the disease to the administered therapeutic agents.

The therapeutics disclosed herein can be administered in a therapeutically effective dosage and amount, in the process of a therapeutically effective protocol for treatment of the patient. For more potent compounds, microgram (ug) amounts per kilogram of patient may be sufficient, for example, in the range of about 1, 10, 100, 1000, 10000, 20000 ug/kg to about 10, 100, 1000, 10000, 20000 or 80000 ug/kg of patient weight though optimal dosages are compound specific, and generally empirically determined for each compound.

In general, routine experimentation in clinical trials will determine specific ranges for optimal therapeutic effect, for each therapeutic, each administrative protocol, and administration to specific patients will also be adjusted to within effective and safe ranges depending on the patient condition and responsiveness to initial administrations. However, the ultimate administration protocol will be regulated according to the judgment of the attending clinician considering such factors as age, condition and size of the patient as well as compounds potency, severity of the disease being treated. For example, a dosage regimen of the compounds can be oral administration of from 10 mg to 2000 mg/day, preferably 10 to 1000 mg/day, more preferably 50 to 600 mg/day, in two to four (preferably two) divided doses. Intermittent therapy (e.g., one week out of three weeks or three out of four weeks) may also be used.

The person to be treated may have a genotype associated with obesity or pathogenic or medically-undesirable weight gain, such as SNP rs7202116 (G), rs1421085 (C), or rs9939609 (A), or a surrogate or proxy SNP in linkage disequilibrium therewith (with respect to the correlative phenotype; see references below) and having a r² value greater than 0.5; and/or (f) pathogenically expresses or over-expresses FTO or *Fto* (*e.g.* comprises and expresses a multi-copy fto gene). Re rs7202116 G, see e.g. Yang et al. , FTO genotype is associated with phenotypic variability of body mass index, Nature, Sep 16, 2012, doi: 10.1038/nature11401 [epub]; re rs9939609 A, see e.g. Freathy RM, et al (2008). "Common variation in the FTO gene alters diabetes-related metabolic traits to the extent expected, given its effect on BMI". Diabetes 57 (5): 1419-26. doi:10.2337/db07-1466. PMC 3073395. PMID 18346983; re rs1421085 C, see e.g. Dina C, et al., (2007). "Variation in FTO contributes to childhood obesity and severe adult obesity". Nature Genetics 39 (6): 724-6. doi:10.1038/ng2048. PMID 17496; and for multi-copy fto gene mouse, see e.g. Church et al., Overexpression of Fto leads to increased food intake and results in obesity, Nature Genetics, published online 14 Nov 2010, doi:10.1038/ng.713.

It is understood that the examples and embodiments described herein are for illustrative purposes only.

### Examples: Compound Preparation.

**Compound 347:** 347 was prepared in two synthetic steps from 3,4-dimethoxy-5-nitrobenzoic acid, according to the following procedure:

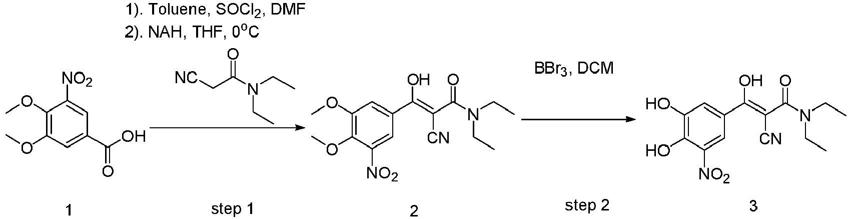

### Step1: Synthesis of 2-cyano-3-(3,4-dimethoxy-5-nitrophenyl)-N,N-diethyl-3-oxopropanamide (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (1.0 g, 4.4 mmol) in toluene (11 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (10 mL).

Under a nitrogen atmosphere, 60% NaH (0.35g, 8.8 mmol) was added to solution of 2-cyano-*N*,*N*-diethylacetamide (0.56 g, 4.0 mmol) in anhydrous THF (15 mL) at -5 °C. The resulting suspension was stirred at -5 °C for 15min and the THF solution of 3,4-dimethoxy-5-nitrobenzoyl chloride was added over 10min and stirred for an additional 1h at -5°C. The reaction mixture was warmed to 0 °C, quenched by the addition of 1N.HCl solution (4 mL) and stirred for 10min at room temperature, extracted by ethyl acetate (25 mL X 2), the organic layers was dried with Na₂SO₄ and concentrated in vacuo to give the title compound as an orange solid (705 mg, 99%). MS [MH]⁺ calcd for C₁₆H₁₉N₃O₆ 350.1, found 350.1.

### Step2: Synthesis of 2-cyano-3-(3,4-dihydroxy-5-nitrophenyl)-N,N-diethyl-3-oxopropanamide (3)

A solution of 2-cyano-3-(3,4-dimethoxy-5-nitrophenyl)-*N,N*-diethyl-3-oxopropanamide (500 mg, 1.43 mmol) in DCM (5 mL) was added 1.0 M solution of BBr₃ in DCM (5 mL, 5 mmol) at -15 °C under a nitrogen atmosphere. The resulting red suspension was stirred for 1h at -15 °C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried over Na₂SO₄. The solvent was eliminated under reduced pressure to give the crude product. Further purification by Prep-HPLC (0.5%TFA, MeOH/H₂O) gave the desired product as a bright yellow solid (80 mg, 17%). ¹H NMR (400 MHz, CDCl₃) δ 10.92 (s, 1H), 8.28 (d, *J* = 2.0 Hz, 1H), 7.74 (d, *J* = 1.9 Hz, 1H), 7.26 (s, 3H), 5.93 (s, 1H), 3.66 (d, *J* = 6.0 Hz, 3H), 1.33 (t, *J* = 7.0 Hz, 6H). MS [MH]⁺ calcd for C₁₄H₁₅N₃O₆ 322.0, found 322.0.

**Compound 315:** 315 was prepared in one synthetic step from 3,4-dihydroxy-5-nitrobenzaldehyde, according to the following procedure:

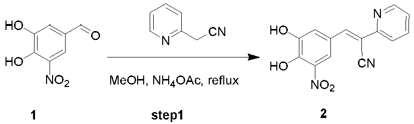

### Step1: Synthesis of (E)-3-(3,4-dihydroxy-5-nitrophenyl)-2-(pyridin-2-yl)acrylonitrile (2)

A solution of 2-(pyridin-2-yl)acetonitrile (142 mg, 1.2 mmol), 3,4-dihydroxy-5-nitrobenzaldehyde (182 mg, 1 mmol) and NH₄OAc (462 mg, 6 mmol) in MeOH (10 mL) was heated to reflux for overnight. LCMS showed no 3,4-dihydroxy-5-nitrobenzaldehyde left. The reaction mixture was cooled to room temperature. The solid was filtered and washed by MeOH and H₂O. The solid was re-dissolved in MeOH (5 mL). 5 mL of IN aqueous HCl was added to adjust pH 3∼4. The desired product was obtained by filter as a bright solid (56 mg, 20%). ¹H NMR (400 MHz, DMSO) δ 8.57 (m,, 1H), 8.12 (s, 1H), 7.92 (d, *J* = 2.2 Hz, 1H), 7.84 (td, *J* = 7.8, 1.8 Hz, 1H), 7.70 (d, *J* = 8.1 Hz, 1H), 7.56 (d, *J* = 2.4 Hz, 1H), 7.31-7.25 (m, 1H). MS [MH]⁺ calcd for C₁₄H₉N₃O₄ 284.0, found 284.0.

**Compound 361:** 361 was prepared in four synthetic steps from malonamide, according to the following procedure:

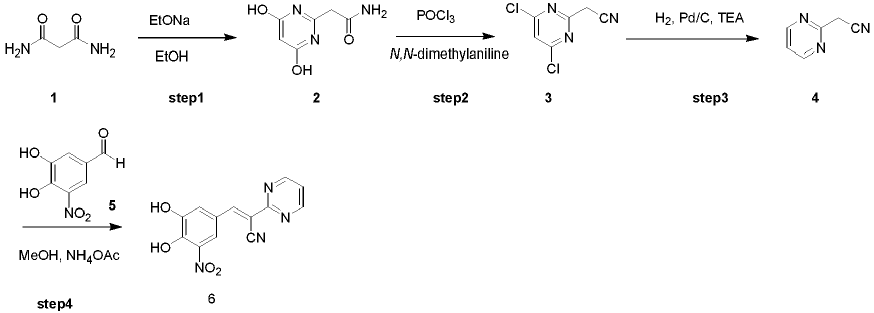

### Step1: Synthesis of 2-(4,6-dihydroxypyrimidin-2-yl)acetamide (2)

To a solution of NaOEt (21% in EtOH, 167 mL, 450 mmol) in EtOH (170 mL) was added malonamide (22.9 g, 224 mmol). After being refluxed for 2 hours, half of EtOH was removed under reduced pressure and the precipitated solid was filtered and dried under high vacuum for overnight. The dried solid sodium salt (24 g) was dissolved in ice-cold H₂O (70 mL) and brought to pH 2∼3 using 3N. HCl (50 mL), recrystallization from water gave 2-(4,6-dihydroxypyrimidin-2-yl)acetamide as a pale yellow solid (6.28 g, 33%).

### Step2: Synthesis of 2-(4,6-dichloropyrimidin-2-yl)acetonitrile (3)

To a solution of 2-(4,6-dihydroxypyrimidin-2-yl)acetamide (6.28 g, 37.1 mmol) in POCl₃ (19 mL, 204 mmol) was placed in a flask which was then attached to a reflux condenser. Through the condenser was added *N,N*-dimethylaniline (10 mL, 79 mmol). The mixture was warmed cautiously in an oil bath which is quickly removed when the reaction began. After the initial vigorous reaction had subsided, the reaction was refluxed for ten minutes longer. The hot material was poured over 100 g ice and the resulting suspension was extracted (DCM). The combined organic layers were dried (Na₂SO₄) and concentrated under reduced pressure. The product was purified by column chromatography (SiO₂, PE / EA = 4/1) to provide the desired product as a yellow solid (5.1 g, 27.1 mmol). MS [MH]⁺ calcd for C₆H₃Cl₂N₃ 189.0, found 189.0.

### Step3: Synthesis of 2-(pyrimidin-2-yl)acetonitrile (4)

To a solution of 2-(4,6-dichloropyrimidin-2-yl)acetonitrile (2.2 g, 11.7 mmol) and triethylamine (3.0 mL, 20.8 mmol) in ethyl acetate / MeOH (1/1, 40 mL) was added 10% Pd/C (400 mg) and the solution was vigorously stirred for 2.5 hours under H2 atmosphere (1 atm). The reaction was filtered through celite and washed the celite with MeOH. The combined filtrates were concentrated under reduced pressure and purified by flash chromatography (SiO₂, PE / EA =1/1) to give the 2-(pyrimidin-2-yl)acetonitrile as a pale red liquid (618 mg, 54%). MS [MH]⁺ calcd for C₆H₅N₃ 120.1, found 120.1.

### Step4: Synthesis of (E)-3-(3,4-dihydroxy-5-nitrophenyl)-2-(pyrimidin-2-yl)acrylonitrile (6)

A solution of 2-(pyrimidin-2-yl)acetonitrile (120 mg, 1 mmol), 3,4-dihydroxy-5-nitrobenzaldehyde (182 mg, 1 mmol) and NH₄OAc (462 mg, 6 mmol) in MeOH (10 mL) was heated to reflux for 5 hours. LCMS showed no starting materials left. The solid was filtered and washed by MeOH and H₂O, then dissolved in MeOH (5 mL). 5 mL of 1N.HCl was added to adjust pH 3∼4, the solid was filtered and dried in vacuo to give the desired product as a bright yellow solid (250 mg, 88%). ¹H NMR (400 MHz, DMSO) δ 8.78 (d, *J* = 4.8 Hz, 2H), 8.35 (s, 1H), 7.96 (d, *J* = 2.5 Hz, 1H), 7.59 (d, *J* = 2.5 Hz, 1H), 7.32 (t, *J* = 4.8 Hz, 1H). MS [MH]⁺ calcd for C₁₃H_{g}N₄O₄ 285.0, found 285.0.

**Compound 395:** 395 was prepared in four synthetic steps from 4-methylpyrimidine, according to the following procedure:

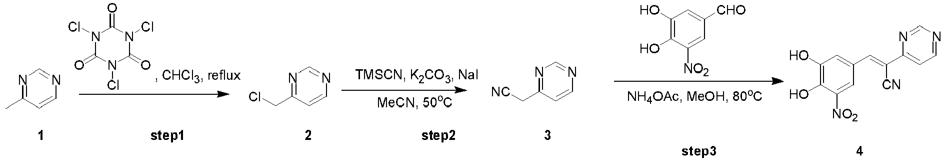

### Step1: Synthesis of 4-(chloromethyl)pyrimidine (2)

4-methylpyrimidine (53.1 mmol, 5 g) was dissolved in CHCl₃ (100 mL), the mixture was heated to 75°C, then 1,3,5-trichloro-1,3,5- triazinane-2,4,6-trione (26.6 mmol, 6.2 g) was added slowly in two portions. The mixture was stirred at 75 °C overnight. After the completion of the reaction, it was filtered and concentrated in vacco. The residue was purified by column chromatograph (silica gel, PE / EA = 30/1 to 10/1) to obtain the desired product (1.64 g, 24%) as a yellow oil. ¹H-NMR (400 MHz, CDCl₃) δ(ppm) 9.16 (s, 1H), 8.77 (d, *J* = 5.2 Hz, 1H), 7.54 (d, *J* = 5.1 Hz, 1H), 4.60 (s, 2H); MS [MH]⁺ calcd for C₅H₆ClN₂ 129.0, found 129.1;

### Step2: Synthesis of 2-(pyrimidin-4-yl)acetonitrile (3)

Anhydrous potassium carbonate (7.78 mmol, 1.08 g), sodium iodide (3.89 mmol, 583 mg) and trimethylsilanecarbonitrile (5.83 mmol, 579 mg) were dissolved in acetonitrile (12 mL), the mixture was heated to 50°C. Finally 4-(chloromethyl)- pyrimidine (3.89 mmol, 500 mg) was dropped into the reaction mixture. The mixture was stirred 50 °C for 2 hours. Then it was concentrated in vacco and the residue was purified by column chromatograph (silica gel, PE / EA = 1/1) to obtain the desired product (120 mg, 26%) as a black oil. ¹H-NMR (400 MHz, CDCl₃) δ (ppm) 9.21 (s, 1H), 8.81 (d, *J=* 5.2 Hz, 1H), 7.52 (d, *J=* 5.0 Hz, 1H), 3.94 (s, 2H); MS [MH]+ calcd for C₆H₆N₃ 120.1, found 120.2.

### Step3: Synthesis of 3-(3,4-dihydroxy-5-nitrophenyl)-2-(pyrimidin-4-yl)acrylonitrile (4)

A mixture of 2-(pyrimidin-4-yl)acetonitrile (0.95 mmol, 113 mg), 3,4-dihydroxy-5-nitrobenzaldehyde (0.79 mmol, 145 mg) and ammonium acetate (4.75 mmol, 366 mg) in methanol (8 mL) was stirred at 80 °C for 4 hours, then it was filtered and washed with methanol and water to obtain the desired product (200 mg, 89%). ¹H-NMR (400 MHz, DMSO) δ(ppm) 9.08 (s, 1H), 8.72 (d, *J* = 5.5 Hz, 1H), 8.31 (s, 1H), 8.02 (s, 1H), 7.74 (d, *J* = 5.5 Hz, 1H), 7.59 (s, 1H), 7.08 (s, 2H); MS [MH]⁻ calcd for C₁₃H₇N₄O₄ 283.1, found 283.0;

**Compound 505:** 505 was prepared in three synthetic steps from 3-chloropyridazine, according to the following procedure:

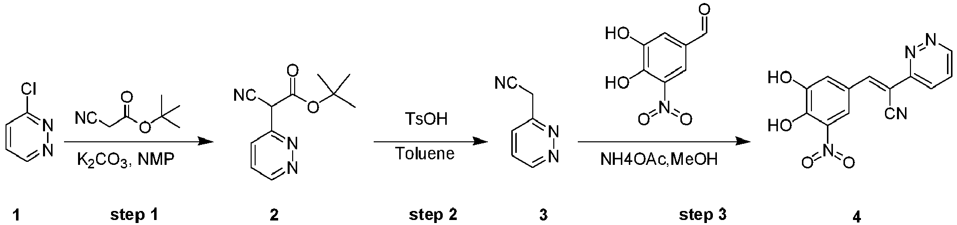

### Step1: Synthesis of tert-butyl 2-cyano-2-(pyridazin-3-yl)acetate (2)

To a solution of 3-chloropyridazine (0.5 g, 4.38 mmol) in NMP (2.5 mL) was added potassium carbonate (1.8 g, 13.15 mmol). Then *tert*-Butyl 2-cyanoacetate (0.88 mL, 6.14 mmol) was added. The yellow suspension was warmed up to 80°C and stirred 3 hours at 80 °C. The brown suspension was cooled down to room temperature. Then it was added to water (10 mL). The brown solution was acidified with HCI (gas evolution, strong foaming). There was a precipitation. The suspension was filtrated and the filter cake was washed with water. The filter cake was dissolved in ethyl acetate, dried with Na₂SO₄, filtrated and the organic phase evaporated to yield 600 mg of desired product as a yellow oil. ¹H-NMR (400 MHz, CDCl₃) δ (ppm): 14.3 (bs, 1 H), 7.68 (dd, 1 H), 7.35 (d, 1 H), 1.55 (s, 9H). MS [MH]⁺ calcd for C₁₀H₁₁N₃O₂ 206.1, found 206.1;

### Step2: Synthesis of 2-(pyridazin-3-yl)acetonitrile (3)

The product prepared above was combined with TsOH (142 mg) in toluene (50 mL). After being stirred at refluxing for 12 hours, the reaction was cooled to 25 °C., diluted with sat. NaHCO₃ and extracted (10percent MeOH/CH₂Cl₂ X 3). The organic layers were washed with brine, dried with Na₂SO₄, filtered and concentrated under reduced pressure. Purification of the crude material by flash chromatography (silica gel, 40-45percent EtOAc/Hexanes) gave the desired product (87 mg, 17% for two steps) as light yellow oil. MS [MH]⁺ calcd for C₆H₅N₃ 120.0, found 120.0.

### Step3: Synthesis of (E)-3-(3,4-dihydroxy-5-nitrophenyl)-2-(pyridazin-3-yl)acrylonitrile (4)

A mixture of 2-(pyrazin-2-yl)acetonitrile (87 mg, 0.73 mmol), 3,4-dihydroxy-5-nitrobenzaldehyde (0.79 mmol, 145 mg) and ammonium acetate (366 mg, 4.75 mmol) in methanol (8 mL) was stirred at 80 °C for 4 hours. Then it was filtered and washed with methanol and water, and dried in vacuo to obtain the desired product (155 mg, 75%) as a yellow solid. ¹H NMR (400 MHz, DMSO) δ 10.92 (s, 2H), 9.25 (d, *J* = 4.8 Hz, 1H), 8.43 (s, 1H), 8.20 (d, *J* = 8.7 Hz, 1H), 8.08 (d, *J* = 2.0 Hz, 1H), 7.95 (d, *J* = 2.0 Hz, 1H), 7.85 (dd, *J* = 8.7, 4.9 Hz, 1H). MS [MH]⁻ calcd for C₆H₅N₃ 283.1, found 283.0.

**Compound 331:** 331 was prepared in one synthetic step from 3,4-dihydroxy-5-nitrobenzaldehyde, according to the following procedure:

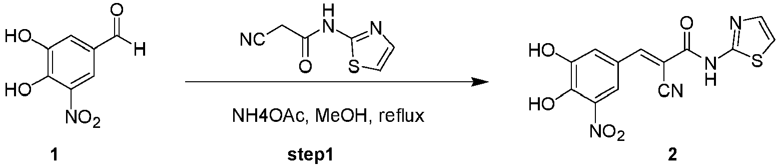

### Step1: Synthesis of (E)-2-cyano-3-(3,4-dihydroxy-5-nitrophenyl)-N-(thiazol-2-yl)acrylamide (2)

A solution of 2-cyano-*N-*(thiazol-2-yl)acetamide (184 mg, 1.1 mmol), 3,4-dihydroxy-5- nitrobenzaldehyde (200 mg, 1.1 mmol) and NH₄OAc (462 mg, 6 mmol) in MeOH (10 mL) was heated to reflux for overnight. LCMS showed no 3,4-dihydroxy-5-nitrobenzaldehyde left. The reaction mixture was cooled to room temperature. The solid was filtered and washed by MeOH and H₂O. The solid was re-dissolved in MeOH (5 mL). 5 mL of IN aqueous HCl was added to adjust pH 3∼4. The desired product was obtained by filter as a bright solid (90 mg, 25%).¹H NMR (400 MHz, DMSO) δ 8.17 (s, 1H), 7.95 (s, 1H), 7.50 (s, 2H), 7.20 (s, 1H). MS [MH]⁺ calcd for C₁₃H₈N₄O₅S 333.0, found 333.0.

**Compound 394:** 394 was prepared in four synthetic steps from benzene-1,2-diamine, according to the following procedure:

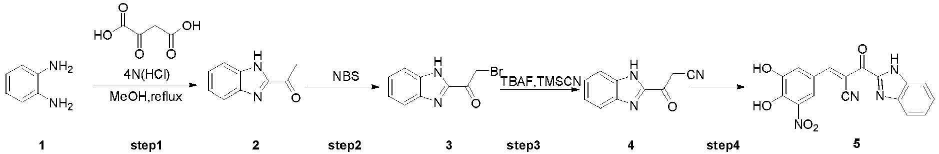

### Step1: Synthesis of 1-(1H-benzo[d]imidazol-2-yl)ethanone (2)

A mixture of 2-oxosuccinic acid (4.9 g, 37 mmol), benzene-1,2-diamine (4 g, 37 mmol) and 4N hydrochloride solution (9 mL) in MeOH (30 mL) was refluxed for 7 hours. After the completion of the reaction, it was concentrated in vacuo to remove the solvent. The residue was dissolved in ethyl acetate, washed with aq. sodium bicarbonate and brine. The organic layers were combined and concentrated under reduced pressure. The residue was purified by column chromatograph (silica gel, PE/EA = 5/1) to obtain the desired product (4 g, 67%). MS [MH]⁺ calcd for C₉H₉N₂O 161.06, found 161.1.

### Step2: Synthesis of 1-(1H-benzo[d]imidazol-2-yl)-2-bromoethanone (3)

1-(1*H*-benzo[*d*]imidazol-2-yl)ethanone (4 g, 25 mmol) was dissolved in tetrachloromethane (50 mL). 1-Bromopyrrolidine-2,5-dione (5.3 g, 30 mmol) and 2,2'-(diazene-1,2-diyl)bis(2-methylpropane-nitrile) (411 mg, 2.5 mmol) were added. The mixture was stirred at 100 °C for 2 hours, then it was concentrated in vacuo and re-dissolved in ethyl acetate. The organic layer was washed with water and concentrated to obtain the crude product (2 g, 33%), which was used in the next step without further purification. MS [MH]⁺ calcd for C₉H₈BrN₂O 238.97, found 239.0.

### Step3: Synthesis of 3-(1H-benzo[d]imidazol-2-yl)-3-oxopropanenitrile (4)

A mixture of 1-(1*H*-benzo[*d*]imidazol-2-yl)-2-bromoethanone (2 g, 8.4 mmol), trimethylsilane- carbonitrile (1.66 g, 16.7 mmol), TBAF (2.2 g, 8.4 mmol) in dichloromethane (15 mL) was stirred at room temperature for 24 hours. Then it was concentrated in vacuo and re-dissolved in ethyl acetate. The organic layer was washed with water and concentrated to obtain the crude product (400 mg, 26%), which was used in the next step without further purification. MS [MH]⁺ calcd for C₁₀H₇N₃O 186.06, found 186.1.

### Step4: Synthesis of 2-(1H-benzo[d]imidazole-2-carbonyl)-3-(3,4-dihydroxy-5-nitrophenyl)acrylo- nitrile (5)

A solution of 3,4-dihydroxy-5-nitrobenzaldehyde (107 mg, 0.59 mmol) and 3-(1*H-*benzo[d]imida- zole-2-yl)-3-oxopropanenitrile (130 mg, 0.7 mmol) and NH₄OAc (273 mg, 3.54 mmol) in methanol (10 mL) was heated to reflux for overnight. LCMS showed the desired product was formed, the reaction mixture was cooled to room temperature and concentrated in vacuo to remove the solvent. The desired product was obtained by Prep-HPLC (50 mg, 24%). ¹H-NMR (400 MHz, DMSO-*d⁶*) *δ* (ppm) 12.63 (s, 1H), 8.95 (s, 1H), 7.85 (s, 1H), 7.76 (d, *J* = 8.2 Hz, 1H), 7.65 (s, 1H), 7.50 (t, *J* = 7.5 Hz, 1H), 7.38 - 7.26 (m, 2H), 7.12 (s, 2H). MS [MH]⁺calcd for C₁₇H₁₁N₄O₅ 351.07, found 351.0.

**Compound 382:** 382 was prepared in two synthetic steps from ethyl 2-cyanoacetate, according to the following procedure:

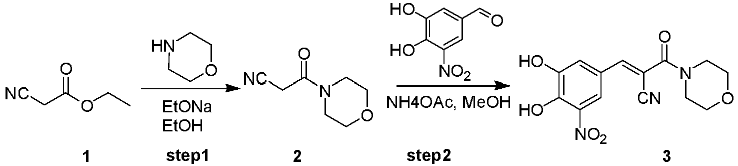

### Step1: Synthesis of 3-morpholino-3-oxopropanenitrile (2)

A mixture of sodium ethoxide (0.1 mmol) in ethanol (3 mL), ethyl cyanoacetate (1.13 g, 10 mmol) and morpholine (0.85 g,10 mmol) was stirred at room temperature for 24 hours. The precipitate was collected by filtration, washed with diethylether and recrystallised in ethanol to provide a white solid of 3-morpholino-3-oxopropanenitrile (0.56 g, 35%).

### Step2: Synthesis of (E)-3-(3,4-dihydroxy-5-nitrophenyl)-2-(piperidine-1-carbonyl)acrylonitrile (3)

A solution of 3-morpholino-3-oxopropanenitrile (300 mg, 2.0 mmol), 3,4-dihydroxy-5-nitro- benzaldehyde (188 mg, 1.1 mmol) and NH₄OAc (462 mg, 6 mmol) in MeOH (10 mL) was heated to reflux for 5 hours. LCMS showed no 3,4-dihydroxy-5-nitrobenzaldehyde left. The reaction mixture was cooled to room temperature, concentrated in vacuo to dryness. Further purification by Prep-HPLC (0.5%TFA, MeOH/H₂O) afforded the desired product as a yellow solid (60 mg, 19%). ¹H NMR (400 MHz, DMSO) δ 10.87 (s, 2H), 7.94 (d, *J* = 2.1 Hz, 1H), 7.77 (d, *J* = 2.1 Hz, 1H), 7.68 (s, 1H), 3.56-3.66 (m, 8H). MS [MH]⁺ calcd for C₁₄H₁₃N₃O₆ 320.1, found 320.0.

**Compound 351:** 351 was prepared in three synthetic steps from 3,4-dimethoxy-5-nitrobenzoic acid, according to the following procedure:

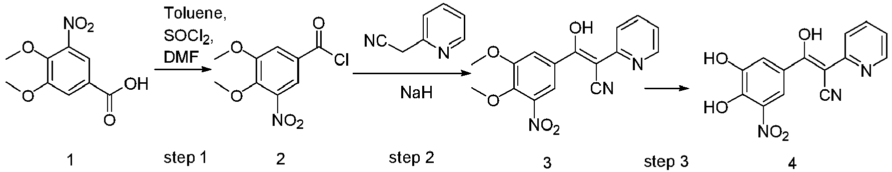

### Step1: Synthesis of 3,4-dimethoxy-5-nitrobenzoyl chloride (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60°C for 15 hours. The solvent was removed under reduced pressure. The resulting yellowish solid (500 mg, 92%) was used in the next step without further workup.

### Step2: Synthesis of 3-(3,4-dimethoxy-5-nitrophenyl)-3-oxo-2-(pyridin-2-yl)propanenitrile (3)

Under a nitrogen atmosphere, NaH (60% w/w, 176 mg, 4.4 mmol) was added to solution of 2-(pyridin- 2-yl)acetonitrile (236 mg, 2.0 mmol) in anhydrous THF (10 mL) at -5 °C. The resulting suspension was stirred at -5 °C for 15 min and the solution of 3,4-dimethoxy-5-nitrobenzoyl chloride (500 mg, 2.2 mmol) in THF (5 mL) was added over 10 min and stirred for an additional 1 hour at -5 °C. The reaction mixture was warmed to 0°C, quenched by the addition of 1N.HCl solution (4 mL) and stirred for 10 min at room temperature. The mixture was extracted with ethyl acetate (25 mL X 2). The combined organic layers were dried with anhydrous sodium sulfate and concentrated in vacuo to give the desired product (425 mg, 64%). MS [MH]⁺calcd for C16H14N3O5 328.09, found 328.1.

### Step3: Synthesis of 3-(3,4-dihydroxy-5-nitrophenyl)-3-oxo-2-(pyridin-2-yl)propanenitrile (4)

A solution of 3-(3,4-dimethoxy-5-nitrophenyl)-3-oxo-2-(pyridin-2-yl)propanenitrile (425 mg, 1.3 mmol) in dichloromethane (5 mL) was added 1.0 M solution of BBr₃ in dichloromethane (10 mL, 10 mmol) at -15 °C under a nitrogen atmosphere. The resulting suspension was stirred for 1 hours at -15 °C and allowed to warm to room temperature for overnight. The reaction was quenched slowly by the addition of water (4 mL) and stirred for another 30 min. The aqueous phase was extracted with ethyl acetate (30 mL X 3). The organic layers were combined, washed with brine and dried over with anhydrous sodium sulfate. The solvent was removed under reduced pressure to give the crude product. Further purification was conducted by Prep-HPLC to obtain the desired product (65 mg, 17%). ¹H-NMR (400 MHz, DMSO-d⁶) δ(ppm) 16.11 (s, 1H), 10.65 (s, 2H), 8.38 (t, *J* = 5.7 Hz, 1H), 8.28 - 7.96 (m, 1H), 7.84 (d, *J* = 2.0 Hz, 1H), 7.55 (d, *J* = 2.1 Hz, 1H), 7.47 (d, *J* = 8.7 Hz, 1H), 7.26 (t, *J* = 6.6 Hz, 1H). MS [MH]⁺calcd for C₁₄H₁₀N₃O₅ 300.05, found 300.0.

**Compound 371:** 371 was prepared in two synthetic steps from 2-cyanoacetyl chloride, according to the following procedure:

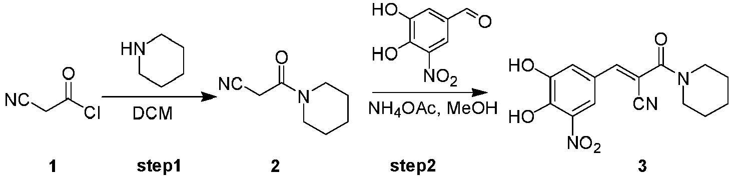

### Step1: Synthesis of 3-oxo-3-(piperidin-1-yl)propanenitrile (2)

A mixture of piperidine (5 mL, 50.6 mmol), in DCM (25 mL) was added 2-cyanoacetyl chloride (5 mL) at 0 °C, then warmed to room temperature overnight. The reaction mixture was quenched by H₂O, and concentrated in vacuo to dryness, the residue was purified by column chromatography (SiO₂, PE / EA = 1/1) to give the 3-oxo-3-(piperidin-1-yl)propanenitrile as a yellow oil (500 mg, 7%). MS [MH]⁺ calcd for C₈H₁₂N₂O 153.1, found 153.1.

### Step2: Synthesis of (E)-3-(3,4-dihydroxy-5-nitrophenyl)-2-(piperidine-1-carbonyl)acrylonitrile (3)

A solution of 3-oxo-3-(piperidin-1-yl)propanenitrile (300 mg, 2 mmol), 3,4-dihydroxy-5- nitrobenzaldehyde (273 mg, 1.5 mmol) and NH₄OAc (924 mg, 12 mmol) in MeOH (15 mL) was heated to reflux for 3 hours. The solid was filtered and washed by MeOH and H₂O to give the crude product. The solid dissolved in MeOH (5 mL) was added 1N.HCl (0.5 mL), the color was changed and the solid was formed, the solid was filtered and washed by H20, dried in vacuo to give the (*E*)-3-(3,4-dihydroxy-5-nitrophenyl)-2-(piperidine-1-carbonyl)acrylonitrile as a bright yellow solid (60 mg, 13%). ¹H NMR (400 MHz, DMSO) δ 10.86 (s, 2H), 7.92 (d, *J* = 2.1 Hz, 1H), 7.75 (d, *J* = 2.0 Hz, 1H), 7.63 (s, 1H), 3.56 - 3.45 (m, 4H), 1.55-1.62 (m, 6H). MS [MH]⁺ calcd for C₁₅H₁₅N₃O₅ 318.3, found 318.0.

**Compound 518:** 518 was prepared in two synthetic steps from 2-(pyridin-3-yl)acetonitrile, according to the following procedure:

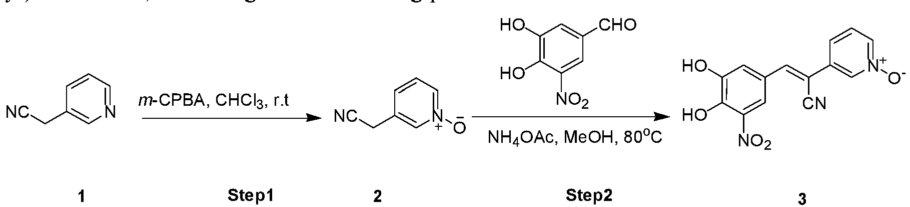

### Step1: Synthesis of 3-(cyanomethyl)pyridine 1-oxide (2)

A solution of 2-(pyridin-3-yl)acetonitrile (625 mg, 5.3 mmol) and m-*CPBA*(1.36 g, 7.95 mmol) in CHCl₃ (20 mL) was stirred at room temperature for overnight. The reaction mixture was quenched by sat.NaHCO₃ and extracted by DCM and MeOH (DCM / MeOH = 10/1). The combined organic layers were dried over anhydrous Na₂SO₄, filtered and dried in vacuo to give the crude product as a white solid (780 mg, >100%), which was used to the next step without further purification. MS [MH]⁺ calcd for C₇H₆N₂O 135.0 found 135.0.

### Step2: Synthesis of (E)-3-(1-cyano-2-(3,4-dihydroxy-5-nitrophenyl)vinyl)pyridine 1-oxide (3)

A solution of 3-(cyanomethyl)pyridine 1-oxide (400 mg, 3.0 mmol), 3,4-dihydroxy-5-nitro- benzaldehyde (270 mg, 1.5 mmol) and NH₄OAc (693 mg, 9 mmol) in MeOH (10 mL) was heated to reflux for overnight. LCMS showed no 3,4-dihydroxy-5-nitrobenzaldehyde left. The reaction mixture was cooled to room temperature. The solid was filtered and washed by MeOH and H₂O. The solid was re-dissolved in MeOH (5 mL). 5 mL of IN aqueous HCl was added to adjust pH 3∼4. The desired product was obtained by filter as a bright solid (110 mg, 18%). ¹H NMR (301 MHz, DMSO) δ 13.71 (s, 1H), 10.89 (s, 2H), 8.65 (s, 1H), 8.26 (d, *J* = 6.2 Hz, 1H), 8.14 (s, 1H), 7.99 (d, *J* = 2.0 Hz, 1H), 7.84 (d, *J* = 2.0 Hz, 1H), 7.67 - 7.50 (m, 2H). MS [MH]⁺ calcd for C₁₄H₉N₃O₅ 300.0 found 300.0.

**Compound 523:** 523 was prepared in three synthetic steps from 3,4-dimethoxy-5-nitrobenzoic acid, according to the following procedure:

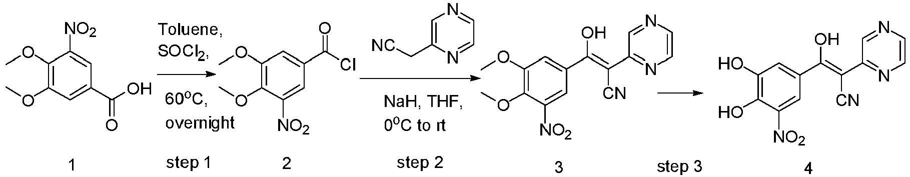

### Step1: Synthesis of 3,4-dimethoxy-5-nitrobenzoyl chloride (2)

Under a nitrogen atmosphere, SOCl₂ (0.76 mL, 10.56 mmol) and anhydrous DMF (0.02 mL, 0.44 mmol) were added to a suspension of 3,4-dimethoxy-5-nitrobenzoic acid (1 g, 4.4 mmol) in toluene (20 mL) at room temperature. The mixture was heated at 60 °C and stirred for 15 hours. The solvent was removed under reduced pressure. The resulting yellowish solid 3,4-dimethoxy-5-nitrobenzoyl chloride (1 g, 93%) was used in the next step without further workup.

### Step2: Synthesis of 3-(3,4-dimethoxy-5-nitrophenyl)-3-hydroxy-2-(pyrazin-2-yl)acrylonitrile (3)

Under a nitrogen atmosphere, 60% NaH (336 mg, 8.4 mmol) was added to solution of 2-(pyrazin-2-yl)acetonitrile (500 mg, 4.2 mmol) in anhydrous THF (5 mL) at -5 °C. The resulting suspension was stirred at -5°C for 15 min and the THF solution of 3,4-dimethoxy-5-nitrobenzoyl chloride (500 mg, 4.07 mmol) was added over 10 min and stirred for an additional 1 hour at -5 °C. The reaction mixture was warmed to 0°C, quenched by the addition of 1N.HCl solution (8 mL) and stirred for 10min at room temperature, then extracted with ethyl acetate (30 mL X 3), the organic layer was dried with anhydrous sodium sulfate and concentrated in vacuo to obtain the desired product (440 mg, 33%). MS [MH]⁻ calcd for C₁₅H₁₁N₄O₅ 327.08, found 327.1.

### Step3: Synthesis of 3-(3,4-dihydroxy-5-nitrophenyl)-3-hydroxy-2-(pyrazin-2-yl)acrylonitrile (4)

To a solution of 3-(3,4-dimethoxy-5-nitrophenyl)-3-hydroxy-2-(pyrazin-2-yl)acrylonitrile (200 mg, 0.61 mmol) in anhydrous dichloromethane (5 mL) was added 1.0 M solution of BBr₃ in dichloromethane (3 mL, 3 mmol) at -15 °C under a nitrogen atmosphere. The resulting suspension was stirred for 1 hour at -15 °C and allowed to warm to room temperature for overnight. The reaction was quenched by the addition of water (2 mL) and stirred for another 30 min. The aqueous phase was extracted with ethyl acetate (30 mL X 3). The organic layers were combined, washed with brine and dried over anhydrous sodium sulfate. The solvent was removed under reduced pressure to give the crude product. Further purification was conducted by Prep-HPLC to obtain the desired product (35 mg, 19%). ¹H-NMR (400 MHz, DMSO-d⁶) δ(ppm) 15.85 (s, 1H), 10.75 (s, 2H), 8.88 (s, 1H), 8.34 (d, *J* = 3.7 Hz, 1H), 8.22 (d, *J* = 2.5 Hz, 1H), 7.88 (d, *J* = 2.1 Hz, 1H), 7.56 (d, *J* = 2.1 Hz, 1H). MS [MH]⁻ calcd for C₁₃H₇N₄O₅ 299.05, found 299.0.

**Compound 525:** 525 was prepared in two synthetic steps from 3,4-dimethoxy-5-nitrobenzoic acid, according to the following procedure:

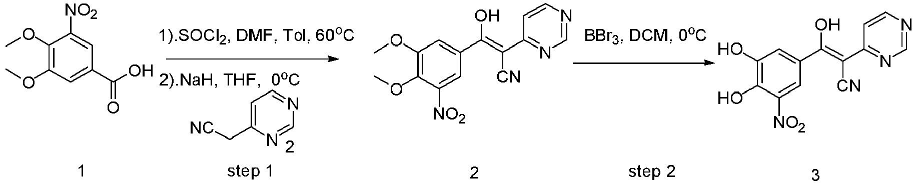

### Step1: Synthesis of (E)-3-(3,4-dimethoxy-5-nitrophenyl)-3-hydroxy-2-(pyrimidin-4-yl)acrylonitrile (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More touene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to solution of 2-(pyrimidin-4-yl)acetonitrile (0.44 g, 2.0 mmol) in anhydrous THF (10 mL) at -5 °C. The resulting suspension was stirred at -5 °C for 15 min and the THF solution of 3,4-dimethoxy-5-nitrobenzoyl chloride was added over 10 min and stirred for an additional 1 hour at -5 °C. The reaction mixture was warmed to 0 °C, quenched by the addition of 1N.HCl solution (4 mL) and stirred for 10min at room temperature, extracted by ethyl acetate (25 mL X 2), the organic layers was dried with Na₂SO₄ and concentrated in vacuo to give the title compound as an orange solid (550 mg, 85%). MS [MH]⁺calcd for C₁₅H₁₂N₄O₅ 329.0 found 329.0.

### Step2: Synthesis of (E)-3-(3,4-dihydroxy-5-nitrophenyl)-3-hydroxy-2-(pyrimidin-4-yl)acrylonitrile (3)

A solution of (*E*)-3-(3,4-dimethoxy-5-nitrophenyl)-3-hydroxy-2-(pyrimidin-4-yl)acrylonitrile (250 mg, 0.76 mmol) in DCM (5 mL) was added BBr₃ (0.5 mL, 5 mmol) at -5 °C under a nitrogen atmosphere. The resulting red suspension was stirred for 1h at -5 °C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried over Na₂SO₄. The solvent was eliminated under reduced pressure to give the crude product. Further purification by Prep-HPLC (0.5%TFA, MeOH/H₂O) gave the desired product as a bright yellow solid (46 mg, 19%). ¹HNMR (400 MHz, DMSO) δ 15.35 (s, 1H), 10.67 (s, 2H), 8.95 - 7.16 (m, 5H). MS [MH]⁺calcd for C₁₅H₁₄N₂O₅ 337.0 found 301.0(free).

**Compound 503:** 503 was prepared in two synthetic steps from 3,4-dimethoxy-5-nitrobenzoic acid, according to the following procedure:

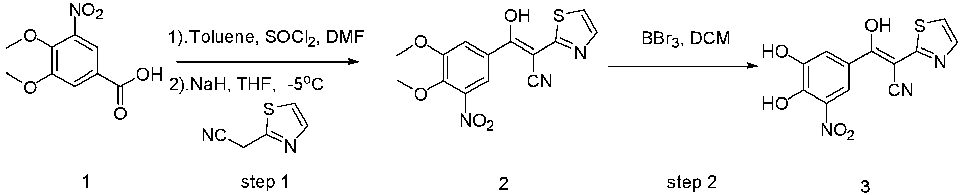

### It Step1: Synthesis of 3-(3,4-dimethoxy-5-nitrophenyl)-3-oxo-2-(thiazol-2-yl)propanenitrile (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to the solution of 2-(thiazol-2-yl) acetonitrile (0.24 g, 2.0 mmol) in anhydrous THF (5 mL) at -5 °C. The resulting suspension was stirred at -5 °C for 15 min and the solution of 3,4-dimethoxy-5-nitrobenzoyl chloride in THF was added over 10 min and stirred for an additional 1 hour at -5 °C. The reaction mixture was warmed to 0 °C, quenched by the addition of 1N.HCl solution (4 mL) and stirred for 10 min at room temperature, extracted by ethyl acetate (25 mL * 2), the organic layers was dried with Na₂SO₄ and concentrated in vacuo to give the title compound as an orange solid (240 mg, 39%). MS [MH]⁺ calcd for C₁₄H₁₁N₃O₅S 334.3, found 334.3.

### Step2: Synthesis of 3-(3,4-dihydroxy-5-nitrophenyl)-3-oxo-2-(thiazol-2-yl)propanenitrile (3)

A solution of 3-(3,4-dimethoxy-5-nitrophenyl)-3-oxo-2-(thiazol-2-yl)propanenitrile (224 mg, 0.67 mmol) in DCM (5 mL) was added 1.0 M solution of BBr₃ in DCM (3 mL, 3 mmol) at -15 °C under nitrogen atmosphere. The resulting red suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried over Na₂SO₄. The solvent was eliminated under reduced pressure to give the crude product. Further purification by Prep-HPLC (0.5%TFA, MeOH/H₂O) gave the desired product as a bright yellow solid (24 mg, 12%). ¹H NMR (400 MHz, DMSO) δ 7.88 (d, *J* = 2.0 Hz, 1H), 7.60 (d, *J* = 4.0 Hz, 1H), 7.56 (d, *J* = 2.0 Hz, 1H), 7.31 (d, *J* = 4.0 Hz, 1H). MS [MH]⁻ calcd for C₁₂H₇N₃O₅S 304.0, found 304.0.

**Compound 374:** 374 was prepared in two synthetic steps from 3,4-dimethoxy-5-nitrobenzoic acid, according to the following procedure:

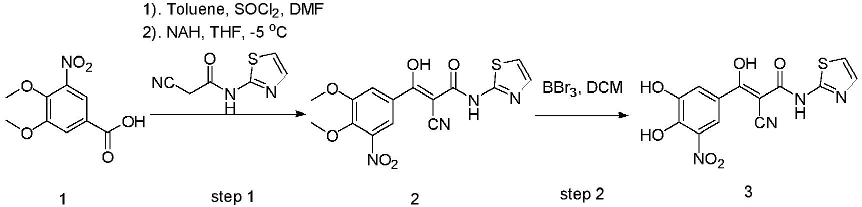

### Step1: Synthesis of 2-cyano-3-(3,4-dimethoxy-5-nitrophenyl)-3-oxo-N-(thiazol-2-yl)propanamide (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More touene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to solution of 2-cyano-*N*-(thiazol-2-yl)acetamide (0.35 g, 2.0 mmol) in anhydrous THF (10 mL) at -5 °C. The resulting suspension was stirred at -5 °C for 15 min and the THF solution of 3,4-dimethoxy-5-nitrobenzoyl chloride was added over 10 min and stirred for an additional 1 hour at -5 °C. The reaction mixture was warmed to 0 °C, quenched by the addition of 1N.HCl solution (4 mL) and stirred for 10min at room temperature, extracted by ethyl acetate (25 mL X 2), the organic layers was dried with Na₂SO₄ and concentrated in vacuo to give the title compound as an orange solid (510 mg, 67%). MS [MH]⁺ calcd for C₁₅H₁₂N₄O₆S 377.0, found 377.0.

### Step2: Synthesis of 2-cyano-3-(3,4-dihydroxy-5-nitrophenyl)-3-oxo-N-(thiazol-2-yl)propanamide (3)

A solution of 2-cyano-3-(3,4-dimethoxy-5-nitrophenyl)-3-oxo-*N*-(thiazol-2-yl)propanamide (400 mg, 1.1 mmol) in DCM (5 mL) was added 1.0 M solution of BBr₃ in DCM (5 mL, 5 mmol) at -15 °C under a nitrogen atmosphere. The sesulting red suspension was stirred for 1h at -15 °C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried over Na₂SO₄. The solvent was eliminated under reduced pressure to give the crude product. Further purification by Prep-HPLC (0.5%TFA, MeOH/H₂O) gave the desired product as a bright yellow solid (100 mg, 28%). ¹H NMR (400 MHz, DMSO) δ 7.88 (d, *J* = 2.0 Hz, 1H), 7.60 (d, *J* = 4.0 Hz, 1H), 7.56 (d, *J* = 2.0 Hz, 1H), 7.31 (d, *J* = 4.0 Hz, 1H). MS [MH]⁺ calcd for C₁₃H₈N₄O₆S 349.0, found 349.0.

**Compound 655:** 655 was prepared in four synthetic steps from methyl 5-chloropyrazine-2-carboxylate, according to the following procedure:

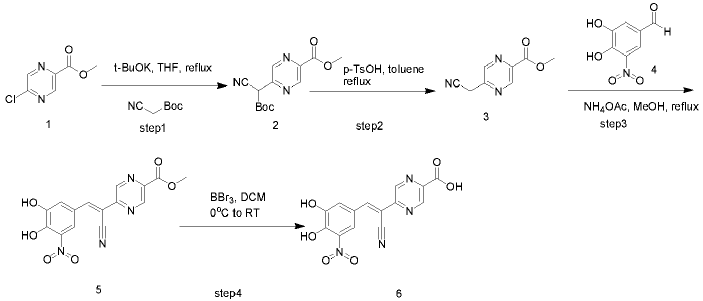

### Step1: Synthesis of methyl 5-(2-(tert-butoxy)-1-cyano-2-oxoethyl)pyrazine-2-carboxylate (2)

A solution of *tert*-butyl 2-cyanoacetate (2.1 g, 15 mmol) and *t*-BuOK (1.6 g, 15 mmol) in dry THF (50 mL) stirred at rt for 30 min, then methyl 5-chloropyrazine-2-carboxylate (1.7 g, 10.0 mmol) was added, the reaction mixture was heated to reflux overnight, After the reaction was completed, cooled it to rt and quenched by H₂O(100 mL), the solid was filtered and dried in vacuo to afford the desired product as yellow solid (1.7 g, 61%). MS [M+H]⁺ calcd for C₁₃H₁₅N₃O₄ 278.1, found 278.1.

### Step2: Synthesis of methyl 5-(cyanomethyl)pyrazine-2-carboxylate (3)

A solution of 5-(2-(*tert*-butoxy)-1-cyano-2-oxoethyl)pyrazine-2-carboxylate(1.7 g, 6.14 mmol) and p-TsOH (314 mg, 1.84 mmol) was heated to reflux for 3h, then TLC showed no starting materials left. The reaction mixture was quenched by H₂O (5 mL), extracted by EA, washed with sat. NaHCO₃, the organic layer was dried with Na₂SO₄, filtered and dried in vacuo to afford the crude product, further purification by column chromatography (SiO₂, 100g, 200-300m, eluted by PE/EA = 5/1) to afford the desired product methyl 5-(cyanomethyl)pyrazine-2-carboxylate (800 mg, 74%) as yellow solid. MS [M+H]⁺ calcd for C₈H₇N₃O₂ 178.1 ound 178.1.

### Step3: Synthesis of methyl (Z)-methyl 5-(1-cyano-2-(3,4-dihydroxy-5-nitrophenyl)vinyl)pyrazine-2-carboxylate (4)

A solution of methyl 5-(cyanomethyl)pyrazine-2-carboxylate (170 mg, 1.0 mmol), 3,4-dihydroxy-5- nitrobenzaldehyde (183 mg, 1.0 mmol) and NH₄OAc (554 mg, 7.2 mmol) in MeOH (15 mL) was heated to reflux for 3 hours, then cooled it to room temperature. The solid was filtered and washed by H₂O (15 mL), The solid was re-dissolved in MeOH (5 mL). 5 mL of IN aqueous HCl was added to till pH=3∼4. The desired product was obtained by filter and dried in vacuo as bright solid (210 mg, 61%). MS [M+H]⁺ calcd for C₁₅H₁₀N₄O₆ 343.0, found 343.0.

### Step4: Synthesis of (Z)-5-(1-cyano-2-(3,4-dihydroxy-5-nitrophenyl)vinyl)pyrazine-2-carboxylic acid (5)

A solution of (Z)-methyl 5-(1-cyano-2-(3,4-dihydroxy-5-nitrophenyl)vinyl)pyrazine-2-carboxylate (150 mg, 0.44 mmol) in DCM (5 mL) was added 1.0 M solution of BBr₃ in DCM (2 mL, 2 mmol) at -15 °C under a nitrogen atmosphere. The suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried with Na₂SO₄. The solvent was eliminated in vacuo to give the crude product. Further purification by Prep-HPLC (0.5%TFA, MeOH/H₂O) gave the desired product as bright yellow solid (55 mg, 38%). ¹H NMR (300 MHz, DMSO) δ 9.27 (s, 1H), 9.22 (s,1H), 8.57 (s, 1H), 8.12 (s, 1H), 7.98 (s, 1H), 2.52 (s, 19H), 0.02 (s, 1H)..MS [MH]⁺ calcd for C₁₄H₈N₄O₆ 329.0, found 329.0.

**Compound 656:** 656 was prepared in two synthetic steps from 5-chloro-1,2,4-thiadiazole, according to the following procedure:

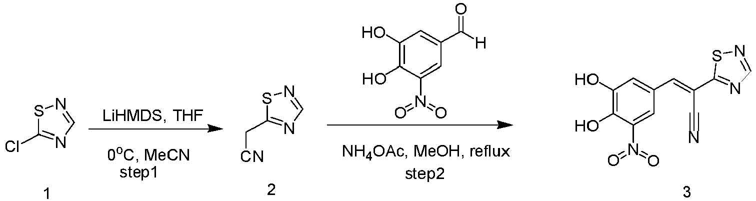

### Step1: Synthesis of 2-(1,2,4-thiadiazol-5-yl)acetonitrile (2)

A solution of dry MeCN (226 mg, 11 mmol) in dry THF (25 ml) was added LiHMDS (5.5 mmol, 5.5 mL) at 0 °C, then the mixture was stirred at 0 °C for 30 min, 5-chloro-1,2,4-thiadiazole (691 mg, 5.5 mmol) in dry THF (5 mL) was added to the mixture at 0 °C, then stirred at rt overnight. The reaction mixture was quenched by H₂O (1 mL), and extracted by EA (30 mL X 3), dried with Na₂SO₄, filtered and dried in vacuo to afford the crude product. Further purification by column chromatography (SiO₂, 100 g, 200-300 m, eluted by PE/EA = 5:1) gave the desired product (410 mg, 60%) as yellow solid. MS [M+H]⁺ calcd for C₄H₃N₃S 126.0, found 126.0.

### Step2: Synthesis of (E)-3-(3,4-dihydroxy-5-nitrophenyl)-2-(1,2,4-thiadiazol-5-yl)acrylonitrile (3)

A solution of 2-(1,2,4-thiadiazol-5-yl)acetonitrile (150 mg, 1.2 mmol), 3,4-dihydroxy-5-nitrobenzaldehyde (182 mg, 1 mmol) and NH₄OAc (462 mg, 6 mmol) in MeOH (10 mL) was heated to reflux for 5 hours. LC-MS showed no starting materials left. The solid was filtered and washed by MeOH and H₂O, then dissolved in MeOH (5 mL). 5 ml of 1N.HCl was added till pH=3∼4. The solid was filtered and dried in vacuo to give the desired product as bright yellow solid (200 mg, 69%). ¹H NMR (400 MHz, DMSO) δ 10.96 (s, 1H), 8.97 (s, 1H), 8.45 (s, 1H), 8.14 (d, *J* = 2.1 Hz, 1H), 7.95 (d, *J* = 2.2 Hz, 1H). MS [M+H]⁺ calcd for C₁₁H₆N₄O₄S 291.0, found 291.0.

**Compound 660:** 660 was prepared in two synthetic steps from (E)-2-cyano-3-(3,4-dihydroxy-5- nitrophenyl)acrylic acid, according to the following procedure:

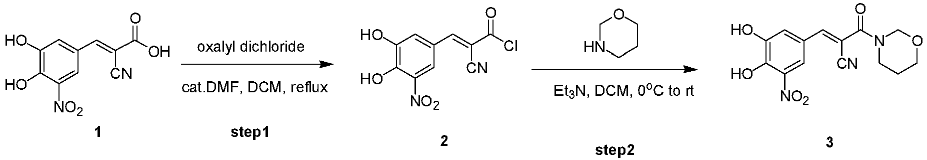

### Step1: Synthesis of (E)-2-cyano-3-(3,4-dihydroxy-5-nitrophenyl)acryloyl chloride (2)

A solution of (*E*)-2-cyano-3-(3,4-dihydroxy-5-nitrophenyl)acrylic acid (25 mg, 0.1 mmol) in DCM(25 ml) was added a drop of DMF and oxalyl dichloride (25 mg,0.2 mmol). The reaction mixture was heated till the solid dissolved, cooled to rt, concentrated in vacuo to dryness. It was used in the next step without purification (27 mg, 100%).

### Step2: Synthesis of (E)-3-(3,4-dihydroxy-5-nitrophenyl)-2-(1,3-oxazinane-3-carbonyl)acrylonitrile (3)

A solution of 1,3-oxazinane (9 mg, 0.12 mmol), Et₃N (24mg, 0.24 mmol) in DCM (3 mL) was added acyl chloride dissolved in DCM (3 mL) dropwise at 0°C, when the addition was completed, the reaction mixture was slowly warmed to rt overnight. The reaction mixture was quenched by H₂O, separated the organic layer, and dried with Na₂SO₄, concentrated in vacuo to afford the crude product, further purification by Prep-HPLC (0.5%TFA, MeOH/H₂O) afford the desired product as yellow solid (5 mg, 16%). 1H NMR (400 MHz, DMSO) δ 10.87 (s, 2H), 7.95 (d, J = 2.0 Hz, 1H), 7.76 (d, J = 2.1 Hz, 1H), 7.69 (s, 1H), 5.01 (s, 2H), 3.88 - 3.84 (m, 2H), 3.74 (s, 2H), 1.72 - 1.66 (m, 2H).MS [M+H]+calcd for C₁₄H₁₄N₃O₆ 320.1 found.319.9

**Compound 661:** 661 was prepared in two synthetic steps from 3,4-dimethoxy-5-nitrobenzoic acid, according to the following procedure:

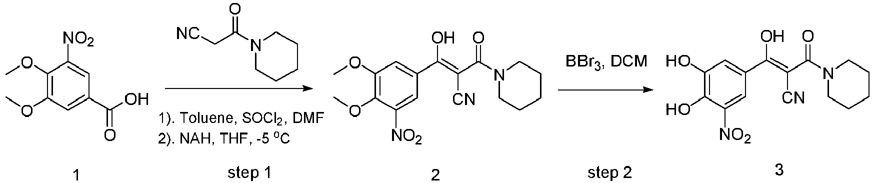

### Step1: Synthesis of (Z)-3-(3,4-dimethoxy-5-nitrophenyl)-3-hydroxy-2-(piperidine-1-carbonyl) acrylonitrile (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60°C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18g, 4.4 mmol) was added to the solution of 3-oxo-3-(piperidin-1-yl)propanenitrile (0.30 g, 2.0 mmol) in anhydrous THF (5 mL) at - 5°C. The resulting suspension was stirred at -5°C for 15 min and the solution of 3,4-dimethoxy-5-nitrobenzoyl chloride in THF was added over 10min and stirred for an additional 1hour at -5°C. The reaction mixture was warmed to 0°C, quenched by the addition of 1N.HCl solution (4 mL) and stirred for 10min at room temperature, extracted by ethyl acetate (25 mL * 2), the organic layers was dried with Na₂SO₄ and concentrated in vacuo to give the title compound **3** as an orange solid (260 mg, 36%). MS [MH]⁺calcd for C₁₇H₂₀N₃O₆ 362.1, found 362.1.

### Step2: Synthesis of (Z)-3-(3,4-dihydroxy-5-nitrophenyl)-3-hydroxy-2-(piperidine-1-carbonyl)acrylonitrile (3)

A solution of (Z)-3-(3,4-dimethoxy-5-nitrophenyl)-3-hydroxy-2-(piperidine-1-carbonyl)acrylonitrile (180 mg, 0.5 mmol) in DCM (5 mL) was added 1.0 M solution of BBr₃ in DCM (3 mL, 3 mmol) at -15°C under nitrogen atmosphere. The resulting red suspension was stirred for 1 hour at -15°C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried with Na₂SO₄. The solvent was eliminated under reduced pressure to give the crude product. Further purification by Prep-HPLC (0.5%TFA, MeOH/H₂O) gave the desired product as yellow solid (63 mg, 38%). 1H NMR (400 MHz, DMSO) δ 10.96 (s, 1H), 7.87 (d, J = 2.0 Hz, 1H), 7.49 (s, 1H), 3.65 (s, 4H), 1.60 (s, 6H). MS [M-H]-calcd for C₁₅H₁₆N₃O₆ 334.1, found 333.9

**Compound 666:** 666 was prepared in two synthetic steps from 3,4-dimethoxy-5-nitrobenzoic acid, according to the following procedure:

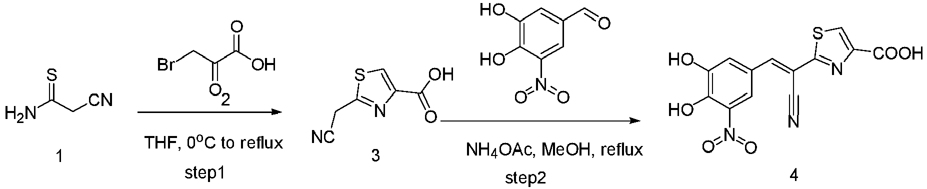

### Step1: Synthesis of 2-(cyanomethyl)thiazole-4-carboxylic acid (3)

Under a nitrogen atmosphere, 3-bromo-2-oxopropanoic acid in dry THF (5 mL) was added to a suspension of 2-cyanoethanethioamide (1.2 g, 12 mmol) in THF (20 ml) at 0 °C. The mixture was heated at 70 °C and stirred for 3 hours. The aqueous phase was extracted with ethyl acetate (30 mL X 3). The organic layers were combined, washed with brine and dried with Na₂SO₄. The solvent was eliminated under reduced pressure to give the crude product. Further purification by column chromatography (SiO₂, 100 g, 200-300 m, eluted by PE/EA = 1/1) afforded the desired product as white solid (350mg, 18%).

### Step2: Synthesis of (E)-2-(1-cyano-2-(3,4-dihydroxy-5-nitrophenyl)vinyl)thiazole-4-carboxylic acid (4)

A solution of 2-(cyanomethyl)thiazole-4-carboxylic acid (168 mg, 1.0 mmol), 3,4-dihydroxy-5- nitrobenzaldehyde (183 mg, 1.0 mmol) and NH₄OAc (554 mg, 7.2 mmol) in MeOH (15 mL) was heated to reflux for 3 hours, then cooled to room temperature. The solid was filtered and washed by H₂O (15 mL), The solid was re-dissolved in MeOH (5 mL). 5 mL of IN aqueous HCl was added to adjust pH 3∼4. The desired product was obtained by filter and dried in vacuo as bright solid (150 mg, 45%). ¹H NMR (400 MHz, DMSO) δ 7.98 (s, 1H), 7.91 (s, 1H), 7.84 (s, 1H), 7.15-7.75 (m, 4H).MS [MH]⁺ calcd for C₁₃H₇N₃O₆S 334.0, found 334.0.

**Compound 668:** 668 was prepared in three synthetic steps from 3,4-dimethoxy-5-nitrobenzoic acid, according to the following procedure:

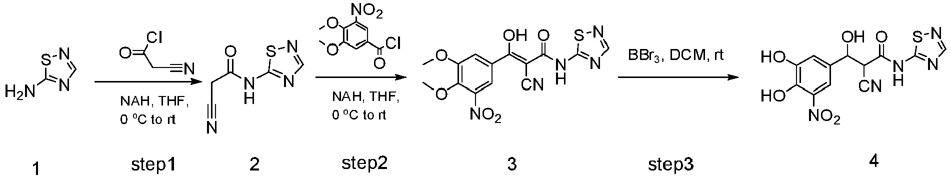

### Step1: Synthesis of 2-cyano-N-(1,2,4-thiadiazol-5-yl)acetamide (2)

Under a nitrogen atmosphere, NaH (200 mg, 5 mmol, 60%) was added to a suspension of 1,2,4-thiadiazol-5-amine (500 mg, 5 mmol) in THF (25 mL) at 0 °C. The resulting suspension was stirred at 0 °C for 15min and the solution of 2-cyanoacetyl chloride (500 mg, 5 mmol) in THF was added over 10min and stirred for an additional 1h at RT, then quenched by the addition of 1N.HCl solution and stirred for 10min at room temperature. Extracted it by ethyl acetate (25 mL X 2), and the organic layers was dried with Na₂SO₄ and concentrated in vacuo to give the crude product. Washed it by PE/EA = 1:1(5 mL) to afford the purity product (260 mg, 30%). MS [MH]⁺ calcd for C₅H₄N₄OS 169.0, found 169.0.

### Step2: Synthesis of 2-cyano-3-(3,4-dimethoxy-5-nitrophenyl)-3-hydroxy-N-(1,2,4-thiadiazol-5-yl)acrylamide (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. When the reaction was complete, the organic solvent was eliminated by distillation under reduced pressure. Additional toluene was added and eliminated again. The resulting yellowish solid 3,4-dimethoxy-5-nitrobenzoyl chloride was dissolved in anhydrous THF (5 mL).

Under a nitrogen atmosphere, 60% NaH (80 mg, 2.0 mmol) was added to solution of 2-cyano-N-(1,2,4-thiadiazol-5-yl)acetamide (250 mg, 1.5 mmol) in anhydrous THF (10 mL) at -5 °C. The resulting suspension was stirred at -5 °C for 15 min and the THF solution of 3,4-dimethoxy-5-nitrobenzoyl chloride was added over 10 min and stirred for an additional 1 hour at -5 °C. The reaction mixture was warmed to 0 °C; quenched by the addition of 1N.HCl solution (4 mL) and stirred for 10min at room temperature. Extracted by ethyl acetate (25 mL X 2), the organic layers was dried with Na₂SO₄ and concentrated in vacuo to give the title compound as orange solid (210 mg, 37%). MS [M+H]⁺ calcd for C₁₄H₁₁N₅O₆S 378.0, found 378.0.

### Step3: Synthesis of 2-cyano-3-(3,4-dihydroxy-5-nitrophenyl)-3-hydroxy-N-(1,2,4-thiadiazol-5-yl)acrylamide (4)

A solution of 2-cyano-3-(3,4-dimethoxy-5-nitrophenyl)-3-hydroxy-*N*-(1,2,4-thiadiazol-5-yl)acrylamide (150 mg, 0.40 mmol) in DCM (5 mL) was added 1.0 M solution of BBr₃ in DCM (2 mL, 2 mmol) at -15 °C under a nitrogen atmosphere. The suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried with Na₂SO₄. The solvent was eliminated under reduced pressure to give the crude product. Further purification by Prep-HPLC (0.5%TFA, MeOH/H₂O) gave the desired product as bright yellow solid (50 mg, 36%).¹H NMR (400 MHz, DMSO) δ 13.80 (s, 1H), 8.33 (s, 1H), 7.72 (d, *J* = 1.8 Hz, 1H), 7.47 (d, *J* = 1.8 Hz, 1H).MS [MH]⁺ calcd for C₁₂H₇N₅O₆S 350.0, found 350.0.

**Compound 673:** 673 was prepared in two synthetic steps from 3,4-dimethoxy-5-nitrobenzoic acid, according to the following procedure:

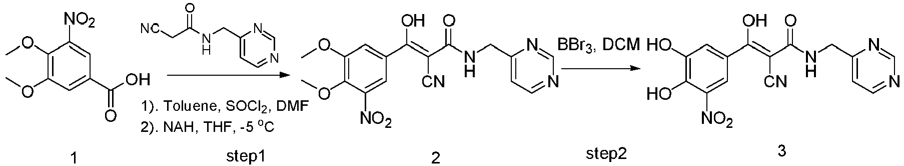

### Step1: Synthesis of (Z)-2-cyano-3-(3,4-dimethoxy-5-nitrophenyl)-3-hydroxy-N-(pyrimidin-4-ylmethyl)acrylamide (2)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60°C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18g, 4.4 mmol) was added to the solution of 2-cyano-N-(pyrimidin-4-ylmethyl)acetamide (0.35 g, 2.0 mmol) in anhydrous THF (5 mL) at -5°C. The resulting suspension was stirred at -5°C for 15 min and the solution of 3,4-dimethoxy-5-nitrobenzoyl chloride in THF was added over 10 min and stirred for an additional 1hour at -5°C. The reaction mixture was warmed to 0°C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the title compound **3** as an orange solid(169 mg, 22%). MS [M+H]⁺ calcd for C₁₇H₁₆N₅O₆ 386.1, found 386.0.

### Step2: Synthesis of (Z)-2-cyano-3-(3,4-dihydroxy-5-nitrophenyl)-3-hydroxy-N-(pyrimidin-4- ylmethyl)acrylamide (3)

A solution of (Z)-2-cyano-3-(3,4-dimethoxy-5-nitrophenyl)-3-hydroxy-N-(pyrimidin-4-ylmethyl)acrylamide (115 mg, 0.3 mmol) in DCM (5 mL) was added 1.0 M solution of BBr₃ in DCM (3 mL, 3 mmol) at -15°C under nitrogen atmosphere. The resulting red suspension was stirred for 1 hour at -15°C and allowed to warm to room temperature overnight. The reaction was quenched by the addition of H₂O (2 mL) and stirred for 30 min. The aqueous phase was extracted with ethyl acetate (30 mL X 3). The organic layers were combined, washed with brine and dried with Na₂SO₄. The solvent was eliminated under reduced pressure to give the crude product. Further purification by Prep-HPLC (0.5% TFA, MeOH/H₂O) gave the desired product as yellow solid (8 mg, 7%).1H NMR (400 MHz, DMSO) δ 14.18 (s, 1H), 10.74 (s, 2H), 9.12 (s, 1H), 8.75 (d, J = 5.2 Hz, 1H), 7.88 (s, 1H), 7.55 (d, J = 2.1 Hz, 1H), 7.45 (d, J = 4.7 Hz, 1H), 4.52 (s, 2H). MS [M+H]⁺ calcd for C₁₅H₁₂N₅O₆ 356.1, found 356.0

**Compound 675:** 675 was prepared by four synthetic steps from methyl 3-hdroxisoxazole-5-carboxlateaccording to the following procedure:

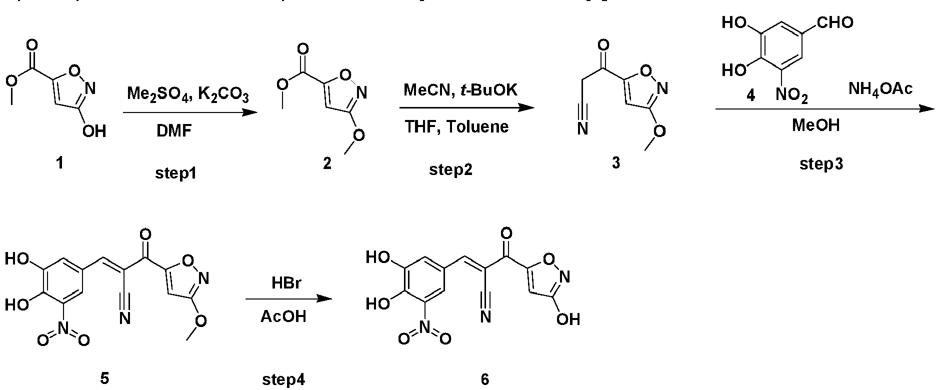

### Step1: Synthesis of methyl 3-methoxyisoxazole-5-carboxylate (2)

A mixture of methyl 3-hydroxyisoxazole-5-carboxylate (1.0 g, 7.0 mmol) and K₂CO₃ (1.9 g, 14 mmol) in dry DMF (15 mL) was added Me₂SO₄ (1.0 g, 8.4 mmol) at 0 °C. The reaction solution was stirred at 0 °C continuously, and monitored by TLC until all the starting material was consumed completely; 50 mL of water was added. The residue was extracted by EA for two times (25 mL X 2), and the organic layer was washed with brine (25 mL X 3), and dried over anhydrous Na₂SO₄. The organic layer was concentrated in vacuo to afford the desired product (850 mg, 77%) without further purification. MS [M+H]⁺ calcd for C₆H₇NO₄158.0, found 158.0.

### Step2: Synthesis of3-(3-methoxyisoxazol-5-yl)-3-oxopropanenitrile (3)

A mixture of MeCN (553 mg, 13.5 mmol) and *t*-BuOK (1.5 g, 13.5 mmol) in dry THF (25 mL) and toluene (15 mL) was added methyl 3-methoxyisoxazole-5-carboxylate (850 mg, 5.4 mmol) at rt. The reaction solution was stirred at 80 °C continuously for 24h, and monitored by TLC until all the starting material was consumed completely. 50 mL of water was added. The aqueous phase was extracted by EA for two times (25 mL X 2), and the organic layer was combined and washed with brine (25 mL X 3), and dried over anhydrous Na₂SO₄. The organic layer was concentrated in vacuo to afford the crude product which was purified by silica chromatograph (100g, 200-300 m, eluted by PE / EA=3/1) to afford the desired product as bright yellow solid (650 mg, 72%). MS [M+H]⁺ calcd for C₇H₆N₂O₃167.0, found 167.0.

### Step3: Synthesis of (E)-3-(3,4-dihydroxy-5-nitrophenyl)-2-(3-methoxyisoxazole-5-carbonyl)acrylonitrile (5)

A mixture of 3-(3-methoxyisoxazol-5-yl)-3-oxopropanenitrile (200 mg, 1.2 mmol), 3,4-dihydroxy-5-nitrobenzaldehyde (183 mg, 1.0 mmol) and NH₄OAc (554 mg, 7.2 mmol) in MeOH (15 mL) was refluxed for 3 hours, then cooled to room temperature. The reaction mixture was filtered and the solid was collected and washed by H₂O (15 mL). The solid was re-dissolved in MeOH (5 mL) and the PH was adjusted to 3∼5 by adding 5 mL of IN aqueous HCl. The desired product was obtained by filter and dried in vacuo as bright yellow solid (250 mg, 76%). MS [MH]⁺calcd forC₁₄H₉N₃O₇332.0, found 332.0.

### Step4: Synthesis of (E)-3-(3,4-dihydroxy-5-nitrophenyl)-2-(3-hydroxyisoxazole-5-carbonyl)acrylonitrile (6)

A solution of (*E*)-3-(3,4-dihydroxy-5-nitrophenyl)-2-(3-methoxyisoxazole-5-carbonyl) acrylonitrile (150 mg, 0.45 mmol) in AcOH (5 mL) was added 0.5 mL 33% HBr (in AcOH) at rt. The reaction solution was stirred at 80 °C continuously for 3h and monitored by LC-MS until all the starting material was consumed completely. The reaction mixture was concentrated in vacuo to afford the crude product which was purified by by Prep-HPLC (0.5%TFA, MeCN/H₂O) to afford the desired product as yellow solid (22 mg, 15%). MS [MH]⁺calcd for C₁₃H₇N₃O₇ 318.0, found 318.0.¹H NMR (400 MHz, DMSO) δ 11.96 (s, 2H), 8.32 (s, 1H), 8.18 (d, *J* = 2.1 Hz, 1H), 7.88 (d, *J* = 2.2 Hz, 1H), 6.93 (s, 1H)

**Compound 687:** 687 was prepared in two synthetic steps from 2-cyanoacetate according, according to the following procedure:

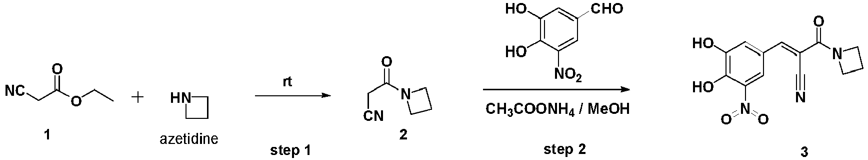

### Step1: Synthesis of 3-(azetidin-1-yl)-3-oxopropanenitrile (2)

A mixture of ethyl 2-cyanoacetate (565 mg, 5.0 mmol) and azetidine (285 mg, 5.0 mmol) was stirred at rt overnight. The reaction solution was purified by HPLC to afford the desire product. MS [M+H]⁺ calcd for C₆H₉N₂O₁ 125.1, found 125.1

### Step2: Synthesis of 3-(azetidin-1-yl)-3-oxopropanenitrile (3)

A mixture of 3-(azetidin-1-yl)-3-oxopropanenitrile (124 mg, 1.0 mmol), 3,4-dihydroxy-5-nitrobenzaldehyde (183 mg, 1.0 mmol) and CH₃COONH₄ (770 mg, 10.0 mmol) in MeOH (15 mL) was stirred at 80 °C overnight and then cooled to rt. The reaction mixture was filtered to collect the solid. The solid was re-dissolved in MeOH and the PH was adjusted to 3∼5 by adding IN HCl. The desire product was collected by filtered and dried in vavuo. (116 mg, 40%). MS [M+H]⁺ calcd for C₁₃H₁₂N₃O₅ 290.2, found 289.9.¹H NMR (400 MHz, DMSO) δ 10.93 (s, 2H), 8.01 (d, *J* = 2.1 Hz, 1H), 7.97 (s, 1H), 7.81 (d, *J* = 2.1 Hz, 1H), 4.48 (t, *J* = 7.2 Hz, 2H), 4.03 (t, *J* = 7.3 Hz, 2H), 2.32 - 2.22 (m, 2H).

**Compound 688:** 688 was prepared in two synthetic steps from 2-cyanoacetate according to the following procedure:

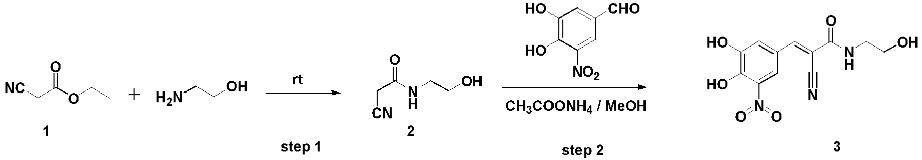

### Step1: Synthesis of 2-cyano-N-(2-hydroxyethyl)acetamide (2)

A mixture of ethyl 2-cyanoacetate (113 mg, 1.0 mmol) and 2-aminoethanol (305 mg, 5.0 mmol) was stirred at rt overnight. The reaction solution was purified by HPLC to afford the desire product. MS [M+H]⁺ calcd for C₅H₉N₂O₂ 129.1, found 129.1

### Step2: Synthesis of (E)-2-cyano-3-(3,4-dihydroxy-5-nitrophenyl)-N-(2-hydroxyethyl)acrylamide (3)

A mixture of 2-cyano-N-(2-hydroxyethyl)acetamide (65 mg, 0.5 mmol), 3,4-dihydroxy-5-nitrobenzaldehyde (92 mg, 0.5 mmol) in MeOH (15 mL) and CH₃COONH₄ (385 mg, 5.0 mmol) was stirred at 80 °C overnight and then cooled to rt. The reaction mixture was filtered to collect the solid. The solid was re-dissolved in MeOH and the PH was adjusted to 3∼5 by adding IN HCl. The desire product was collected by filtered and dried in vavuo. (80 mg, 54%). MS [M+H]⁺ calcd for C₁₃H₁₂N₃O₆ 294.2, found 293.8. ¹H NMR (400 MHz, DMSO) δ 10.92 (s, 2H), 8.30 (t, *J* = 5.5 Hz, 1H), 8.06 (s, 1H), 7.95 (d, *J* = 2.1 Hz, 1H), 7.78 (d, *J* = 2.2 Hz, 1H), 3.49 (t, *J* = 6.1 Hz, 2H), 3.28 (q, *J* = 5.9 Hz, 2H), 3.17 (s, 1H).

**Compound 691:** 691 was prepared in two synthetic steps from 3,4-dimethoxy-5-nitrobenzoic acid, according to the following procedure:

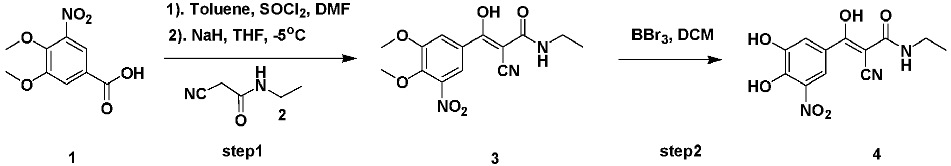

### Step1: Synthesis of (Z)-2-cyano-3-(3,4-dimethoxy-5-nitrophenyl)-3-hydroxy-N-ethylacrylamide (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to the solution of 2-cyano-N-ethylacetamide (0.23 g, 2.0 mmol) in anhydrous THF (5 mL) at -5 °C. The resulting suspension was stirred at -5 °C for 15 min and the solution of 3,4-dimethoxy-5-nitrobenzoyl chloride in THF was added over 10 min and stirred for an additional 1h at -5 °C. The reaction mixture was warmed to 0 °C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the title compound 3 as an yellow solid (288 mg, 39 %).

### Step2: Synthesis of (Z)-2-cyano-3-(3,4-dihydroxy-5-nitrophenyl)-N-ethyl-3-hydroxyacrylamide (4)

A solution of (Z)-2-cyano-3-(3,4-dimethoxy-5-nitrophenyl)-3-hydroxy-N-ethylacrylamide (167 mg, 0.5 mmol) in DCM (5 mL) was added 1.0 M solution of BBr₃ in DCM (3 mL, 3 mmol) at -15 °C under nitrogen atmosphere. The resulting red suspension was stirred for 1 hour at -15°C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product as yellow solid (48 mg, 33%).1H NMR (400 MHz, DMSO) δ 14.18 (s, 1H), 10.74 (s, 2H), 9.12 (s, 1H), 8.75 (d, J = 5.2 Hz, 1H), 7.88 (s, 1H), 7.55 (d, J = 2.1 Hz, 1H), 7.45 (d, J = 4.7 Hz, 1H), 4.52 (s, 2H). MS [M+H]⁺ calcd for C₁₂H₁₂N₃O₆ 294.2, found 293.8

**Compound 692:** 692 was prepared in two synthetic steps from 3,4-dimethoxy-5-nitrobenzoic acid, according to the following procedure:

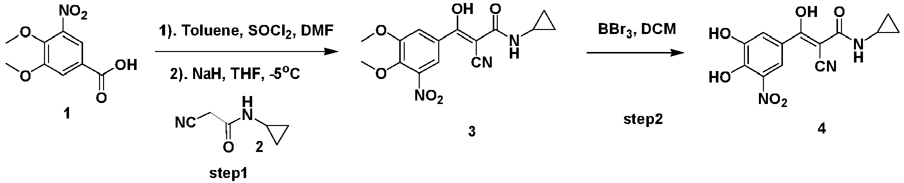

### Step1: Synthesis of (Z)-2-cyano-N-cyclopropyl-3-(3,4-dimethoxy-5-nitrophenyl)-3-hydroxyacrylamide (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to the solution of 2-cyano-N-cyclopropylacetamide (250 mg, 2.0 mmol) in anhydrous THF (5 mL) at -5 °C. The resulting suspension was stirred at -5 °C for 15 min and the solution of 3,4-dimethoxy-5-nitrobenzoyl chloride in THF was added over 10 min and stirred for an additional 1h at -5 °C. The reaction mixture was warmed to 0 °C, quenched by the addition of 1N.HCl solution (4 mL) and stirred for 10 min at room temperature, extracted by ethyl acetate (25 mL ^{∗}2), the organic layers was dried with Na₂SO₄ and concentrated in vacuo to give the compound **3** as an yellow solid (242 mg, 36%).

### Step2: Synthesis of (Z)-2-cyano-N-cyclopropyl-3-(3,4-dihydroxy-5-nitrophenyl)-3-hydroxyacrylamide (4)

A solution of (Z)-2-cyano-N-cyclopropyl-3-(3,4-dimethoxy-5-nitrophenyl)-3-hydroxyacrylamide (166 mg, 0.5 mmol) in DCM (5 mL) was added 1.0 M solution of BBr₃ in DCM (3 mL, 3 mmol) at -15 °C under nitrogen atmosphere. The resulting red suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product as yellow solid (25 mg, 26%). ¹H NMR (400 MHz, DMSO) δ 10.81 (s, 1H), 8.82 (s, 1H), 7.88 (d, J = 1.9 Hz, 1H), 7.54 (d, J = 2.1 Hz, 1H), 2.83 - 2.77 (m, 1H), 0.74 - 0.64 (m, 4H). MS [M-H]⁻ calcd for C₁₃H₁₀N₃O₆ 304.2, found 303.9.

**Compound 697:** 697 was prepared in two synthetic steps from methyl 5-(cyanomethyl)pyrazine-2-carboxylate, according to the following procedure:

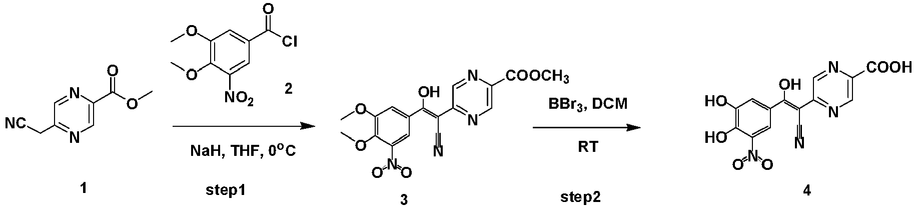

### Step1: Synthesis of (E)-methyl 5-(1-cyano-2-(3,4-dimethoxy-5-nitrophenyl)-2-hydroxyvinyl)pyrazine-2-carboxylate (3)

Under a nitrogen atmosphere, 60% NaH (0.072 g, 1.8 mmol) was added to the solution of methyl 5-(cyanomethyl)pyrazine-2-carboxylate (0.15 g, 0.9 mmol) in anhydrous THF (5 mL) at -5 °C. The resulting suspension was stirred at -5 °C for 15 min and the solution of 3,4-dimethoxy-5-nitrobenzoyl chloride in THF was added over 10 min and stirred for an additional 1hour at -5 °C. The reaction mixture was warmed to 0 °C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the title compound (E)-methyl 5-(1-cyano-2-(3,4-dimethoxy-5-nitrophenyl)-2-hydroxyvinyl)pyrazine-2-carboxylate as an orange solid(125 mg, 30%). MS [M+H]⁺ calcd for C₁₇H₁₄N₄O₇ 387.0, found 387.0.

### Step2: Synthesis of (E)-5-(1-cyano-2-(3,4-dihydroxy-5-nitrophenyl)-2-hydroxyvinyl) pyrazine-2-carboxylic acid (4)

A solution of (E)-methyl 5-(1-cyano-2-(3,4-dimethoxy-5-nitrophenyl)-2-hydroxyvinyl) pyrazine-2-carboxylate (125 mg, 0.3 mmol) in DCM (5 mL) was added 1.0 M solution of BBr₃ in DCM (3 mL, 3 mmol) at -15 °C under nitrogen atmosphere. The resulting red suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC (0.5% TFA, MeOH / H₂O) gave the desired product as yellow solid (15 mg, 15%). 1H NMR (400 MHz, DMSO) δ 10.57 (s, 1H), 9.17 (s, 1H),8.68 (s, 1H), 7.81 (s, 1H), 7.51 (s, 1H). MS [M+H]⁺ calcd for C₁₄H_{g}N₄O₇ 345.0, found 345.0

**Compound 701:** 701 was prepared in two synthetic steps from 3,4-dimethoxy-5-nitrobenzoic acid, according to the following procedure:

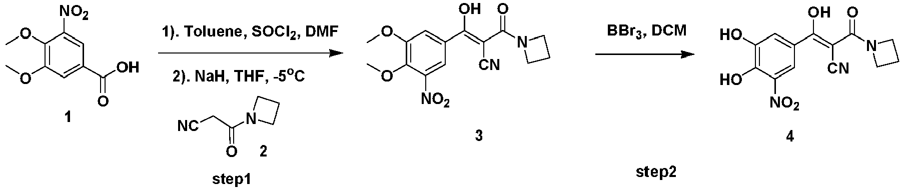

### Step1: Synthesis of (Z)-2-(azetidine-1-carbonyl)-3-(3,4-dimethoxy-5-nitrophenyl)-3-hydroxyacrylonitrile (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to the solution of 2-cyano-N-cyclopropylacetamide (250 mg, 2.0 mmol) in anhydrous THF (5 mL) at -5 °C. The resulting suspension was stirred at -5°C for 15 min and the solution of 3,4-dimethoxy-5-nitrobenzoyl chloride in THF was added over 10 min and stirred for an additional 1h at -5 °C. The reaction mixture was warmed to 0 °C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the compound **3** (146 mg, 22%).

### Step2: Synthesis of (Z)-2-(azetidine-1-carbonyl)-3-(3,4-dihydroxy-5-nitrophenyl)-3-hydroxyacrylonitrile (4)

A solution of (Z)-2-(azetidine-1-carbonyl)-3-(3,4-dimethoxy-5-nitrophenyl)-3-hydroxyacrylonitrile (146 mg, 0.4 mmol) in DCM (5 mL) was added 1.0 M solution of BBr₃ in DCM (2 mL, 2 mmol) at -15 °C under nitrogen atmosphere. The resulting red suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product (18 mg, 15%). 11H NMR (400 MHz, DMSO) δ 10.93 (s, 2H), 7.93 (d, J = 2.1 Hz, 1H), 7.55 (d, J = 2.1 Hz, 1H), 4.36 (s, 4H), 2.35 - 2.27 (m, 2H). MS [M+H]+ calcd for C13H12N3O6 306.2, found 305.9

**Compound 711:** 711 was prepared in two synthetic steps from 3-oxo-3-(thiazol-4-yl)propanenitrile, according to the following procedure:

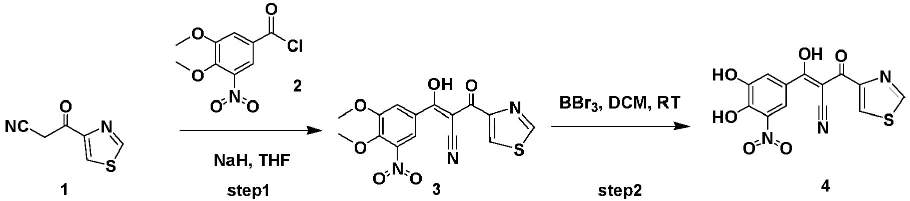

### Step1: Synthesis of (Z)-3-(3,4-dimethoxy-5-nitrophenyl)-3-hydroxy-2-(thiazole-4-carbonyl)acrylonitrile (2)

Under a nitrogen atmosphere, 60% NaH (0.072 g, 1.8 mmol) was added to the solution of 3-oxo-3-(thiazol-4-yl)propanenitrile (0.41 g, 2.7 mmol) in anhydrous THF (15 mL) at -5 °C. The resulting suspension was stirred at -5°C for 15 min and the solution of 3,4-dimethoxy-5-nitrobenzoyl chloride (661 mg, 2.7 mmol) in THF was added over 5 min and stirred for an additional 1hour at -5 °C. The reaction mixture was warmed to 0 °C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the title compound (E)-methyl 5-(1-cyano-2-(3,4-dimethoxy-5-nitrophenyl)-2-hydroxyvinyl)pyrazine-2-carboxylate as an orange solid(600 mg, 62%). MS [M+H]+ calcd for C₁₅H₁₁N₃O₆S 362.0, found 362.0.

### Step2: Synthesis of (Z)-3-(3,4-dihydroxy-5-nitrophenyl)-3-hydroxy-2-(thiazole-4-carbonyl)acrylonitrile (4)

A solution of (Z)-3-(3,4-dimethoxy-5-nitrophenyl)-3-hydroxy-2-(thiazole-4-carbonyl)acrylonitrile(70 mg, 0.2 mmol) in DCM (5 mL) was added 1.0 M solution of BBr₃ in DCM (1 mL, 1 mmol) at -15 °C under nitrogen atmosphere. The resulting red suspension was stirred for 1 hour at -15 °C and allowed to warm to room temperature overnight. The reaction was quenched by the addition of 0.5 N.NH₄OH (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC (0.5% TFA, MeOH / H₂O) gave the desired product as yellow solid (11 mg, 17%). 1H NMR (400 MHz, DMSO) δ 10.66 (s, 1H), 8.61 (s,1H), 7.97 (s, 1H), 7.65 (s, 1H). MS [M+H]⁺ calcd for C₁₃H₇N₃O₆S 334.0, found 334.0.

**Compound 709:** 709 was prepared in two synthetic steps from (E)-2-cyano-3-(3,4-dihydroxy-5-nitrophenyl)acrylic acid, according to the following procedure:

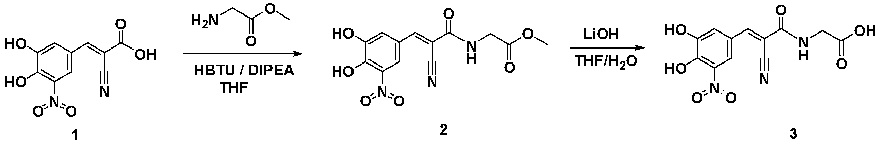

### Step1: Synthesis of (E)-methyl 2-(2-cyano-3-(3,4-dihydroxy-5-nitrophenyl)acrylamido)acetate (2)

A solution of (E)-2-cyano-3-(3,4-dihydroxy-5-nitrophenyl)acrylic acid (1) (250 mg, 1 mmol) and methyl 2-aminoacetate (89 mg, 1mmol) in THF (15 mL) was added HBTU (600 mg, 1.5 mmol) and DIPEA (388 mg, 3 mmol) at rt. The resulting suspension was stirred for 5 hours at 60 °C and allowed to cool to room temperature overnight. The reaction was added H₂O and extracted with ethyl acetate (30 mL ^{∗} 3). The organic layers were combined to afford the crude product which was used in the next step without further purification (120 mg, 37.4%).

### Step2: Synthesis of (E)-2-(2-cyano-3-(3,4-dihydroxy-5-nitrophenyl)acrylamido)acetic acid (3)

A solution of (E)-methyl 2-(2-cyano-3-(3,4-dihydroxy-5-nitrophenyl)acrylamido)acetate (2) (120 mg, 0.37 mmol) in THF / H₂O (5 mL/ 5 mL) was added LiOH (24 mg, 0.55 mmol) at 0 °C. The resulting suspension was stirred for 1 hour at 0 °C and was quenched by adding aqueous HCl. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product (7 mg, 6.2%). 1H NMR (400 MHz, DMSO) δ 8.65 (t, J = 5.8 Hz, 1H), 8.10 (s, 1H), 7.98 (d, J = 2.1 Hz, 1H), 7.79 (d, J = 2.1 Hz, 1H), 3.88 (d, J = 5.8 Hz, 2H). MS [M+H]⁺ calcd for C₁₂H₁₀N₃O₇ 308.2, found 308.0.

**Compound 693:** 693 was prepared in three synthetic steps from ethyl 2-cyanoacetate, according to the following procedure:

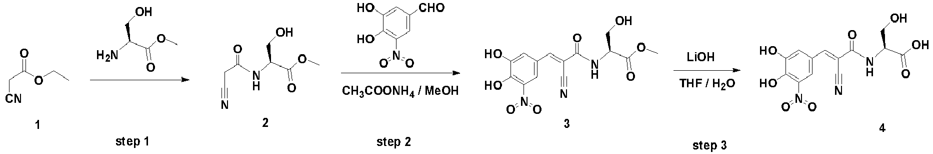

### Step1: Synthesis of (S)-methyl 2-(2-cyanoacetamido)-3-hydroxypropanoate (2)

A mixture of ethyl 2-cyanoacetate (1130 mg, 10.0 mmol) and (S)-methyl 2-amino-3-hydroxypropanoate (1190 mg, 10.0 mmol) was stirred at r.t. overnight. The reaction solution was added 10 mL MeOH and filtered to collect the solid as crude product, which was used in next step without further purification. MS [M+H]⁺ calcd for C₇H₁₁N₂O₄ 187.2, found 187.2.

### Step2: Synthesis of (S,E)-methyl 2-(2-cyano-3-(3,4-dihydroxy-5-nitrophenyl)acrylamido)-3-hydroxypropanoate (3)

A solution of (S)-methyl 2-(2-cyanoacetamido)-3-hydroxypropanoate (930 mg, 5 mmol) (2) and 3,4-dihydroxy-5-nitrobenzaldehyde (915 mg, 5 mmol) in MeOH (25 mL) was added CH₃COONH₄ (3850 mg, 50 mmol) at rt. The reaction solution was stirred at 60 °C continuously for 5h and monitored by TLC until all the starting material was consumed completely. Then the reaction mixture was cooled to rt and the solvent was eliminated under reduced pressure, then the Sat. aq. NaCl (100 mL) was added. The aqueous solution was extracted by EA for three times (50 mL ^{∗} 3). The organic layer was concentrated in *vacuo* to afford crude product which was purified by silica chromatograph chromatography to afford the desired product (705 mg, 40%). MS [M+H]⁺ calcd for C₁₄H₁₄N₃O₈ 352.3, found 352.3.

### Step3: Synthesis of (S,E)-2-(2-cyano-3-(3,4-dihydroxy-5-nitrophenyl)acrylamido)-3-hydroxypropanoic acid (4)

A solution of (S,E)-methyl 2-(2-cyano-3-(3,4-dihydroxy-5-nitrophenyl)acrylamido)-3-hydroxypropanoate (3) (705 mg, 2 mmol) in THF / H₂O (10 mL/ 10 mL) was added LiOH (126 mg, 3 mmol) at 0 °C. The resulting suspension was stirred for 1 hour at 0 °C and was quenched by adding aqueous HCl. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product (220 mg, 32.6%). 1H NMR (400 MHz, DMSO) δ 12.89 (s, 1H), 10.94 (s, 2H), 8.22 (d, J = 7.6 Hz, 1H), 8.15 (s, 1H), 7.97 (d, J = 2.1 Hz, 1H), 7.82 (d, J = 2.1 Hz, 1H), 5.05 (s, 1H), 4.41 (m, 1H), 3.79 (m, 2H). MS [M+H]⁺ calcd for C₁₃H₁₂N₃O₈ 338.2, found 337.8

**Compound 702:** 702 was prepared in three synthetic steps from ethyl 2-cyanoacetate, according to the following procedure:

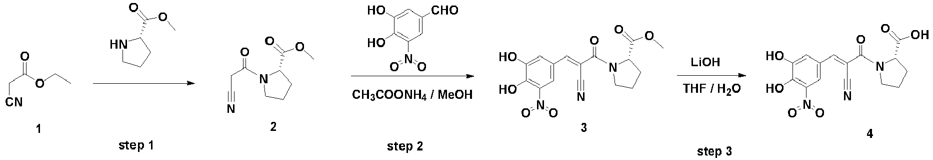

### Step1: Synthesis of (S)-methyl 1-(2-cyanoacetyl)pyrrolidine-2-carboxylate (2)

A mixture of ethyl 2-cyanoacetate (113 mg, 1.0 mmol) and (S)-methyl pyrrolidine-2-carboxylate (129 mg, 1.0 mmol) was stirred at r.t. overnight. The reaction solution was added 10 mL MeOH and filtered to collect the solid as crude product, which was used in next step without further purification. MS [M+H]⁺calcd for C₉H₁₃N₂O₃ 197.2, found 197.2.

### Step2: Synthesis of (S,E)-methyl 2-(2-cyano-3-(3,4-dihydroxy-5-nitrophenyl)acrylamido)-3-hydroxypropanoate (3)

A solution of (S)-methyl 1-(2-cyanoacetyl)pyrrolidine-2-carboxylate (2) (196 mg, 1 mg) and 3,4-dihydroxy-5-nitrobenzaldehyde (183 mg, 1 mmol) in MeOH (5 mL) was added CH₃COONH₄ (770 mg, 10 mmol) at rt. The reaction solution was stirred at 60 °C overnight and monitored by TLC until all the starting material was consumed completely. Then the reaction mixture was cooled to r.t. and the solvent was eliminated under reduced pressure, then the Sat. aq. NaCl (100 mL) was added. The aqueous solution was extracted by EA for three times (50 mL ^{∗} 3). The organic layer was concentrated in *vacuo* to afford crude product which was purified by silica chromatograph chromatography to afford the desired product (43 mg, 12%). MS [M+H]⁺calcd for C₁₆H₁₆N₃O₇ 362.3, found 362.3.

### Step3: Synthesis of (S,E)-1-(2-cyano-3-(3,4-dihydroxy-5-nitrophenyl)acryloyl)pyrrolidine-2-carboxylic acid (4)

A solution of (S,E)-methyl 2-(2-cyano-3-(3,4-dihydroxy-5-nitrophenyl)acrylamido)-3-hydroxypropanoate (3) (43 mg, 0.12 mmol) in THF / H₂O (2 mL/ 1 mL) was added LiOH (8 mg, 0.18 mmol) at 0 °C. The resulting suspension was stirred for 1 hour at 0 °C and was quenched by adding aqueous HCl. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product (6 mg, 15%). 1H NMR (400 MHz, DMSO) δ 7.25 (t, J = 7.4 Hz, 1H), 7.21 - 7.09 (m, 2H), 2.30 (s, 2H), 2.07 (s, 4H). MS [M+H]⁺ calcd for C₁₅H₁₄N₃O₇ 348.3, found 347.8

**Compound 347N:** 347N was prepared in single synthetic step from 2-cyano-3-(3,4-dihydroxy-5-nitrophenyl)-*N*,*N*-diethyl-3-oxopropanamide (1, compound 347), according to the following procedure:

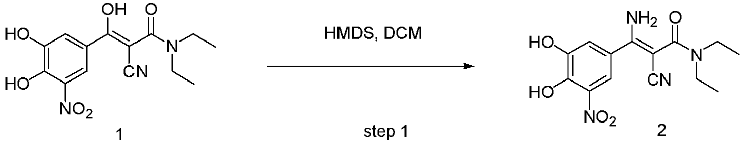

### Step1: Synthesis of 2-cyano-3-(3,4-dihydroxy-5-nitrophenyl)-N,N-diethyl-3-hydroxyacrylamide (2)

A solution of 2-cyano-3-(3,4-dihydroxy-5-nitrophenyl)-*N,N*-diethyl-3-oxopropanamide (350 mg, 1 mmol) in DCM (5 mL) was added HMDS (0.5 mL) at rt, then stirred for 48h. After the reaction was completed, the reaction mixture was concentrated in vacuo to afford the crude product, further purification by Prep-HPLC afforded the desired product as yellow solid (60 mg, 19%). MS [M+H]⁺ calcd for C₁₄H₁₆N₄O₅ 321.1, found 321.1.

**Compound 661N:** 661N was prepared in one synthetic step from (Z)-3-(3,4-dihydroxy-5-nitrophenyl)-3-hydroxy-2-(piperidine-1-carbonyl)acrylonitrile, according to the following procedure:

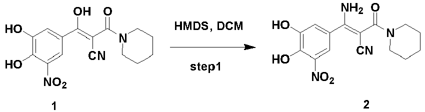

### Step1: Synthesis of 3-amino-3-(3,4-dihydroxy-5-nitrophenyl)-2-(piperidine-1-carbonyl)acrylonitrile (2)

A solution of 3-(3,4-dihydroxy-5-nitrophenyl)-3-hydroxy-2-(piperidine-1-carbonyl)acrylonitrile (20 mg, 0.06 mmol) in DCM (5 mL) was added HMDS (1 mL) and stirred at rt for 72h. And then the reaction mixture was concentrated in vacuo to afford the crude product, which was further purified by HPLC to afford the desired product as a yellow solid (1 mg, 5%). MS [MH]+ calcd for C₁₅H₁₆N₄O₅ 333.3, found 333.1. 1H NMR (400 MHz, DMSO) δ 8.21 (s, 1H), 7.66 (s,1H), 6.74 (s, 1H), 1.25-1.65 (m, 6H), 1.23 (s, 1H).

**Compound 691N:** 691N was prepared in four synthetic steps from 3,4-dimethoxy-5-nitrobenzoic acid, according to the following procedure:

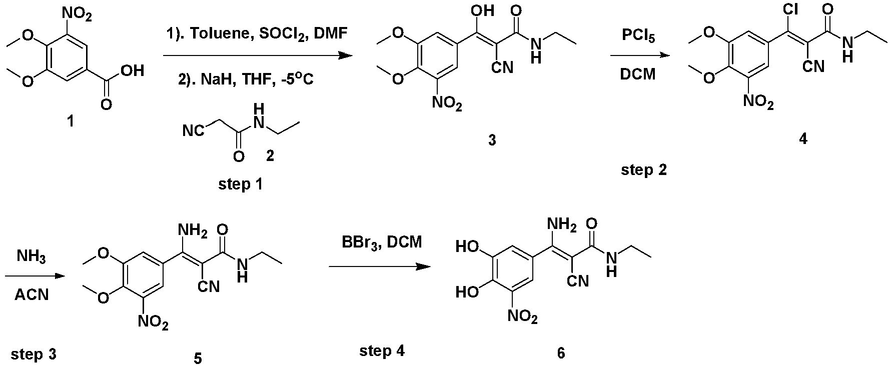

### Step1: Synthesis of (Z)-2-cyano-3-(3,4-dimethoxy-5-nitrophenyl)-3-hydroxy-N-ethylacrylamide (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60°C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18g, 4.4 mmol) was added to the solution of 2-cyano-N-ethylacetamide (0.23 g, 2.0 mmol) in anhydrous THF (5 mL) at -5°C. The resulting suspension was stirred at -5°C for 15 min and the solution of 3,4-dimethoxy-5-nitrobenzoyl chloride in THF was added over 10 min and stirred for an additional 1hour at - 5°C. The reaction mixture was warmed to 0°C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the title compound **3** as an yellow solid(265 mg, 36%).

### Step2: Synthesis of (Z)-3-chloro-2-cyano-3-(3,4-dimethoxy-5-nitrophenyl)-N-ethylacrylamide (4)

A solution of (Z)-2-cyano-3-(3,4-dimethoxy-5-nitrophenyl)-3-hydroxy-N-ethylacrylamide (176 mg, 0.5 mmol) in DCM (5 mL) was added PCl₅ (104 mg, 0.5 mmol) at 0 °C. The resulting suspension was stirred at overnight until all the start materials were consumed detected by LC-MS. Then the reaction mixture was allowed to cool to room temperature and used in the next step without further purification.

### Step3: Synthesis of (Z)-3-amino-2-cyano-3-(3,4-dimethoxy-5-nitrophenyl)-N-ethylacrylamide (5)

A solution of (Z)-3-chloro-2-cyano-3-(3,4-dimethoxy-5-nitrophenyl)-N-ethylacrylamide (150 mg, 0.4 mmol) in DCM was added a saturated solution of NH₃ in ACN at 0 °C, and then the reaction solution was stirred at 0 °C continuously until all the starting materials were consumed completely. The reaction was quenched by the addition of H₂O and stirred for 30 min. The aqueous phase was extracted with ethyl acetate. The organic layers were combined, washed with brine and dried with Na₂SO₄. The solvent was eliminated under reduced pressure to give the crude product.

### Step4: Synthesis of (Z)-3-amino-2-cyano-3-(3,4-dihydroxy-5-nitrophenyl)-N-ethylacrylamide (6)

A solution of (Z)-3-amino-2-cyano-3-(3,4-dimethoxy-5-nitrophenyl)-N-ethylacrylamide (67 mg, 0.2 mmol) in DCM (5 mL) was added 1.0 M solution of BBr₃ in DCM (1 mL, 1 mmol) at -15 °C under nitrogen atmosphere. The resulting red suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product as yellow solid (5 mg, 9%). 1H NMR (400 MHz, DMSO) δ 10.53 (s, 2H), 9.44 (s, 1H), 8.02 (s, 2H), 7.63 (d, J = 2.0 Hz, 1H), 7.36 (d, J = 2.0 Hz, 1H), 3.32 - 3.25 (m, 2H), 1.14 (t, J = 7.1 Hz, 3H). MS [M+H]⁺ calcd for C₁₂H₁₃N₄O₅ 293.2, found 292.8.

**Compound 692N:** 692N was prepared in four synthetic steps from 3,4-dimethoxy-5-nitrobenzoic acid, according to the following procedure:

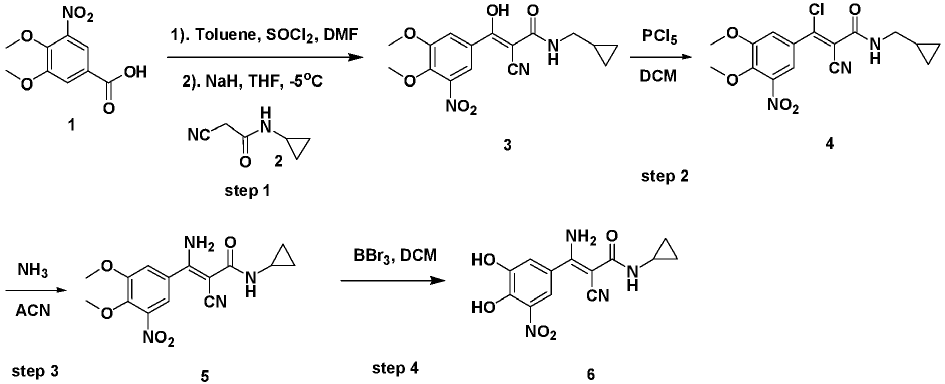

### Step1: Synthesis of (Z)-2-cyano-N-(cyclopropylmethyl)-3-(3,4-dimethoxy-5-nitrophenyl)-3-hydroxyacrylamide (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to the solution of 2-cyano-N-cyclopropylacetamide (250 mg, 2.0 mmol) in anhydrous THF (5 mL) at -5 °C. The resulting suspension was stirred at -5 °C for 15 min and the solution of 3,4-dimethoxy-5-nitrobenzoyl chloride in THF was added over 10 min and stirred for an additional 1hour at -5 °C. The reaction mixture was warmed to 0 °C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the title compound **3** as an yellow solid(165 mg, 23%).

### Step2: Synthesis of (Z)-3-chloro-2-cyano-N-(cyclopropylmethyl)-3-(3,4-dimethoxy-5-nitrophenyl)acrylamide (4)

A solution of (Z)-2-cyano-3-(3,4-dimethoxy-5-nitrophenyl)-3-hydroxy-N-propylacrylamide (165 mg, 0.5 mmol) in DCM (5 mL) was added PCl₅ (104 mg, 0.5 mmol) at 0 °C. The resulting suspension was stirred at overnight until all the start materials were consumed detected by LC-MS. Then the reaction mixture was allowed to cool to room temperature and used in the next step without further purification.

### Step3: Synthesis of (Z)-3-amino-2-cyano-N-cyclopropyl-3-(3,4-dimethoxy-5-nitrophenyl)acrylamide (5)

A solution of (Z)-3-chloro-2-cyano-N-(cyclopropylmethyl)-3-(3,4-dimethoxy-5-nitrophenyl)acrylamide (164 mg, 0.5 mmol) in DCM was added a saturated solution of NH₃ in ACN at 0 °C, and then the reaction solution was stirred at 0 °C continuously until all the starting materials were consumed completely. The reaction was quenched by the addition of H₂O and stirred for 30 min. The aqueous phase was extracted with ethyl acetate. The organic layers were combined, washed with brine and dried with Na₂SO₄. The solvent was eliminated under reduced pressure to give the crude product which was used in the next step without further purification.

### Step4: Synthesis of (Z)-3-amino-2-cyano-N-cyclopropyl-3-(3,4-dihydroxy-5-nitrophenyl)acrylamide (6)

A solution of (Z)-3-amino-2-cyano-N-cyclopropyl-3-(3,4-dimethoxy-5-nitrophenyl)acrylamide (66 mg, 0.2 mmol) in DCM (5 mL) was added 1.0 M solution of BBr₃ in DCM (1 mL, 1 mmol) at -15 °C under nitrogen atmosphere. The resulting red suspension was stirred for 1 hour at -15°C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product. (7 mg, 12%).1H NMR (400 MHz, DMSO) δ 10.52 (s, 2H), 9.53 (s, 1H), 7.63 (d, J = 2.0 Hz, 1H), 7.35 (d, J = 2.0 Hz, 1H), 2.61 - 2.55 (m, 1H), 0.86 - 0.81 (m, 2H), 0.66 - 0.61 (m, 2H). MS [M+H]⁺ calcd for C₁₃H₁₃N₄O₅ 305.3, found 304.9

**Compound 697N:** 697N was prepared in two synthetic steps from (E)-methyl 5-(1-cyano-2-(3,4-dimethoxy-5-nitrophenyl)-2-hydroxyvinyl)pyrazine-2-carboxylate, according to the following procedure:

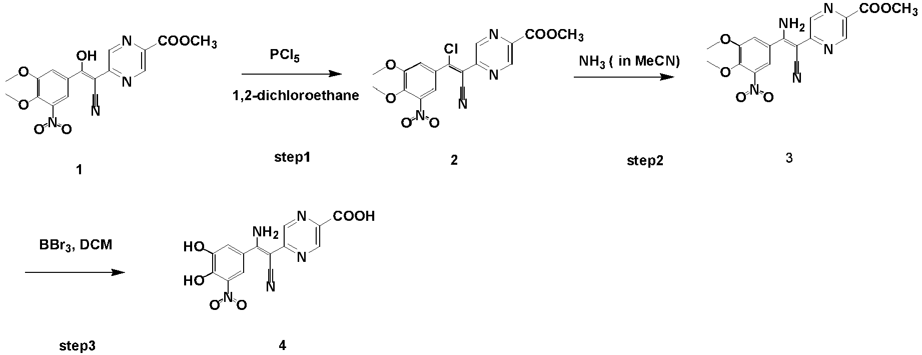

### Step1: Synthesis of (E)-methyl 5-(2-chloro-1-cyano-2-(3,4-dimethoxy-5-nitrophenyl)vinyl)pyrazine-2-carboxylate (2)

A solution of (E)-methyl 5-(1-cyano-2-(3,4-dimethoxy-5-nitrophenyl)-2-hydroxyvinyl) pyrazine-2-carboxylate (150 mg, 0.39 mmol) in 1,2-dichloroethane (25 mL) was added PCl₅ (83 mg, 0.40 mmol) at 0 °C. The resulting suspension was stirred at overnight until all the start materials were consumed detected by LC-MS. Then the reaction mixture was allowed to cool to room temperature and used in the next step without further purification.

### Step2: Synthesis of (E)-methyl 5-(2-amino-1-cyano-2-(3,4-dimethoxy-5-nitrophenyl)vinyl)pyrazine-2-carboxylate (3)

A solution of (E)-methyl 5-(2-chloro-1-cyano-2-(3,4-dimethoxy-5-nitrophenyl)vinyl) pyrazine-2-carboxylate (180 mg, 0.45 mmol) in 1,2-dichloroethane (25 mL) was added a saturated solution of NH₃ in ACN at 0 °C, and then the reaction solution was stirred at 0 °C continuously until all the starting materials were consumed completely. The reaction was quenched by the addition of H₂O and stirred for 30 min. The aqueous phase was extracted with ethyl acetate. The organic layers were combined, washed with brine and dried with Na₂SO₄. The solvent was eliminated under reduced pressure to give the crude product which was used in the next step without further purification.

### Step3: Synthesis of (E)-methyl 5-(2-chloro-1-cyano-2-(3,4-dimethoxy-5-nitrophenyl)vinyl)pyrazine-2-carboxylate (4)

A solution of (E)-methyl 5-(2-amino-1-cyano-2-(3,4-dimethoxy-5-nitrophenyl)vinyl) pyrazine-2-carboxylate (98 mg, 0.25 mmol) in DCM (5 mL) was added 1.0 M solution of BBr₃ in DCM (3 mL, 3 mmol) at -15 °C under nitrogen atmosphere. The resulting red suspension was stirred for 1 hour at -15 °C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC (0.5%TFA, MeOH / H₂O) gave the desired product as yellow solid (11 mg, 13%). 1H NMR (400 MHz, DMSO) δ 10.96-11.2 (m, 1H), 10.42 (s, 1H), 8.94 (s, 1H), 8.66 (s, 1H), 7.76 (s, 1H), 6.79 (s, 1H). MS [M+H]⁺ calcd for C₁₄H₉N₅O₆ 344.0, found 344.0

**Compound 701N:** 701N was prepared in four synthetic steps from 3,4-dimethoxy-5-nitrobenzoic acid, according to the following procedure:

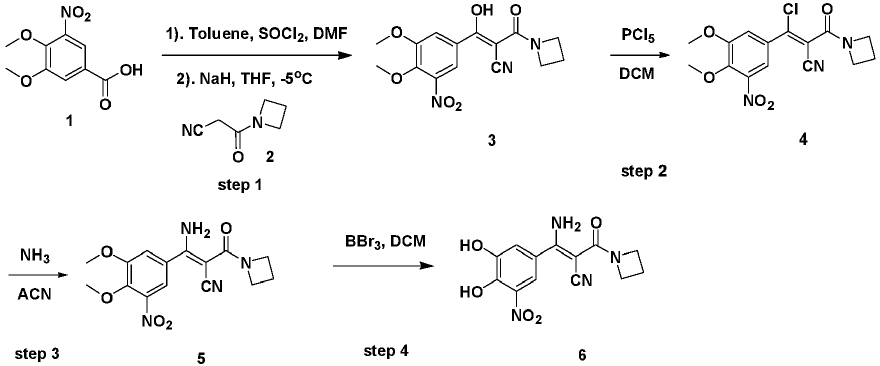

### Step1: Synthesis of (Z)-2-(azetidine-1-carbonyl)-3-(3,4-dimethoxy-5-nitrophenyl)-3-hydroxyacrylonitrile (3)

Under a nitrogen atmosphere, SOCl₂ (0.38 mL, 5.28 mmol) and anhydrous DMF (0.01 mL, 0.22 mmol) were added to a suspension of 3,4-dimethoxy-5-nitrobenzoic acid (500 mg, 2.2 mmol) in toluene (10 mL) at room temperature. The mixture was heated at 60 °C and stirred for 15 hours. The organic solvent was eliminated by distillation under reduced pressure. More toluene was added and eliminated again. The resulting yellowish solid 3,4-dimethoxy-5-nitrobenzoyl chloride was dissolved in anhydrous THF (5 mL).

Under a nitrogen atmosphere, 60% NaH (0.18 g, 4.4 mmol) was added to the solution of 3-(azetidin-1-yl)-3-oxopropanenitrile (250 mg, 2.0 mmol) in anhydrous THF (5 mL) at -5 °C. The resulting suspension was stirred at -5 °C for 15 min and the solution of 3,4-dimethoxy-5-nitrobenzoyl chloride in THF was added over 10 min and stirred for an additional 1hour at -5 °C. The reaction mixture was warmed to 0 °C, quenched by the addition of 1N.HCl solution (4 mL) and stirred for 10 min at room temperature, extracted by ethyl acetate (25 mL ^{∗} 2), the organic layers was dried with Na₂SO₄ and concentrated in vacuo to give the compound **3.**

### Step2: Synthesis of (Z)-2-(azetidine-1-carbonyl)-3-chloro-3-(3,4-dimethoxy-5-nitrophenyl)acrylonitrile (4)

A solution of (Z)-2-(azetidine-1-carbonyl)-3-(3,4-dimethoxy-5-nitrophenyl)-3-hydroxyacrylonitrile (175 mg, 0.5 mmol) in DCM (5 mL) was added PCl₅ (104 mg, 0.5 mmol) at 0 °C. The resulting suspension was stirred at overnight until all the start materials were consumed detected by LC-MS. Then the reaction mixture was allowed to cool to room temperature and used in the next step without further purification.

### Step3: Synthesis of (Z)-3-amino-2-(azetidine-1-carbonyl)-3-(3,4-dimethoxy-5-nitrophenyl)acrylonitrile (5)

A solution of (Z)-2-(azetidine-1-carbonyl)-3-chloro-3-(3,4-dimethoxy-5-nitrophenyl)acrylonitrile (164 mg, 0.5 mmol) in DCM was added a saturated solution of NH₃ in ACN at 0 °C, and then the reaction solution was stirred at 0 °C continuously until all the starting materials were consumed completely. The reaction was quenched by the addition of H₂O and stirred for 30 min. The aqueous phase was extracted with ethyl acetate. The organic layers were combined, washed with brine and dried with Na₂SO₄. The solvent was eliminated under reduced pressure to give the crude product which was used in the next step without further purification.

### Step4: Synthesis of (Z)-3-amino-2-(azetidine-1-carbonyl)-3-(3,4-dihydroxy-5-nitrophenyl)acrylonitrile (6)

A solution of (Z)-3-amino-2-(azetidine-1-carbonyl)-3-(3,4-dimethoxy-5-nitrophenyl)acrylonitrile (66 mg, 0.2 mmol) in DCM (5 mL) was added 1.0 M solution of BBr₃ in DCM (1 mL, 1 mmol) at -15 °C under nitrogen atmosphere. The resulting red suspension was stirred for 1 hour at -15°C and allowed to warm to room temperature overnight. The reaction was quenched by the addition of H₂O (2 mL) and stirred for 30 min. The aqueous phase was extracted with ethyl acetate (30 mL ^{∗} 3). The organic layers were combined, washed with brine and dried with Na₂SO₄. The solvent was eliminated under reduced pressure to give the crude product. Further purification by Prep-HPLC gave the desired product (7 mg, 12%). 1H NMR (400 MHz, DMSO) δ 10.52 (s, 2H), 9.87 (s, 1H), 7.60 (d, J = 2.1 Hz, 1H), 7.35 (d, J = 2.1 Hz, 1H), 4.26 (s, 4H), 2.29 (m, 2H). MS [M+H]⁺ calcd for C₁₃H₁₃N₄O₅ 305.3, found 305.0

**Compound 711N:** 711N was prepared in three synthetic steps from (Z)-3-(3,4-dimethoxy-5-nitrophenyl)-3-hydroxy-2-(thiazole-4-carbonyl)acrylonitrile, according to the following procedure:

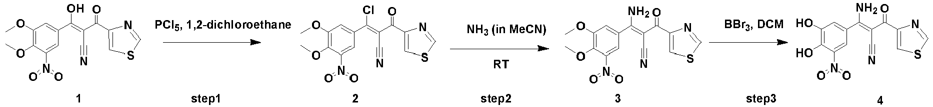

### Step1: Synthesis of (Z)-3-chloro-3-(3,4-dimethoxy-5-nitrophenyl)-2-(thiazole-4-carbonyl)acrylonitrile (2).

A solution of (Z)-3-(3,4-dimethoxy-5-nitrophenyl)-3-hydroxy-2-(thiazole-4-carbonyl)acrylonitrile (150 mg, 0.42 mmol) in 1,2-dichloroethane (25 mL) was added PCl₅ (86 mg, 0.42 mmol) at 0 °C. The resulting suspension was stirred at overnight until all the start materials were consumed detected by LC-MS. Then the reaction mixture was allowed to cool to room temperature and used in the next step without further purification.

### Step2: Synthesis of (Z)-3-amino-3-(3,4-dimethoxy-5-nitrophenyl)-2-(thiazole-4-carbonyl)acrylonitrile (3)

A solution of (Z)-3-chloro-3-(3,4-dimethoxy-5-nitrophenyl)-2-(thiazole-4-carbonyl)acrylonitrile (185 mg, 0.42 mmol) in 1,2-dichloroethane (25 mL) was added a saturated solution of NH₃ in ACN at 0 °C, and then the reaction solution was stirred at 0 °C continuously until all the starting materials were consumed completely. The reaction was quenched by the addition of H₂O and stirred for 30 min. The aqueous phase was extracted with ethyl acetate. The organic layers were combined, washed with brine and dried with Na₂SO₄. The solvent was eliminated under reduced pressure to give the crude product which was used in the next step without further purification. MS [M+H]⁺ calcd for C₁₅H₁₂N₄O₅S 361.0, found 361.0

### Step3: Synthesis of ammonium (Z)-5-(1-amino-2-cyano-3-oxo-3-(thiazol-4-yl)prop-1-enyl)-2-hydroxy-3-nitrophenolate (4)

A solution of (Z)-3-amino-3-(3,4-dimethoxy-5-nitrophenyl)-2-(thiazole-4-carbonyl)acrylonitrile (100 mg, 0.28 mmol) in DCM (5 mL) was added 1.0 M solution of BBr₃ in DCM (1 mL, 1 mmol) at -15 °C under nitrogen atmosphere. The resulting red suspension was stirred for 1 hour at -15°C and allowed to warm to room temperature overnight. The reaction was quenched by the addition of 0.5 N.NH₄OH (2 mL) and stirred for 5 min. The aqueous phase was washed with ethyl acetate. The hydrous layer was eliminated under reduced pressure to give the crude product. Further purification by Prep-HPLC (0.05% FA, MeOH / H₂O) gave the desired product as yellow solid (11 mg, 15%). 1H NMR (400 MHz, DMSO) δ 8.85 (s, 1H), 7.66 (s, 1H), 7.38 (s, 1H), 6.80-6.99 (m, 1H), 6.66-6.74 (m, 1H). MS [M+H]⁺ calcd for C₁₃H₁₁N₅O₅S 333.0, found 333.0

**Compound 347M:** 347M was prepared in three synthetic steps from 2-cyano-3-(3,4-dimethoxy-5-nitrophenyl)-N,N-diethyl-3-hydroxyacrylamide according to the following procedure:

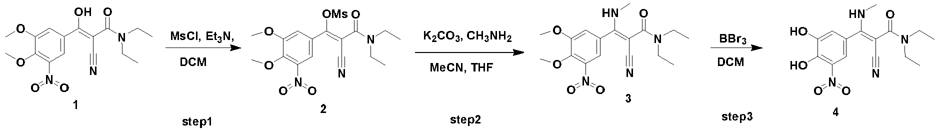

### Step1: Synthesis of 2-cyano-3-(diethylamino)-1-(3,4-dimethoxy-5-nitrophenyl)-3-oxoprop-1-enyl methanesulfonate (2)

A solution of 2-cyano-3-(3,4-dimethoxy-5-nitrophenyl)-*N,N*-diethyl-3-hydroxyacrylamide (350 mg, 1 mmol) and Et₃N (202 mg, 2 mmol) in DCM (15 mL) was added MsCl (250 mg, 2 mmol) at 0 °C. The reaction solution was stirred at rt for 3h, then it was concentrated in vacuo to afford the crude product (360 mg), which was used in the next step without further purification.

### Step2: Synthesis of 2-cyano-3-(3,4-dimethoxy-5-nitrophenyl)-N,N-diethyl-3-(methylamino)acrylamide (3)

A solution of 2-cyano-3-(diethylamino)-1-(3,4-dimethoxy-5-nitrophenyl)-3-oxoprop-1-enyl methanesulfonate (250 mg, 0.7 mmol) and K₂CO₃ (193 mg, 1.4 mmol) in MeCN (10 mL) was added MeNH₂ (1.4 mmol, 0.7 mL, 2N in THF) at rt. The reaction solution was refluxed for 2h, and then 50 mL of water was added. The aqueous phase was extracted by EA for two times (50 mL X 2), and then the organic layer was concentrated in vacuo to afford the crude product (210 mg), which was used in the next step without further purification. MS [M+H]⁺calcd for C₁₇H₂₂N₄O₅363.1., found 363.1.

### Step3: Synthesis of 2-cyano-3-(3,4-dihydroxy-5-nitrophenyl)-N,N-diethyl-3-(methylamino)acrylamide (4)

A solution of 2-cyano-3-(3,4-dimethoxy-5-nitrophenyl)-*N,N*-diethyl-3-(methylamino)acrylamide (210 mg, 0.58 mmol) in DCM (5 mL) was added 1.0 M solution of BBr₃ in DCM (2 mL, 2 mmol) at -15 °C under nitrogen atmosphere. The reaction solution was stirred at -15 °C for 1h then at room temperature overnight. The reaction was quenched by the addition of H₂O (2 mL) and stirred for 30min. The aqueous phase was extracted by ethyl acetate for three times (30 mL X 3). The organic layer was washed with brine and dried over Na₂SO₄, then it was concentrated in vacuo to afford the crude product, which was purified by Prep-HPLC (0.5%TFA, MeOH/H₂O) to gain the desired product as bright yellow solid (25 mg, 13%). MS ¹H NMR (400 MHz, DMSO) δ 10.69 (s, 2H), 7.43 (s, 1H), 7.20(s, 1H), 3.40-3.43 (m, 4H), 2.69 (d, *J* = 4.9 Hz, 3H), 1.14 (*t, J* = 6.8 Hz, 13H), 1.14 (t, *J* = 6.8 Hz, 3H). [M+H]⁺calcd for C₁₂H₇N₅O₆S 350.0, found 350.0.

**Enzymatic Inhibition.** We measured compound inhibition activity in a demethylation reaction catalyzed by FTO (US2014/0148383A1). The reaction system was incubated at 37 °C for 2 h and stopped by heating at 95 °C for 5 min. ssDNA was digested by nuclease PI and alkaline phosphatase. The concentrations of N⁶-mA and A were analyzed by HPLC-MS/MS. When concentration of substrate and enzyme are 0.5 µM and 0.1 µM, respectively, the measured IC₅₀ value of entacapone against FTO is ∼ 3 µM. The compounds of Tables 1-3 were consistently active, with IC₅₀'s less than 10 µM, and most less than 1µM.

### Exemplary IC50 Data of submicromolar representative compounds

| Compound No. | Enzymatic inhibition (IC50) µM |
|---|---|
| 687 | <1 |
| 317 | >1 |
| 371 | ∼1 |
| 660 | <1 |
| 382 | >1 |
| 702 | ∼1 |
| 698 | >1 |
| 675 | >1 |
| 394 | >1 |
| 664 | ∼1 |
| 684 | >1 |
| 688 | >1 |
| 713 | <1 |
| 709 | <1 |
| 712 | ∼1 |
| 693 | ∼1 |
| 331 | ∼1 |
| 333 | >1 |
| 318 | ∼1 |
| 365 | ∼1 |
| 366 | >1 |
| 390 | ∼1 |
| 656 | <1 |
| 666 | <1 |
| 315 | ∼1 |
| 319 | <1 |
| 389 | ∼1 |
| 502 | <1 |
| 505 | <1 |
| 395 | <1 |
| 396 | <1 |
| 522 | ∼1 |
| 655 | <1 |
| 518 | >1 |
| 520 | <1 |
| 347 | <1 |
| 351 | <1 |
| 523 | <1 |
| 524 | ∼1 |
| 525 | <1 |
| 503 | ∼1 |
| 359 | >1 |
| 374 | <1 |
| 668 | <1 |
| 661 | ∼1 |
| 673 | <1 |
| 674 | >1 |
| 691 | <1 |
| 692 | <1 |
| 697 | <1 |
| 701 | <1 |
| 711 | ∼1 |
| 715 | <1 |
| 722 | <1 |
| 347N | <1 |
| 661N | <1 |
| 691N | ∼1 |
| 692N | >1 |
| 697N | <1 |
| 701N | >1 |
| 711N | >1 |
| 347M | ∼1 |

In vivo Anti-obesity Efficacy. Male wistar rats (6 weeks) were fed with high-fat diet (45% fat, OpenSource Diets D12451), and compound (100 mg/kg) was administered to 12 randomly selected rats by gavage. After 8 weeks, the mean body weight of drug treatment group was less than that of control group. However, the body-weight-normalized food intakes of the two groups showed no difference. The LDL-c (Low Density Lipoprotein - cholesterol) in serum of drug treatment group was reduced compared to that of control group, and the adipose and hepatic tissues of rat in drug-treated groups showed reduced size of adipose cells and reduced level of liver steatosis.

Atherosclerosis Model: Ldlr-Deficient Mice. We measured compound anti-atherosclerosis efficacy using *Ldlr*^{*-*/}*⁻* mice fed western style diet (20% fat, 0.15% cholesterol), compound (100 mg/day) was orally administered by blending with diet. After 8 weeks, the mean lesion area in aortic sinuses of drug treatment group was less than that of the control group.

In vivo Anti-obesity Efficacy in obese mice. Male C57BL/6 mice were fed with high-fat diet (45% fat, OpenSource Diets D12451) for 8 weeks. Then obese mice with body weight 20% larger than that of mice fed with normal diet (20 mice) were selected for experiments. Compound (100 mg/kg) was orally administered to 10 randomly selected obese mice by blending with diet. After 13 weeks, the mean body weight gain of drug treatment group was about less than that of the control group.

In vivo Anti-diabetic Efficacy in genetically diabetic db/db mice. Compound (100 mg/kg) was orally administered to 11 randomly selected male db/db mice by blending with normal diet, while the other 9 male db/db mice fed with normal diet as control group. After 20 weeks, the mean fasting plasma glucose of drug treatment group was significantly lower than that of control group (*p-value < 0.05).

From our results herein and further data we determine that preferred anti-weight gain, anti-obestity and anti-obesity related disease, such as anti-diabetes, effective human dosages of the compounds should be 0.1-10, 0.5-10, 0.5-5, 0.5-2.5, 0.5-1, 1-10, 1-5, 1-2.5, 2-10, or 2-5g/day.

It is understood that the examples and embodiments described herein are for illustrative purposes only.

## Claims

1. A pharmaceutical composition comprising a compound of formula I: 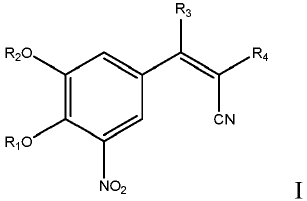 wherein:
R1 and R2 are independently H or Me;
R3 is OH or NHR, wherein R is H or an optionally substituted, optionally hetero-, optionally cyclic C1-C18 hydrocarbyl; and
R4 is optionally substituted, optionally hetero-, optionally cyclic C1-C18 hydrocarbyl;
or a stereoisomer, a hydrate, or a pharmaceutically acceptable salt thereof, and
a pharmaceutically-acceptable excipient,
formulated in a unit dosage form.

2. The pharmaceutical composition of claim 1, wherein the optionally substituted, optionally hetero-, optionally cyclic C1-C18 hydrocarbyl in each instance is an optionally substituted C1-C9 alkyl, C2-C9 alkenyl, C2-C9 alkynyl, or C5-C14 aryl hydrocarbon, comprising 1-5 heteroatoms that are N, S, O or P, including 1-5 nitrogen atoms, or a heteroatom substituted with the hydrocarbon.

3. The pharmaceutical composition of claim 1, wherein:
one or both R1 and R2 is H.

4. The pharmaceutical composition of claim 1, wherein:
R3 is OH.

5. The pharmaceutical composition of claim 1, wherein R is H or C1-C4 alkyl.

6. The pharmaceutical composition of claim 1, having formula:

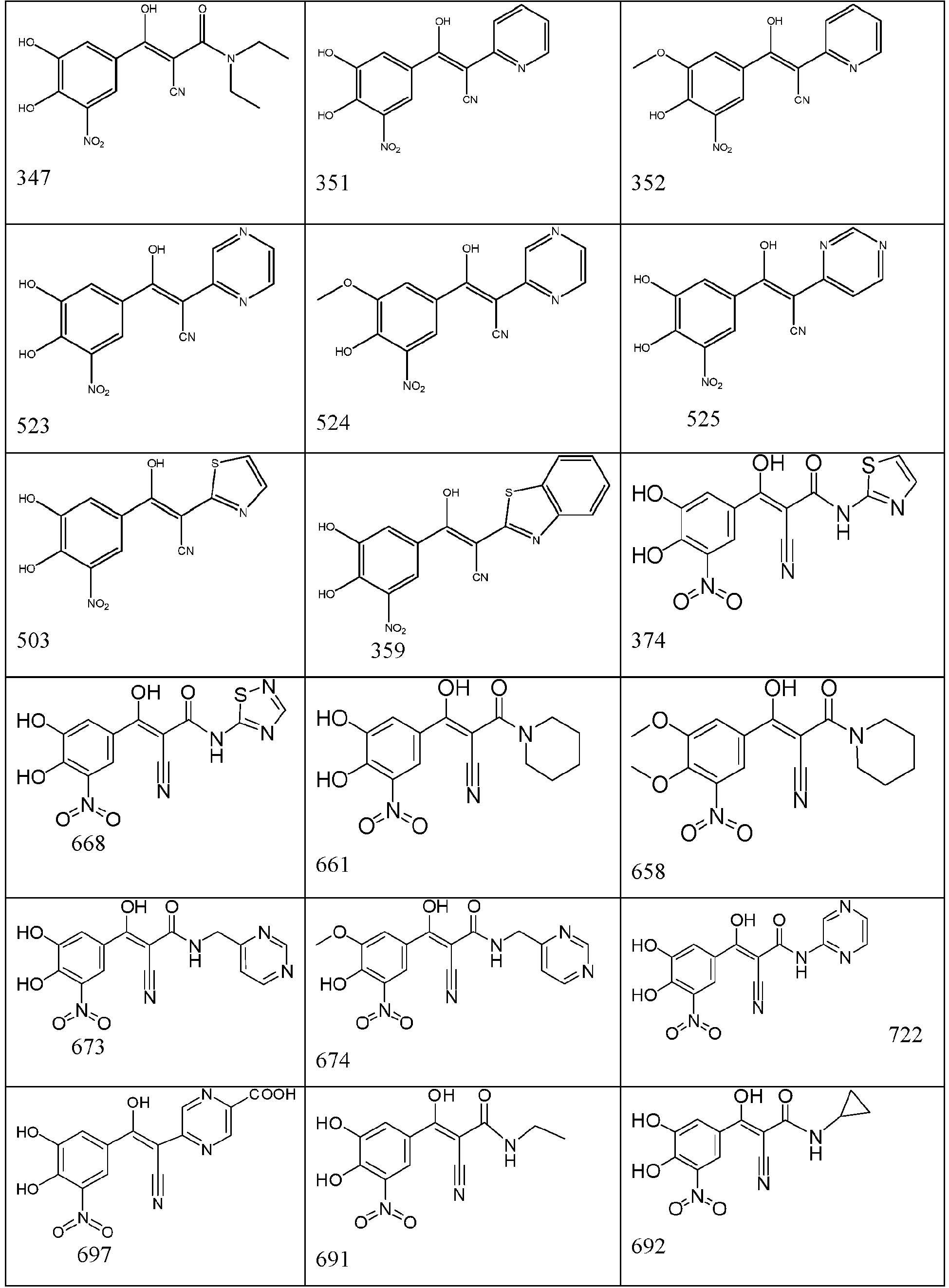

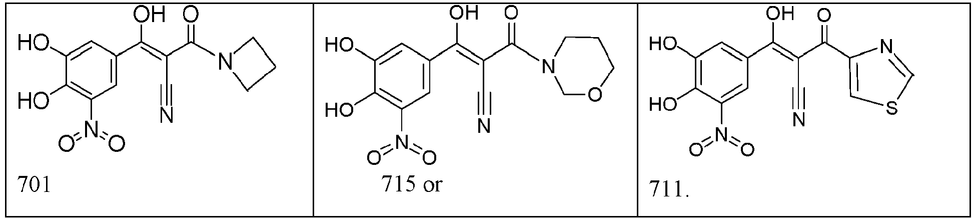

7. The pharmaceutical composition of claim 1, wherein R3 is NHR, having formula:

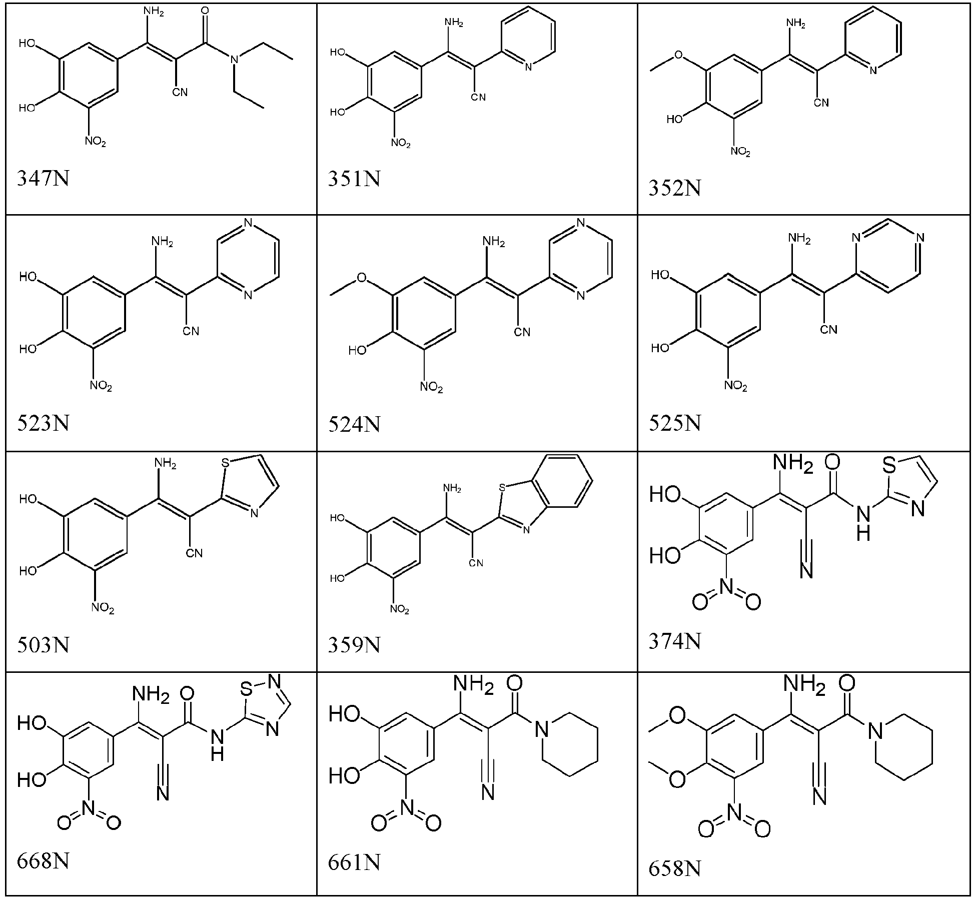

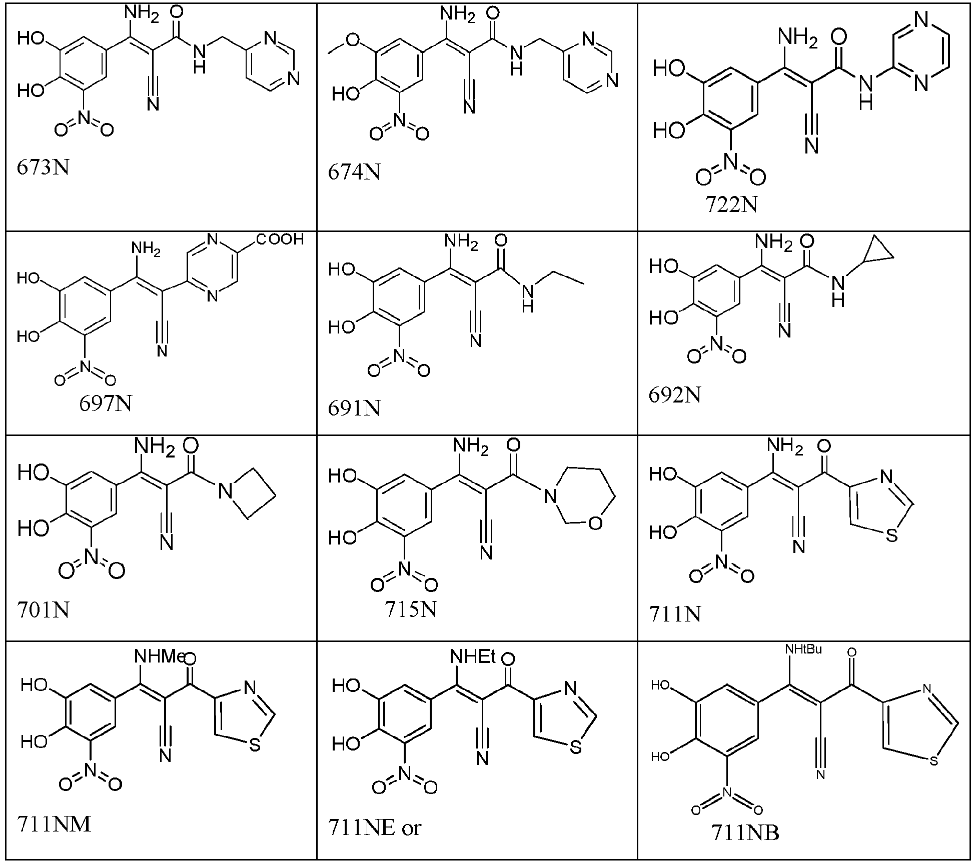

8. The pharmaceutical composition of any of the claims 1-7 for use in inhibiting weight gain, promoting weight loss, reducing serum LDL, cholesterol, LDL-c, or triglycerides, or treating obesity, an obesity related disease or Alzheimer's disease.

9. The pharmaceutical composition for use according to claim 8, wherein the obesity related disease is obesity related diabetes, hyperglycemia, diabetic nephropathy, hyperlipemia, coronary heart disease, atherosclerosis, hypertension, cardiovascular or cerebrovascular disease.

10. The pharmaceutical composition for use according to claim 8, copackaged or coformulated with a second, different medicament for inhibiting weight gain, promoting weight loss, reducing serum LDL, cholesterol, LDL-c, or triglycerides, or for treating obesity, an obesity related disease or Alzheimer's disease.

## Patentansprüche

1. Pharmazeutische Zusammensetzung umfassend eine Verbindung der Formel I: 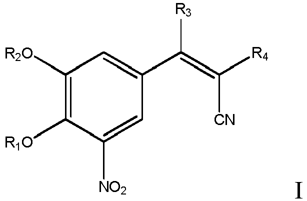 worin:
R1 und R2 unabhängig voneinander H oder Me sind;
R3 OH oder NHR ist, wobei R H oder ein wahlweise substituierter, wahlweise hetero-, wahlweise zyklischer C1-C18 Kohlenwasserstoff ist; und
R4 wahlweise ein substituierter, wahlweise hetero-, wahlweise zyklischer C1-C18 Kohlenwasserstoff ist;
oder ein Stereoisomer, ein Hydrat oder ein pharmazeutisch verträgliches Salz davon, und
ein pharmazeutisch verträglicher Hilfsstoff,
formuliert in einer Dosierungseinheitsform.

2. Pharmazeutische Zusammensetzung nach Anspruch 1, worin der wahlweise substituierte, wahlweise hetero-, wahlweise zyklische C1-C18 Kohlenwasserstoff in jedem Fall ein wahlweise substituierter C1-C9 Alkyl, C2-C9 Alkenyl, C2-C9 Alkynyl, oder C5-C14 Aryl-Kohlenwasserstoff ist, umfassend 1-5 Heteroatome die N, S, O oder P sind, einschließlich 1-5 Stickstoffatome, oder ein Heteroatom, das mit dem Kohlenwasserstoff substituiert ist.

3. Pharmazeutische Zusammensetzung nach Anspruch 1, worin:
Einer oder beide von R1 und R2 H ist.

4. Pharmazeutische Zusammensetzung nach Anspruch 1, worin:
R3 OH ist.

5. Pharmazeutische Zusammensetzung nach Anspruch 1, worin R H oder C1-C4 Alkyl ist.

6. Pharmazeutische Zusammensetzung nach Anspruch 1 mit der Formel:

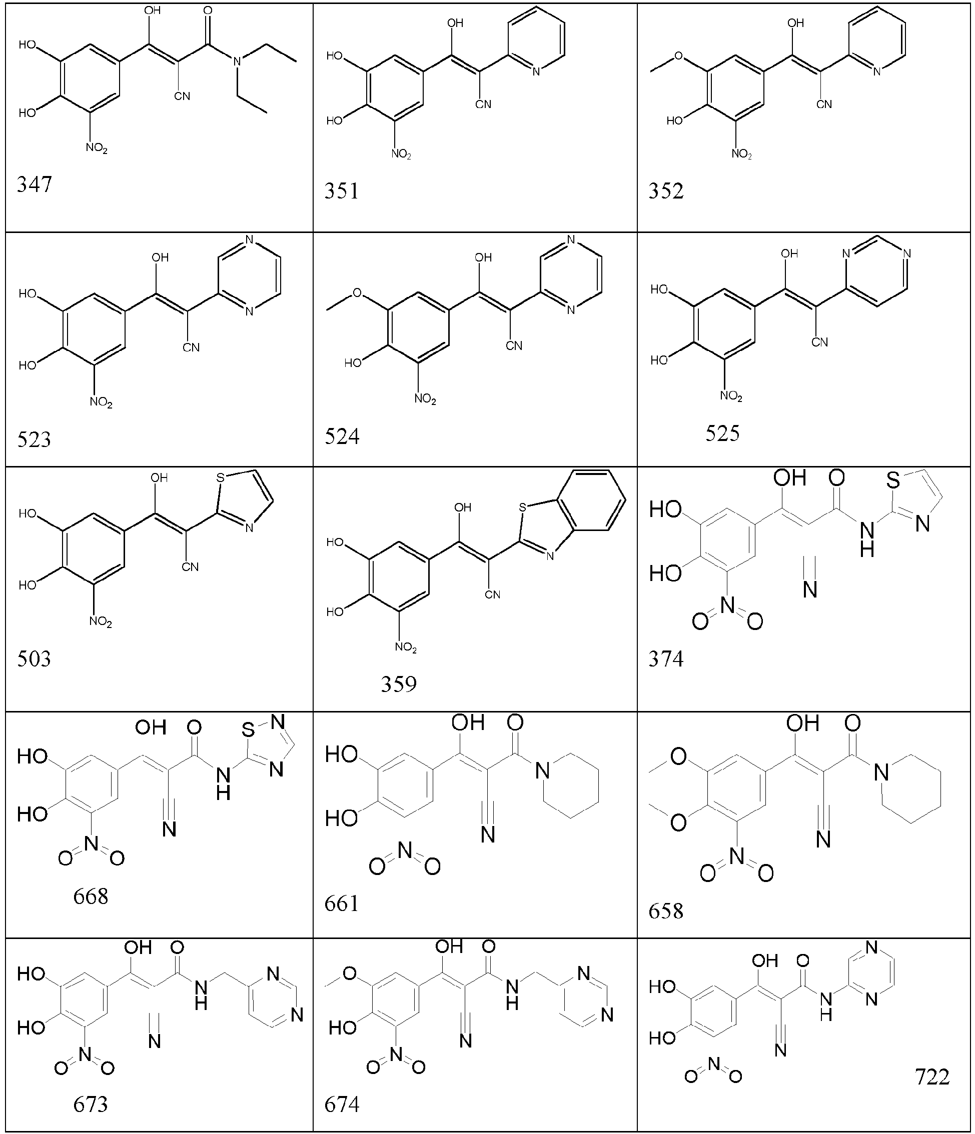

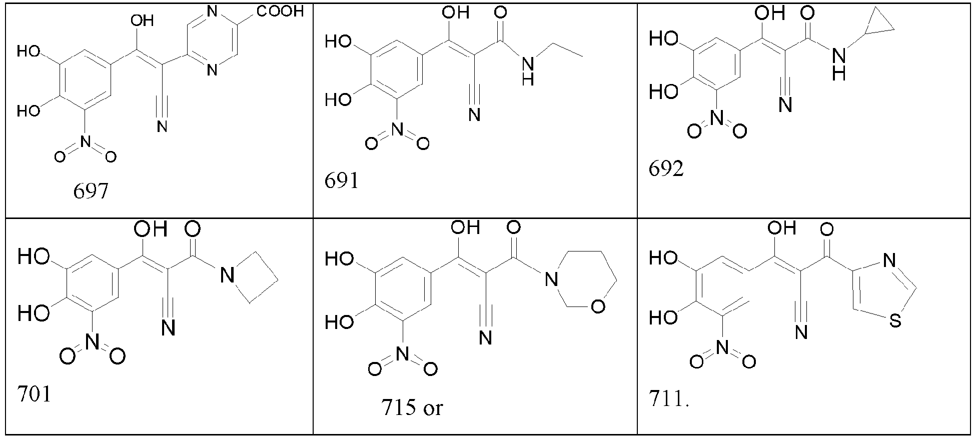

7. Pharmazeutische Zusammensetzung nach Anspruch 1, worin R3 NHR ist mit der Formel:

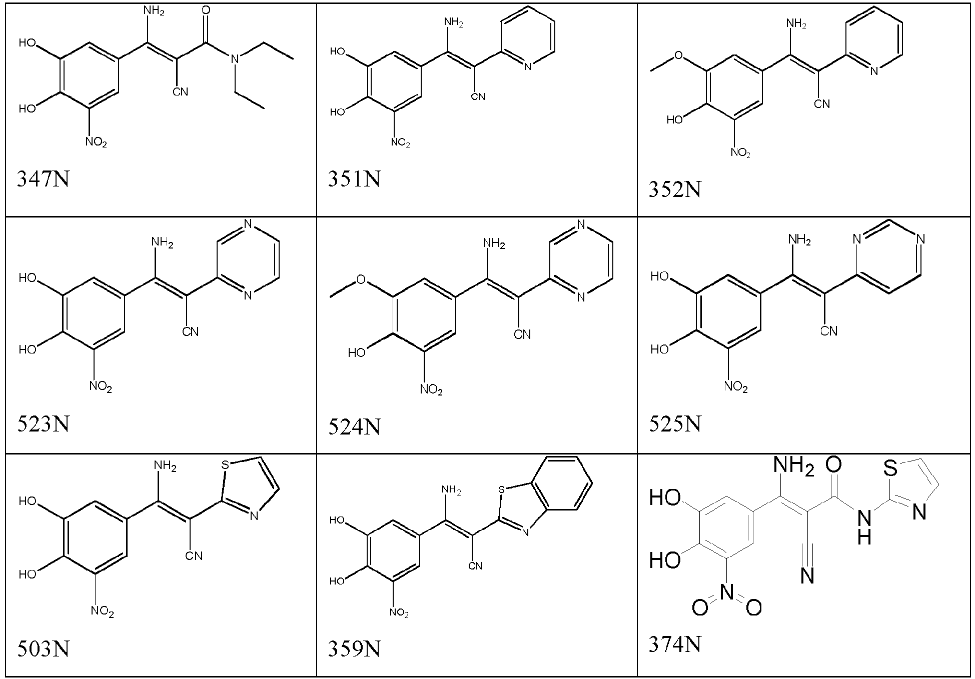

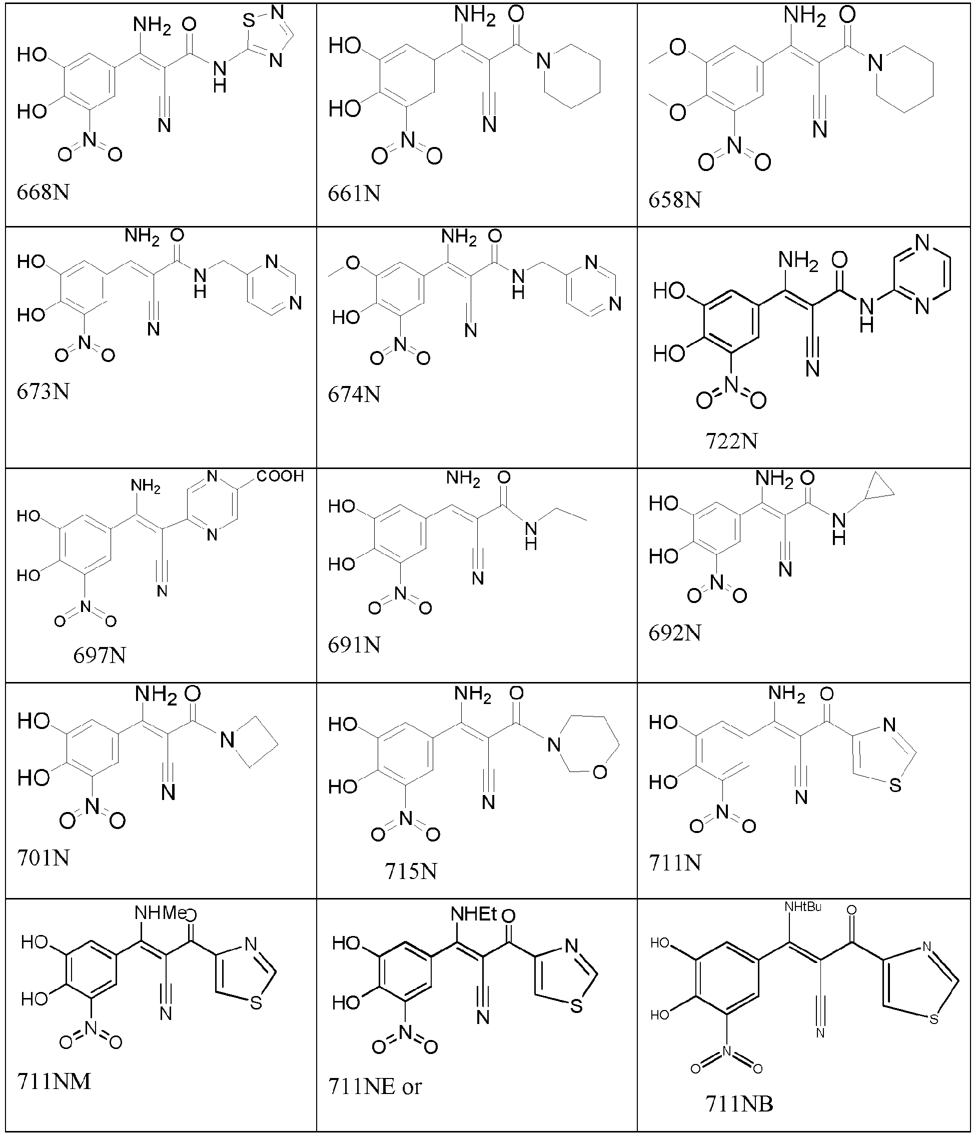

8. Pharmazeutische Zusammensetzung nach einem der Ansprüche 1-7 zur Verwendung beim Verhindern von Gewichtszunahme, beim Fördern von Gewichtsverlust, beim Verringern von Serum LDL, Cholesterin, LDL-c oder Triglyceriden, oder zur Behandlung von Adipositas, einer mit Adipositas in Verbindung stehenden Erkrankung, oder zur Behandlung von Alzheimer.

9. Pharmazeutische Zusammensetzung zur Verwendung nach Anspruch 8, worin die mit Adipositas in Verbindung stehende Erkrankung mit Adipositas in Verbindung stehende Diabetes, Hyperglykämie, diabetische Nephropathie, Hyperlipidemie, koronare Herzerkrankung, Atherosklerose, Bluthochdruck, kardiovaskuläre oder zerebrovaskuläre Erkrankung ist.

10. Pharmazeutische Zusammensetzung zur Verwendung nach Anspruch 8, mitverpackt oder zusammen formuliert mit einem zweiten verschiedenen Medikament zum Verhindern von Gewichtszunahme, zum Fördern von Gewichtsverlust, zum Verringern von Serum LDL, Cholesterin, LDL-c oder Triglyceriden, oder zum Behandeln von Adipositas, einer mit Adipositas in Verbindung stehenden Erkrankung, oder zum Behandeln von Alzheimer.

## Revendications

1. Composition pharmaceutique comprenant un composé qui répond à la formule (I) : 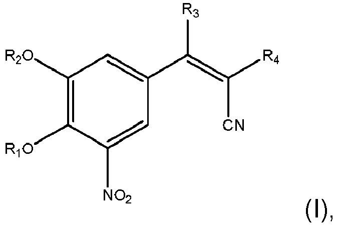 ou un stéréoisomère, un hydrate ou un sel pharmacologiquement admissible d'un tel composé,
dans laquelle formule :
- R₁ et R₂ représentent, indépendamment, un atome d'hydrogène ou un groupe méthyle,
- R₃ représente un groupe hydroxyle (OH) ou un groupe symbolisé par NHR, R désignant un atome d'hydrogène ou un groupe hydrocarbyle en C₁-C₁₈, en option cyclique, en option hétérocyclique, en option porteur de substituant(s),
- et R₄ représente un groupe hydrocarbyle en C₁-C₁₈, en option cyclique, en option hétérocyclique, en option porteur de substituant(s),
et un excipient pharmacologiquement admissible,
préparée sous une forme galénique unitaire.

2. Composition pharmaceutique selon la revendication 1, dans laquelle le groupe hydrocarbyle en C₁-C₁₈, en option cyclique, en option hétérocyclique, en option porteur de substituant(s), est à chaque fois un groupe hydrocarboné alkyle en C₁-C₉, alcényle en C2-C9, alcynyle en C2-C9 ou aryle en C₅-C₁₄, en option porteur de substituant(s), comportant de 1 à 5 hétéroatomes qui sont des atomes d'azote, de soufre, d'oxygène ou de phosphore, incluant de 1 à 5 atomes d'azote, ou un hétéroatome porteur du groupe hydrocarboné à titre de substituant.

3. Composition pharmaceutique selon la revendication 1, dans laquelle un ou chacun des symboles R₁ et R₂ représente un atome d'hydrogène.

4. Composition pharmaceutique selon la revendication 1, dans laquelle R₃ représente un groupe hydroxyle.

5. Composition pharmaceutique selon la revendication 1, dans laquelle R représente un atome d'hydrogène ou un groupe alkyle en C₁-C₄.

6. Composition pharmaceutique selon la revendication 1, la formule étant la suivante :

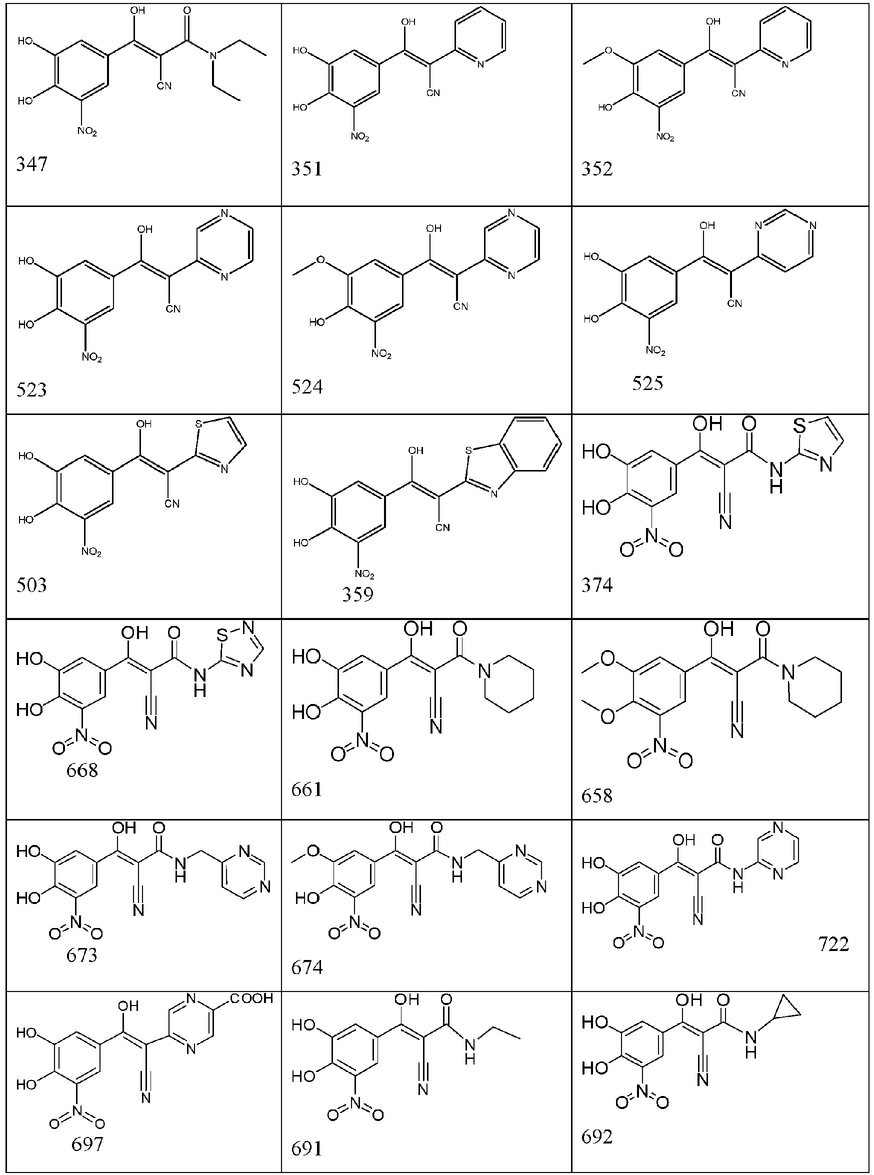

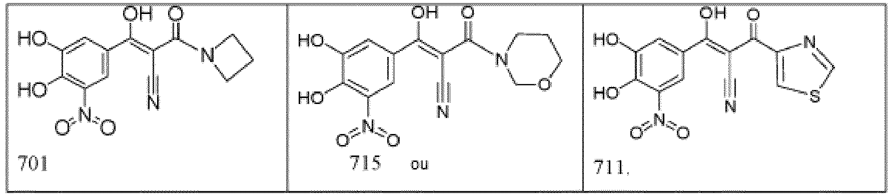

7. Composition pharmaceutique selon la revendication 1, dans laquelle R₃ représente un groupe symbolisé par NHR, la formule étant la suivante :

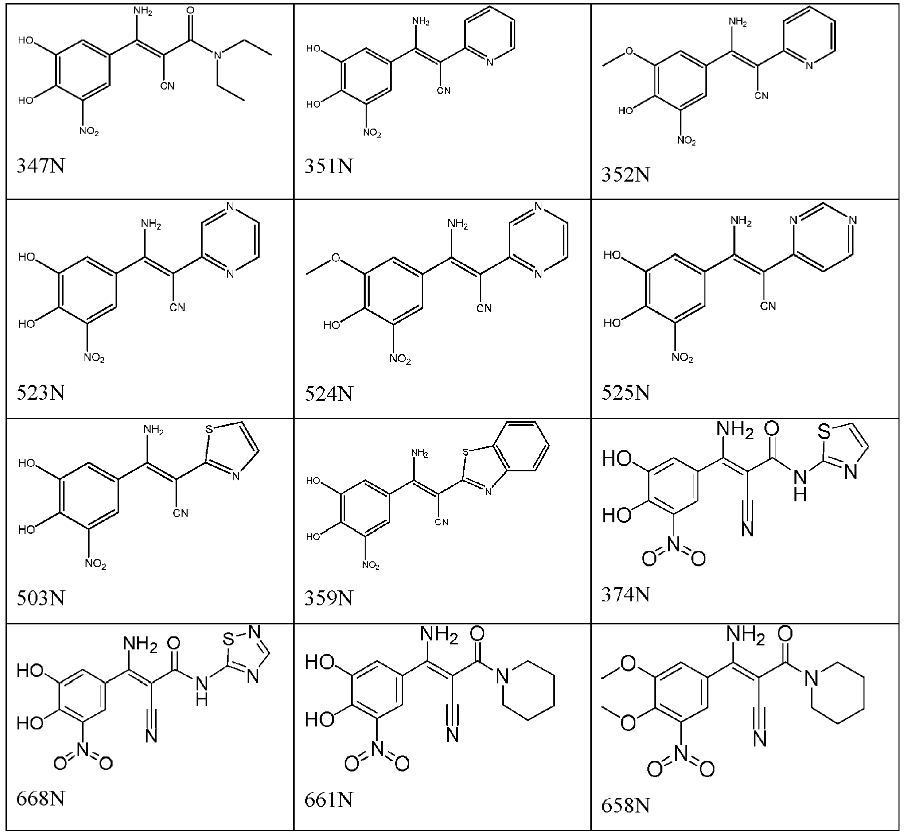

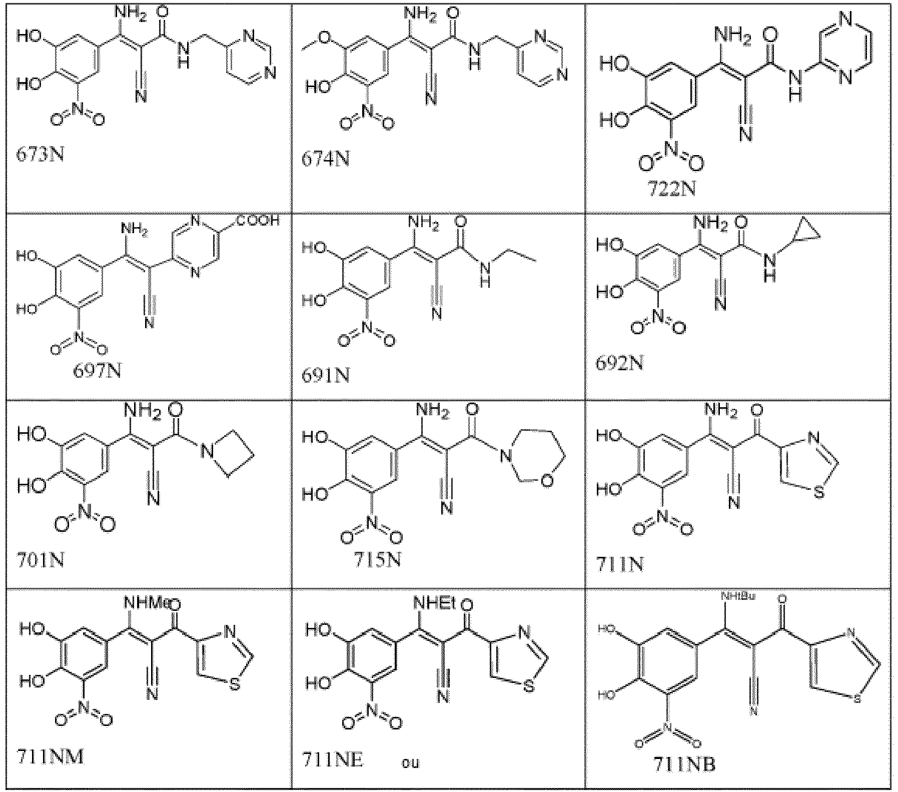

8. Composition pharmaceutique selon n'importe lesquelles des revendications 1 à 7, pour l'utilisation visant à empêcher la prise de poids, favoriser la perte de poids, réduire le taux sérique de LDL, cholestérol, LDL-C ou triglycérides, ou en vue de traiter l'obésité, une pathologie liée à l'obésité ou une maladie d'Alzheimer.

9. Composition pharmaceutique pour l'utilisation conforme à la revendication 8, la pathologie liée à l'obésité étant un diabète lié à l'obésité, une hyperglycémie, une néphropathie diabétique, une hyperlipidémie, une coronaropathie, une athérosclérose, une hypertension, ou une maladie cardiovasculaire ou cérébrovasculaire.

10. Composition pharmaceutique pour l'utilisation conforme à la revendication 8, conditionnée ou préparée conjointement avec un deuxième médicament différent visant à empêcher la prise de poids, favoriser la perte de poids, réduire le taux sérique de LDL, cholestérol, LDL-C ou triglycérides, ou en vue de traiter l'obésité, une pathologie liée à l'obésité ou une maladie d'Alzheimer.
